(19) Europäisches Patentamt
European Patent Office
Office européen des brevets

(11) Veröffentlichungsnummer: **0 234 485 B1**

(12) # EUROPÄISCHE PATENTSCHRIFT

(45) Veröffentlichungstag der Patentschrift: **01.04.92**

(51) Int. Cl.$^5$: **C07D 495/04**, A61K 31/435, //(C07D495/04,333:00,235:00)

(21) Anmeldenummer: **87102248.9**

(22) Anmeldetag: **17.02.87**

Die Akte enthält technische Angaben, die nach dem Eingang der Anmeldung eingereicht wurden und die nicht in dieser Patentschrift enthalten sind.

(54) **Substituierte Thienoimidazol-Derivate, Verfahren zu ihrer Herstellung, sie enthaltende pharmazeutische Zubereitungen und ihre Verwendung als Magensäuresekretionshemmer.**

(30) Priorität: **20.02.86 DE 3605395**
**12.07.86 DE 3623683**
**09.01.87 DE 3700436**

(43) Veröffentlichungstag der Anmeldung:
**02.09.87 Patentblatt 87/36**

(45) Bekanntmachung des Hinweises auf die Patenterteilung:
**01.04.92 Patentblatt 92/14**

(84) Benannte Vertragsstaaten:
**AT BE CH DE ES FR GB GR IT LI LU NL SE**

(56) Entgegenhaltungen:
**EP-A- 150 586**
**EP-A- 167 943**
**EP-A- 0 201 094**
**GB-A- 2 134 523**

**Chem. Soc. Rev.8,563 (1979); D. LEDNICER, L.A. MITSCHER, The Organic Chemistry of Drug Synthesis, Vol. 2, Wiley Interscience 1980, S. 232/233**

(73) Patentinhaber: **HOECHST AKTIENGESELLSCHAFT**
**Postfach 80 03 20**
**W-6230 Frankfurt am Main 80(DE)**

(72) Erfinder: **Lang, Hans-Jochen, Dr.**
**Rüdesheimer Strasse 7**
**W-6238 Hofheim am Taunus(DE)**
Erfinder: **Rippel, Robert, Dr.**
**Frankfurter Strasse 66**
**W-6238 Hofheim am Taunus(DE)**
Erfinder: **Herling, Andreas W., Dr.**
**Dieburger Strasse 43**
**W-6072 Dreieich(DE)**
Erfinder: **Weidmann, Klaus, Dr.**
**Talweg 11**
**W-6242 Kronberg/Taunus(DE)**

EP 0 234 485 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Substituierte Thienoimidazol-Derivate, Verfahren zu ihrer Herstellung, sie enthaltende pharmazeutische Zubereitungen und ihre Verwendung als Magensäuresekretionshemmer

Benzimidazol-Derivate mit magensäuresekretionshemmender Wirkung sind z.B. aus DE-A-25 48 340, EP-A-5129 und DE-A-32 40 248 bekannt. EP-A-176 308 (offengelegt am 2. April 1986) betrifft N-substituierte Benzimidazol-Derivate.

In der EP-A-0 201 094, einer älteren europäischen Anmeldung, werden Thieno-[2,3-d]-Imidazole zur Magensäuresekretionshemmung beschrieben.

In Chem. Soc. Rev. 8, 563 (1979) und von D. Lednicer, L.A. Mitscher, The Organic Chemistry of Drug Synthesis, Vol 2, Wiley-Intersience, 1980, Seite 232/33 wird die Bioisosterie von -CH=CH- und -S- gelehrt.

Die vorliegende Erfindung betrifft Thienoimidazol-Derivate der Formel I

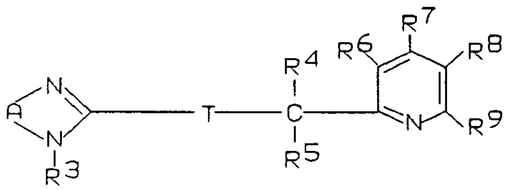

(I)

in welcher

A für

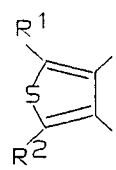

steht,

T -S-, -SO- oder $-SO_2-$ bedeutet,

$R^1$ und $R^2$ gleich oder verschieden sind und Wasserstoff, Halogen, Cyano, Nitro, Trifluormethyl, $(C_1-C_6)$-Alkyl, $(C_1-C_6)$-Hydroxyalkyl, $(C_1-C_6)$-Alkoxy, $(C_1-C_4)$-Fluoralkoxy, $-OCF_2Cl-$, $-O-CF_2-CHFCl$, $(C_1-c_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl bedeuten,

$R^3$ Wasserstoff, Alkanoyl, $(C_1-C_6)$-Alkylcarbamoyl oder eine andere physiologisch verträgliche, vorzugsweise im sauren Medium und/oder unter physiologischen Bedingungen abspaltbare $N^{im}$-Schutzgruppe bedeutet,

$R^4$ und $R^5$ gleich oder verschieden sind und Wasserstoff oder $(C_1-C_3)$-Alkyl bedeuten,

$R^6$, $R^7$, $R^8$ und $R^9$ gleich oder verschieden sind und Wasserstoff, Halogen, $(C_1-C_{12})$-Alkyl, $(C_1-C_{12})$-Alkoxy, $-O-CH_2-C_fH_{(2f+1-g)}F_g$, -NR'R", $(C_1-C_{12})$-Alkoxy-$(C_1-C_{12})$-alkyl, $(C_1-C_{12})$-Alkoxy-$(C_1-C_{12})$-alkoxy, $(C_7-C_{11})$-Aralkyloxy, $(C_1-C_{12})$-Alkylmercapto, $(C_1-C_{12})$-Alkylsulfinyl oder $(C_1-C_{12})$-Alkylsulfonyl bedeuten, oder

$R^5$ und $R^6$ gemeinsam für $-[CH_2]_i-$ stehen,

R' und R" gleich oder verschieden sind und Wasserstoff oder $(C_1-C_4)$-Alkyl bedeuten oder

R' und R" gemeinsam für $-[CH_2]_h-$ stehen, worin eine $CH_2$-Gruppe durch O, S, N-$(C_1-C_4)$-Alkanoylimino oder N-$(C_1-C_4)$-Alkoxylcarbonylimino ersetzt sein kann,

f = 1, 2, 3 oder 4 ist,

g = 1 bis (2f + 1) ist,

h = 4, 5 oder 6 ist,

i = 1, 2 oder 3 ist

und

n = 3 oder 4 ist,

sowie deren physiologisch verträglichen Salze.

Verbindungen der Formel I, in welchen $R^9$ für Wasserstoff steht, sind bevorzugt. T ist vorzugsweise eine -SO-Gruppe.

Besonders bevorzugt sind Verbindungen der Formel I, worin

T vorzugsweise eine -SO-Gruppe bedeutet,

$R^1$ und $R^2$ gleich oder verschieden sind und Wasserstoff, ($C_1$-$C_3$)-Alkyl, Halogen, ($C_1$-$C_4$)-Alkoxy oder ($C_1$-$C_4$)-Alkoxycarbonyl bedeuten,

$R^3$ wie oben definiert ist,

$R^4$ und $R^5$ jeweils Wasserstoff bedeuten und/oder

$R^6$, $R^7$, $R^8$ und $R^9$ gleich oder verschieden sind und Wasserstoff, Halogen, ($C_1$-$C_3$)-Alkyl, ($C_1$-$C_4$)-Alkoxy, Benzyloxy oder ($C_1$-$C_7$)-Alkoxy-($C_1$-$C_3$)-alkyl bedeuten, wobei $R^9$ vorzugsweise für Wasserstoff steht, und wobei Halogen vorzugsweise Chlor oder Brom bedeutet,

insbesondere aber Verbindungen der Formel I, worin

T vorzugsweise eine -SO-Gruppe bedeutet,

$R^1$ und $R^2$ gleich oder verschieden sind und Wasserstoff oder ($C_1$-$C_3$)-Alkyl bedeuten,

$R^3$ wie oben definiert ist,

$R^4$ und $R^5$ jeweils Wasserstoff bedeuten,

$R^6$ und $R^8$ gleich oder verschieden sind und Wasserstoff, Chlor, Methyl oder Ethyl bedeuten,

$R^9$ Wasserstoff bedeutet und/oder

$R^7$ Wasserstoff, ($C_1$-$C_4$)-Alkoxy, ($C_1$-$C_3$)-Alkyl oder Benzyloxy bedeutet.

Von besonderer Bedeutung sind

2-(2-Picolylsulfinyl)-1H-thieno[3,4-d]imidazol;
2-(4-Methoxy-2-picolylsulfinyl)-1H-thieno[3,4-d]imidazol;
2-(4-Methoxy-3-methyl-2-picolylsulfinyl)-1H-thieno[3,4-d]-imidazol;
2-(4-Methoxy-3,5-dimethyl-2-picolylsulfinyl)-1H-thieno-[3,4-d]imidazol;
2-(3-Methyl-2-picolylsulfinyl)-1H-thieno[3,4-d]imidazol;
2-(5-Methyl-2-picolylsulfinyl)-1H-thieno[3,4-d]imidazol;
2-(4-Methyl-2-picolylsulfinyl)-1H-thieno[3,4-d]imidazol;
2-(5-Ethyl-2-picolylsulfinyl)-1H-thieno[3,4-d]imidazol;
4,6-Dimethyl-2-(5-methyl-2-picolylsulfinyl)-1H-thieno-[3,4-d]imidazol;
2-(3-Chlor-4-methoxy-2-picolylsulfinyl)-1H-thieno-[3,4-d]-imidazol.

Alkyl und davon abgeleitete Reste wie beispielsweise Alkoxy, Alkylmercapto, Alkylsulfinyl, Alkylsulfonyl, Aralkyl oder Alkanoyl können geradkettig oder verzweigt sein.

($C_6$-$C_{12}$)-Aryl ist beispielsweise Phenyl, Naphthyl oder Biphenylyl, bevorzugt ist Phenyl.

($C_7$-$C_{11}$)-Aralkyl ist beispielsweise Benzyl oder Phenethyl, vorzugsweise Benzyl. Entsprechendes gilt für davon abgeleitete Reste wie Aralkyloxy.

Halogen steht für Fluor, Chlor, Brom oder Jod.

$R^3$ steht vorzugsweise für Wasserstoff, ($C_1$-$C_6$)-Alkylcarbamoyl oder einen Rest der Formel VI,

$$-(CO\text{-}O\text{-})_p\ (CR^{11}R^{12}\text{-}O\text{-})_q\ W\text{-}B \qquad (VI)$$

worin p = 0 oder 1, q = 0 oder 1 und B Wasserstoff, einen Acylrest oder einen gegebenenfalls substituierten Alkylrest bedeuten.

$R^{11}$ und $R^{12}$ sind gleich oder verschieden und bedeuten Wasserstoff, ($C_1$-$C_6$)-Alkyl, ($C_3$-$C_8$)-Cycloalkyl, ($C_7$-$C_{11}$)-Aralkyl oder ($C_6$-$C_{12}$)-Aryl.

B und $R^{11}$ können auch gemeinsam für eine -$[CH_2]_r$-Kette mit r = 3, 4 oder 5 - vorzugsweise 4 - stehen, wobei an einer oder mehreren der $CH_2$-Gruppen jeweils ein Wasserstoffatom durch OH, geschütztes OH, Amino, Acylamino und/oder Halogen ersetzt sein kann. Bei einem Rest mit substituierter -$[CH_2]_r$-Kette handelt es sich vorzugsweise um einen gegebenenfalls mit in der Kohlenhydratchemie üblichen Schutzgruppen teilweise oder vollständig geschützten Glycosylrest, der sich von einer Glycopyranose, Glycofuranose oder einem Oligosaccharid ableitet.

Der Glycosylrest kann sowohl $\alpha$- als auch $\beta$-glycosidisch verknüpft sein.

Er kann beispielsweise einen Glucofuranosyl- oder Glucopyranosyl-Rest sein, der sich von natürlich vorkommenden Aldotetrosen, Aldopentosen, Aldohexosen, Ketopentosen, Desoxyaldosen, Aminoaldosen und Oligosacchariden, wie Di- und Trisacchariden, sowie deren Stereoisomeren ableiten.

Diese Glycosylreste leiten sich insbesondere von natürlichen, in Mikroorganismen, Pflanzen, Tieren

oder Menschen vorkommenden D- oder L-Monosacchariden wie Ribose (Rib), Arabinose (Ara), Xylose (Xyl), Lyxose (Lyx), Allose (All), Altrose (Alt), Glucose (Glc), Mannose (Man), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetyl-glucosamin (GlcNAc), N-Acetylgalactosamin (GalNAc), N-Acetyl-mannosamin (ManNAc) oder Disacchariden, wie Maltose (Mal), Lactose (Lac), Cellobiose (Cel), Gentiobiose (Gen), N-Acetyl-lactosamin (LacNAc), Chitobiose (Chit), β-Galactopyranosyl -(1-3)-N-acetylgalactosamin und β-Galactopyranosyl-(1-3)- oder -(1-4)-N-acetyl-glucosamin, sowie deren synthetischen Derivate, wie 2-Desoxy-, 2-Amino-, 2-Acetamido- oder 2-Halogeno-, bevorzugt Bromo- oder Iodo-Zucker ab.

Unter den in der Kohlenhydratchemie üblichen Schutzgruppen versteht man z.B. die $(C_1\text{-}C_{10})$-Acylschutzgruppen wie $(C_1\text{-}C_6)$-Alkanoyl (z.B. Acetyl-, Trichloracetyl-, Trifluoracetyl-), Benzoyl- oder p-Nitrobenzoyl-, sowie gegebenenfalls modifizierte Methyl-, Methyloxymethyl-, Benzyl-, Tetrahydropyranyl-, Benzyliden-, Isopropyliden-oder Trityl-Gruppe, wobei hier die Acylschutzgruppen, insbesondere die Acetyl-(Ac)-Gruppe, bevorzugt sind.

a) Falls $p = q = 0$ ist, haben die Reste vorzugsweise folgende Bedeutungen:
W ist eine Bindung oder bedeutet -CO-, $-CR^{13}R^{14}-$ oder $-CO-CR^{13}R^{14}-$.
B bedeutet Wasserstoff (nur, falls W keine Bindung ist), $(C_1\text{-}C_{10})$-Alkyl; $(C_2\text{-}C_{12})$-Alkenyl; $(C_3\text{-}C_{12})$-Cycloalkyl; $(C_6\text{-}C_{12})$-Aryl, das gegebenenfalls durch 1,2 oder 3 gleiche oder verschiedene Reste aus der Reihe $(C_1\text{-}C_4)$-Alkyl, Chlor, Brom, Fluor, Nitro, Trifluormethyl, $(C_1\text{-}C_4)$-Alkoxy und Hydroxy substituiert ist; $-(CH_2)_s-CH(NH_2)-R^{15}$ mit s = 1 - 9; den Acylrest einer Aminosäure oder $(C_1\text{-}C_6)$-Alkyl, das durch bis zu 4 gleiche oder verschiedene Reste aus der Reihe F, Cl oder Br substituiert ist.

$R^{13}$ und $R^{14}$ sind gleich oder verschieden und bedeuten Wasserstoff, $(C_1\text{-}C_6)$-Alkyl, $(C_1\text{-}C_6)$-Alkoxy, $(C_3\text{-}C_8)$-Cycloalkyl, $(C_7\text{-}C_{11})$-Aralkyl, $(C_6\text{-}C_{12})$-Aryl oder Pyridyl oder $R^{13}$ und $R^{14}$ stehen gemeinsam für $-[CH_2]_4-$, $-[CH_2]_5-$ oder $[CH_2]_6-$, worin 1 oder 2 $CH_2$-Gruppen durch 0 ersetzt sein können.

$R^{15}$ bedeutet Wasserstoff oder $(C_1\text{-}C_{10})$-Alkyl.

b) Falls q = 1 ist, sind W und B wie oben unter (a) definiert. Darüber hinaus kann W -CO-O- und $-CO-O-CR^{13}-R^{14}-$ bedeuten, wobei $R^{13}$ und $R^{14}$ die obengenannten Bedeutungen haben. B kann auch im Falle von W = Bindung für Wasserstoff stehen.

c) Falls p = 1 und q = 0 ist, steht W für eine Bindung oder bedeutet $-CR^{13}R^{14}-$, wobei $R^{13}$ und $R^{14}$ die Bedeutungen wie unter (a) haben. B ist definiert wie unter (a), kann aber nicht für den Acylrest einer Aminosäure stehen. -CO-O-W-B kann darüber hinaus für weitere $N^{im}$-Schutzgruppen vom Urethantyp stehen, die von der vorstehenden Definition nicht umfaßt werden (vgl. z.B. Hubbuch, Kontakte Merck 3/79 14-23; Büllesbach, Kontakte Merck 1/80 23-35).

Unter einem gegebenenfalls substituierten $(C_6\text{-}C_{12})$-Arylrest (siehe oben unter (a)) wird beispielsweise Phenyl, (o-, m-, p-)Tolyl, (o-, m-, p-)Ethylphenyl, 2-Ethyl-tolyl, 4-Ethyl-o-tolyl, 5-Ethyl-m-tolyl, (o-, m-oder p-)Propylphenyl, 2-Propyl-(o-, m- oder p-)Tolyl, 4-Isopropyl-2,6-xylyl, 3-Propyl-4-ethylphenyl, (2,3,4-, 2,3,6-, oder 2,4,5-)Trimethylphenyl, (o-, m- oder p-)-Fluorphenyl, (o-, m- oder p-Trifluormethyl)phenyl, 4-Fluor-2,5-xylyl, (2,4-, 2,5-, 2,6-, 3,4- oder 3,5-)Difluorphenyl, (o-, m- oder p-)Chlorphenyl, 2-Chlor-p-tolyl, (3-, 4-, 5- oder 6-)Chlorotolyl, 4-Chlor-2-propylphenyl, 2-Isopropyl-4-chlorphenyl, 4-Chlor-3,5-xylyl, (2,3-, 2,4-, 2,5-, 2,6- oder 3,5-)Dichlorphenyl, 4-Chlor-3-fluorphenyl, (3- oder 4-)-Chlor-2-fluorphenyl, (o-, m- oder p-)Trifluormethylphenyl, (o-, m- oder p-)Ethoxyphenyl, (4- oder 5-) Chlor-2-methoxyphenyl, 2,4-Dichlor-(5- oder 6-)methylphenyl oder (o-, m- oder p-)Methoxyphenyl verstanden.

$(C_1\text{-}C_{10})$-Alkyl ist beispielsweise Methyl, Ethyl, Propyl, Butyl, Pentyl, Hexyl, Heptyl, Octyl, Nonyl, Decyl oder deren isomere Formen.

$(C_3\text{-}C_{12})$-Cycloalkyl schließt alkylsubstituiertes Cycloalkyl und bi- und mehrcyclische Systeme mit ein. Es wird darunter beispielsweise verstanden; Cyclopropyl, 2-Methylcyclopropyl, 2,2-Dimethylcyclopropyl, 2,3-Diethylcyclopropyl, 2-Butylcyclopropyl, Cyclobutyl, 2-Methylcyclobutyl, 3-Propylcyclobutyl, 2,3,4-Triethylcyclobutyl, Cyclopentyl, 2,2-Dimethylcyclophenyl, 2-Pentylcyclopentyl, 3-tert-Butylcyclopentyl, 2,2-Dimethylcyclohexyl, Cycloheptyl, Cyclononyl, Cyclodecyl, Norbornyl oder Adamantyl.

Unter einem Acylrest einer Aminosäure wird vorzugsweise der Rest einer α-Aminosäure, insbesondere aus der Reihe der natürlich vorkommenden α-Aminosäuren oder deren Antipoden verstanden, wie z.B. H-Gly-, H-Ala-, H-Val-, H-Leu-, H-Ile, H-Phe-, H-Lys-, H-Pro-, H-Trp-, H-Met-, H-Ser-, H-Thr-, H-Cys-, H-Tyr-, H-Asn-, H-Gln-, H-Asp-, H-Glu-, H-Arg-, H-Orn- oder die entsprechenden Reste in der D-Konfiguration.

Ohne daß der Erfindungsgegenstand darauf beschränkt wäre, seien im folgenden einige erfindungsgemäße Urethanschutzgruppen $R^3$ = -CO-O-WB genannt. $(C_1\text{-}C_6)$-Alkoxycarbonyl wie Boc; $(C_3\text{-}C_{12})$-Cycloalkyloxycarbonyl wie Mboc, Iboc oder Adoc;

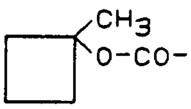

Mboc

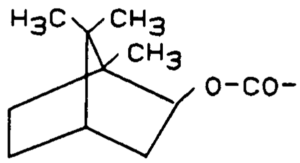

Iboc

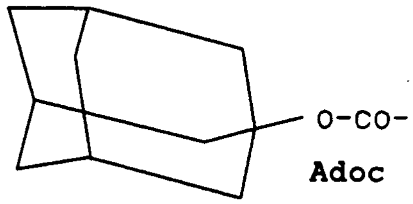

Adoc

($C_3$-$C_{12}$)-Cycloalkyl-($C_1$-$C_6$)-alkoxycarbonyl wie Adpoc;

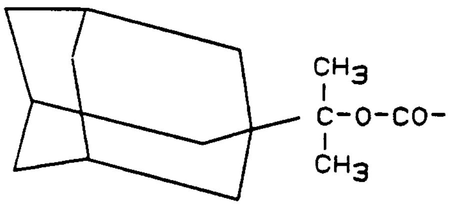

Adpoc

($C_6$-$C_{12}$)-Aryl-($C_1$-$C_6$)-alkoxycarbonyl wie Z, Fmoc oder Bpoc,

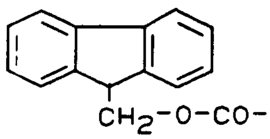

Fmoc

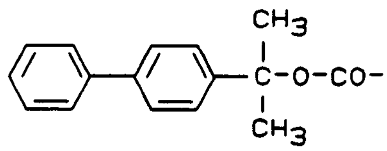

Bpoc

substituierte Z-Reste wie Moc, Ddz und Z (p-$NO_2$)

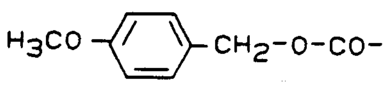

Moc

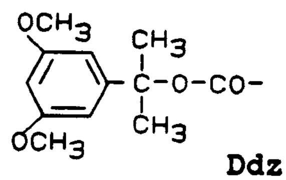

Ddz

und modifizierte Z-Reste wie Pyoc und deren von 2- bzw. 3-Picolin abgeleitete Reste, die wie oben bei ($C_6$-$C_{12}$)-Aryl angegeben substituiert sein können.

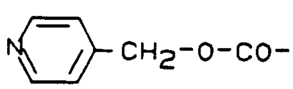

Pyoc

Bevorzugte $N^{im}$-Schutzgruppen sind solche, die in Gegenwart von Säuren, vorzugsweise in einem pH-Bereich von etwa 1 - 6 und/oder unter physiologischen Bedingungen abgespalten werden können.

Es ist überraschend, daß Verbindungen der Formel I mit $R^3 \neq H$ vielfach stabiler sind als die entsprechenden Verbindungen mit $R^3 = H$. Sie sind vor allem stabiler unter sauren Bedingungen, wie sie beispielsweise im Magen herrschen, sowie in Gegenwart von Wasser. Durch gezielte Auswahl einer $N^{im}$-Schutzgruppe ist es somit für den Fachmann möglich, die Freisetzung der aktiven Verbindungen so zu

steuern, daß diese selektiv am Wirkort erfolgt.

Gegebenenfalls vorhandene chirale C- und S-Atome können sowohl in der R- als auch in der S-Konfiguration vorkommen. In solchen Fällen liegen Verbindungen der Formel I in Form der reinen Enantiomeren oder als Stereoisomerengemisch (wie Enantiomerengemisch und Diastereomerengemisch) vor.

Als Salze kommen insbesondere Alkali- und Erdalkalisalze und Salze mit physiologisch verträglichen Aminen in Frage.

Die Erfindung betrifft weiterhin ein Verfahren zur Herstellung von Verbindungen der Formel I, das dadurch gekennzeichnet ist, daß man

a) Verbindungen der Formel II

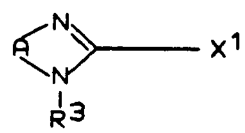

(II)

in welcher A, $R^1$, $R^2$ und $R^3$ wie oben definiert sind und

$X^1$ i. eine Abgangsgruppe oder
ii. -SH, $-S^-$ oder $-SO_2^-$ bedeutet,

umsetzt mit Verbindungen der Formel III

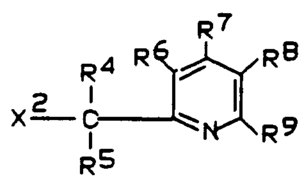

(III)

in welcher $R^4$, $R^5$, $R^6$, $R^7$, $R^8$ und $R^9$ wie oben definiert sind und

$X^2$ im oben genannten Fall i. -SH, $-S^-$ oder $-SO_2^-$ und
im oben genannten Fall ii. eine Abgangsgruppe bedeutet, oder

b) Verbindungen der Formel IV,

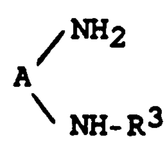

(IV)

in welcher A, $R^1$, $R^2$ und $R^3$ wie oben definiert sind, umsetzt mit Verbindungen der Formel V

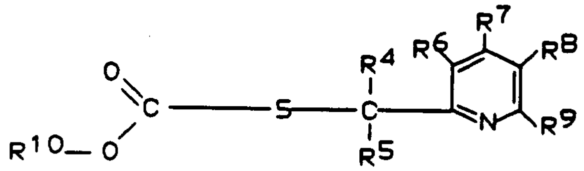

(V)

in welcher $R^4$, $R^5$, $R^6$, $R^7$, $R^8$ und $R^9$ wie oben definiert sind und $R^{10}$ für eine veresternde Gruppe steht,

i. in Verbindungen der Formel I (eine) gegebenenfalls vorhandene -S-Gruppe(n) gewünschtenfalls zu(r) -SO-oder $-SO_2$-Gruppe(n) oxidiert,

ii. in Verbindungen der Formel I (eine) gegebenenfalls vorhandene -SO-Gruppe(n) gewünschtenfalls zu(r) $-SO_2$-Gruppe(n) oxidiert,

iii. Verbindungen der Formel I, worin $R^3$ für Wasserstoff steht, gewünschtenfalls acyliert, alkyliert oder aralkyliert,

iv. Verbindungen der Formel I, worin $R^3$ nicht Wasserstoff bedeutet, gewünschtenfalls verseift und

v. Verbindungen der Formel I gewünschtenfalls in ihre physiologisch verträglichen Salze überführt,

wobei zwei oder mehr der Maßnahmen i.-iv. auch in einer anderen als der angegebenen Reihenfolge ausgeführt werden können.

Setzt man gemäß der hier bevorzugten Verfahrensvariante (a) Verbindungen der Formel II mit Verbindungen der Formel III um, so steht $X^1$ oder $X^2$ für eine Abgangsgruppe, die nucleophil ablösbar ist, wie Cl, Br, J, $-O-SO_2-CH_3$, $-O-SO_2-CF_3$ oder $-O-SO_2-(C_6H_4-pCH_3)$.

Die Umsetzung einer Verbindung der Formel II mit einer Verbindung der Formel III oder deren Salzen erfolgt in einem inerten Lösungsmittel wie z.B. Wasser, Methylenchlorid, Methanol, Ethanol, Aceton, Essigsäureethylester, Toluol, Tetrahydrofuran, Acetonitril, Dimethylformamid, Dimethylsulfoxid oder Gemischen dieser Lösungsmittel zweckmäßigerweise in Gegenwart einer anorganischen oder organischen Base, wie z.B. Natrium- oder Kaliumhydroxid, -carbonat, -alkoxid, -hydrid, -amid, Ammoniak, Triethylamin, Tributylamin, Pyridin bei -20 bis +150 ° C, vorzugsweise bei 0 - 80 ° C.

Die Verbindungen der Formel II können in Analogie zu bekannten Verfahren hergestellt werden, z.B. durch Ringschluß entsprechend substituierter 2,3-, 3,4- oder 4,5-Diaminothiophene der oben definierten Formel IV mit entsprechenden Schwefelverbindungen wie Schwefelkohlenstoff (z.B. DE-A-31 32 167).

Die hierfür benötigten 2,3-, 3,4- oder 4,5-Diaminothiophene sind entweder literaturbekannt oder können in Analogie zu bekannten Verfahren hergestellt werden. Sie werden z.B. durch Reduktion entsprechend substituierter Aminonitrothiophene erhalten.

In den bei Verfahrensvariante (b) eingesetzten Estern der Formel V steht $R^{10}$ für eine veresternde Gruppe, vorzugsweise $(C_1-C_6)$-Alkyl oder Benzyl.

Die Umsetzung einer Verbindung der Formel IV mit einer Verbindung der Formel V gemäß Verfahrensvariante (b) erfolgt analog der in Preston et al., Benzimidazoles and Congeneric Tricyclic Compounds, Part 1, New York, Seiten 10-13 beschriebenen Verfahrensweisen.

Die so erhaltenen Verbindungen der Formel I können, falls $R^3$ Wasserstoff bedeutet, in physiologisch verträgliche Salze umgewandelt werden.

Verbindungen der Formel I mit T = -S- können ferner mit geeigneten Oxidationsmitteln in solche mit T = -SO- oder $-SO_2-$ umgewandelt werden. In gleicher Weise lassen sich auch -S-Gruppen in den Substituenten $R^1$, $R^2$ und $R^6$ bis $R^9$ oxidieren.

Diese Reaktion erfolgt in einem geeigneten, inerten Lösungsmittel wie z.B. Methylenchlorid, Chloroform, Tetrachlorkohlenstoff, 1,2-Dichlorethan, Toluol, Essigsäureethylester, Essigsäure, Trifluoressigsäure, Wasser, Methanol, Ethanol oder Gemischen derselben bei -20 ° C bis +150 ° C vorzugsweise bei -10 ° C bis +40 ° C.

Als Oxidationsmittel kommen z.B. in Betracht:

Wasserstoffperoxid, Persäuren, und Perester wie Peressigsäure, Trifluorperessigsäure, Monoperphthalsäure, m-Chlorperbenzoesäure und deren Ester, Ozon, Distickstofftetroxid, Jodosobenzol, N-Chlorsuccinimid, 1-Chlorbenzotriazol, Natriumhypochlorit, Kaliumperoxodisulfat, t-Butylhypochlorit, Tetrabutylammoniumperjodat oder -permanganat, Natrium-meta-perjodat, Selen- oder Mangandioxid, Cerammonnitrat, Chromsäure, Chlor, Brom, Diazabicyclo-[2.2.2]octan-Bromkomplex, Dioxandibromid, Pyridiniumperbromid, Sulfurylchlorid, 2-Arylsulfonyl-3-aryloxaziridine, Titantetraisopropylat/tert. Butylhydroperoxid (gegebenenfalls unter Zusatz von Dialkylestern der (D)- bzw. (L)-Weinsäure und einer definierten Menge Wasser).

Ebenso können isolierte, ggf. immobilisierte oxidierende Enzyme oder Mikroorganismen als Oxidationsmittel Anwendung finden.

Die Oxidationsmittel werden in äquimolaren Mengen, ggf. auch in einem geringen Überschuß von 5 - 10 Mol-% bei der Oxidation zu T = -SO- oder auch in größerem Überschuß und/oder bei höherer Reaktionstemperatur eingesetzt wenn eine Oxidation zu T = $-SO_2-$ gewünscht wird.

Verbindungen der Formel I mit $R^3 \neq H$ können hergestellt werden ausgehend von Verbindungen der Formel IV mit $R^3 = H$ und Verbindungen der Formel V oder durch Acylierung, Alkylierung oder Aralkylierung von Verbindungen der Formel I mit $R^3 = H$. Im folgenden soll auf den zweiten Weg etwas näher eingegangen werden.

Die Acylierung, Alkylierung oder Aralkylierung von Verbindungen der Formel I wird in an sich bekannter Weise mit den entsprechenden Acylierungsmitteln, Alkylierungsmitteln bzw. Aralkylierungsmitteln in einem geeigneten organischen Lösungsmittel in der Regel bei einer Temperatur zwischen -78 ° C und dem Siedepunkt des Reaktionsgemisches gegebenenfalls in Gegenwart einer Base durchgeführt.

$N^{im}$-Schutzgruppen der Formel VI mit p = 0, q = 1, W = Bindung und B = Wasserstoff lassen sich in Verbindungen der Formel I ($R^3$ = H, T = S) beispielsweise durch Hydroxyalkylierung einführen, wobei man $N^{im}$-Schutzgruppen mit $R^{11} = R^{12}$ = Wasserstoff in an sich bekannter Weise (vgl. z.B. Eur. J. Med. Chem. 15 [1980] 586; J. Med. Chem. 22 [1979] 1113) durch Hydroxymethylierung mit Formaldehyd in einem

organischen Lösungsmittel wie z.B. Acetonitril einführen kann. Die Hydroxyalkylierung wird bei einer Temperatur zwischen 0 ° C und dem Siedepunkt des Reaktionsgemisches gegebenenfalls in Gegenwart einer Base wie Triethylamin durchgeführt.

Hydroxymethylverbindungen der Formel VII

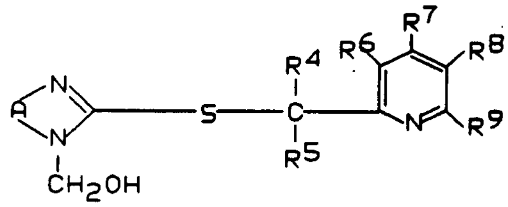

(VII)

können in der in EP-A-176308, Seite 11 beschriebenen Weise in Acylderivate der Formel VIII

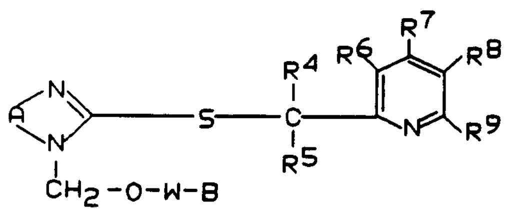

(VIII)

worin W-B ein Acylrest ist, überführt werden.

Verbindungen der Formel I mit $R^3$ = H können auch mit Reagenzien der Formel IX

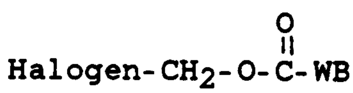

(IX)

wie z.B. Chlormethylpivalat, in bekannter Weise alkyliert werden, wobei die entsprechenden Carbonate (W = -CO-O- oder -CO-O-$CR^{13}$ $R^{14}$-) erhalten werden. Die Umsetzung wird z.B. in der im EP-A-176308, Seite 12 beschriebenen Weise durchgeführt.

Acylreste von Aminosäuren werden in bekannter Weise (z.B. DCC/HOBt- oder Dialkylphosphinsäureanhydrid-Methode) an Verbindungen der Formel I mit $R^3$ = H gekuppelt.

$N^{im}$-Schutzgruppen der Formel VI mit p = 0, q = 1 und $R^{11}$ und/oder $R^{12}$ $\neq$ Wasserstoff werden eingeführt, indem man eine Verbindung der Formel I ($R^3$ = H, T = S) mit 1 bis 10 Äquivalenten, vorzugsweise 2 bis 3 Äquivalenten des entsprechenden $\alpha$-Halogenalkylesters umsetzt. Die verwendeten $\alpha$-Halogenalkylester werden aus Säurehalogeniden und Aldehyden nach bekannten Methoden erhalten (vgl. z.B. J. Amer. Chem. Soc. 43 [1921] 660; J. Med. Chem. 23 [1980] 469-474).

Bevorzugt werden Bromalkylester verwendet. Alternativ kann man das Anion einer Verbindung der Formel I ($R^3$ = H, T = S), welches aus dieser und NaH zugänglich ist, mit dem $\alpha$-Halogenalkylester behandeln.

An Stelle der $\alpha$-Halogenalkylester können auch 1-(Alkylcarbonyloxy)-alkyl -pyridinium-Salze, die analog den bekannten 1-(Arylcarbonyloxy)-alkyl -pyridinium-Salzen (vgl. Angew. Chem. Suppl. 1982, 675-685) aus den entsprechenden Acylhalogeniden, Aldehyden und Pyridin hergestellt werden, Verwendung finden.

Alkylaminoacetale der Formel I, worin $R^3$ für einen Rest der Formel VI steht, in dem p = 0, q = 1 ist und W eine Bindung oder -$CR^{13}R^{14}$- bedeutet und B obige Bedeutung hat, werden hergestellt, indem man eine Verbindung der obengenannten Formel VI in einem dipolar aprotischen Lösungsmittel wie Dimethylformamid bei etwa 20 bis 50 ° C, vorzugsweise bei etwa 25 ° C mit etwa einem Äquivalent NaH behandelt. Das so erhaltene Anion wird dann mit etwa einem Äquivalent eines Halogenethers der Formel Halogen-$CR^{11}R^{12}$-W-B (Halogen = Chlor oder Brom) umgesetzt, wobei man die Reaktionsmischung 15 Minuten lang bei etwa 20 bis 50 ° C, vorzugsweise bei ca. 25 ° C rührt. Die Halogenether sind bekannt und sind vielfach im Handel erhältlich oder sie können in Analogie zu bekannten Verbindungen hergestellt werden.

Urethane der Formel I, worin $R^3$ für eine Urethanschutzgruppe der Formel VI (p = 1, q = 0 und W =

Bindung oder -$CR^{13}R^{14}$-) steht, werden aus den entsprechenden Verbindungen mit $R^3$ = H erhalten, in dem man diese gegebenenfalls in Gegenwart einer Base, wie NaH, in einem geeigneten Lösungsmittel, wie DMF, mit Fluor- oder Chlorameisensäureestern der Formel Cl(F)-CO-O-WB umsetzt (in Analogie zu der in EP-A-176308, Seite 12 beschriebenen Verfahrensweise).

Die Fluor- bzw. Chlorformiate sind bekannt und oft im Handel erhältlich oder können nach bekannten Methoden hergestellt werden.

Aralkyloxycarbonyl- und Alkoxycarbonylgruppen können auch mit den bekannten, oft käuflichen Dicarbonaten, wie Di-tert. Butyldicarbonat, Dibenzyldicarbonat, eingeführt werden.

Substituierte bzw. modifizierte Z-Gruppen in denen $R^{13}$ und/oder $R^{14}$ ≠ Wasserstoff sind, werden durch Reaktion der entsprechenden ungeschützten Verbindung der Formel I, wenn notwendig, unter Zuhilfenahme einer Base mit den entsprechenden Aziden oder den entsprechenden Carbonaten hergestellt.

Zur Acylierung der Verbindungen der Formel I ($R^3$ = H, T = S) können neben den üblichen Standardbedingungen (z.B. Acetanhydrid, Triethylamin, Dimethylaminopyridin) auch andere Verfahren, wie z.B. Umsetzung mit N- 1-(Arylcarbonyloxy)-alkyl -pyridinium-Salzen (bekannt aus Angew. Chem. Suppl. 1982, 675-685) angewandt werden.

Zur Herstellung von Dialkoxyderivaten der Formel I ($R^3$ = -$CR^{13}R^{14}$-B, worin $R^{13}$ und $R^{14}$ jeweils Alkoxy oder zusammen Alkylendioxy und B = H bedeuten; T = S oder SO) wird vorzugsweise die entsprechende Verbindung der Formel I mit $R^3$ = H in Gegenwart einer Base mit den entsprechenden Orthoameisensäureestern, wie Orthoameisensäuretrialkylestern, umgesetzt.

Erfindungsgemäß können außer den in den Ausführungsbeispielen beschriebenen Thienoimidazol-Derivaten beispielsweise auch die in der folgenden Tabelle 1 zusammengestellten Verbindungen der allgemeinen Formel I bzw. deren Salze erhalten werden.

Verwendete Abkürzungen:

Methyl (Me), Ethyl (Et), Propyl (Pr), Butyl (Bu), Hexyl (Hex), Acetyl (Ac), Phenyl (Ph), cyclo (c), iso (i).

**Tabelle 1**

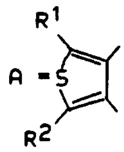

, T = S, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | H | H | Me | H | H | H |
| H | H | H | H | Me | H | OMe | H |
| H | H | H | H | Me | H | OEt | H |
| H | H | H | H | Me | Me | H | H |
| H | H | H | H | Me | Me | OMe | H |
| H | H | H | H | Me | Me | OMe | Me |
| H | H | H | H | Me | H | H | Me |
| H | H | H | H | Me | H | Me | H |
| H | H | H | H | H | Me | Me | H |
| H | H | H | H | H | Et | H | H |
| H | H | H | H | H | H | Et | H |
| H | H | H | H | H | H | Pr | H |
| H | H | H | H | H | H | H | Pr |
| H | H | H | H | H | H | H | Bu |
| H | H | H | H | H | Me | OEt | H |
| H | H | H | H | H | H | OPr | H |
| H | H | H | H | H | H | OBu | H |
| H | H | H | H | H | H | OHex | H |
| H | H | H | H | H | H | H | Hex |
| H | H | H | H | H | Me | Me | Me |
| H | H | H | H | H | H | O-iPr | H |
| H | H | H | H | H | H | iPr | H |
| H | H | H | H | H | H | H | iPr |

**Tabelle, Fortsetzung**

A = 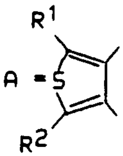 , T = S, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | H | H | H | Cl | H | H |
| H | H | H | H | H | H | Cl | H |
| H | H | H | H | H | H | H | Cl |
| H | H | H | H | H | Cl | H | Me |
| H | H | H | H | H | Cl | Me | H |
| H | H | H | H | H | H | Cl | Me |
| H | H | H | H | H | H | Me | Cl |
| H | H | H | H | H | Cl | 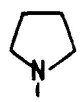 | H |
| H | H | H | H | H | Cl | 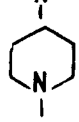 | H |
| H | H | H | H | H | Cl | OEt | H |
| H | H | H | H | H | Cl | OPr | H |
| H | H | H | H | H | Cl | OBu | H |
| H | H | H | H | H | Cl | 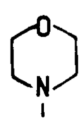 | H |
| H | H | H | H | H | Cl | $O-(CH_2)_2-OMe$ | H |
| H | H | H | H | H | Me | $O-(CH_2)_2-OMe$ | H |
| H | H | H | H | H | H | $O-(CH_2)_2-Ph$ | H |
| H | H | H | H | H | H | $O-(CH_2)_3-Ph$ | H |
| H | H | H | H | H | H | $OCH_2CF_3$ | H |
| H | H | H | H | H | H | $OCH_2(CF_2)_2CF_3$ | H |

**Tabelle, Fortsetzung**

A = 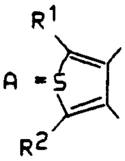 , T = S, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | H | H | H | H | $OCH_2CF_2CF_3$ | H |
| H | H | H | H | H | H | $OCH_2-CF_2-CF_2H$ | H |
| H | H | H | H | H | Me | $OCH_2-CF_3$ | H |
| H | H | H | H | H | H | $OCH_2CF_3$ | Me |
| H | H | H | H | H | Cl | $OCH_2CF_3$ | H |
| H | H | H | H | H | Me | $OCH_2mF_2CF_3$ | H |
| Me | H | H | H | H | H | H | H |
| Me | H | H | H | H | H | OMe | H |
| Me | H | H | H | H | Me | OMe | H |
| Me | H | H | H | H | Me | OMe | Me |
| Me | H | H | H | H | Me | H | H |
| Me | H | H | H | H | H | Me | H |
| Me | H | H | H | H | H | H | Me |
| Me | H | H | H | H | H | H | Et |
| Me | H | H | H | H | H | $O-CH_2-Ph$ | H |
| Me | H | H | H | H | Cl | 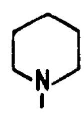 | H |
| Me | H | H | H | H | Cl | 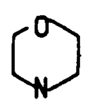 | H |
| Me | H | H | H | H | Cl | H | H |
| Me | H | H | H | H | H | Cl | H |
| Me | H | H | H | H | Cl | Me | H |

**Tabelle, Fortsetzung**

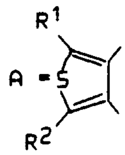

, T = S, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | H | H | H | H | H | $OCH_2CF_3$ | H |
| Me | H | H | H | H | Me | H | Me |
| Et | H | H | H | H | H | H | H |
| Et | H | H | H | H | H | OMe | H |
| Et | H | H | H | H | Me | H | H |
| Et | H | H | H | H | H | H | Me |
| i-Pr | H | H | H | H | H | H | H |
| i-Pr | H | H | H | H | H | OMe | H |
| i-Pr | H | H | H | H | H | H | Me |
| -CH(OH)-Me | H | H | H | H | H | H | H |
| -CH(OH)-Me | H | H | H | H | H | OMe | H |
| -CH(OH)-Me | H | H | H | H | Me | H | H |
| -CH(OH)-Me | H | H | H | H | H | H | Me |
| -CH(OH)-Me | H | H | H | H | Me | H | Me |
| OMe | Me | H | H | H | H | H | H |
| OMe | Me | H | H | H | Me | OMe | Me |
| OMe | Me | H | H | H | Me | H | H |
| OMe | Me | H | H | H | H | H | Me |
| OMe | Me | H | H | H | H | OMe | H |
| OMe | Me | H | H | H | H | Me | H |

**Tabelle, Fortsetzung**

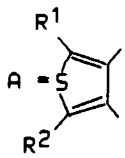 , T = S, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | Me | H | H | H | Cl | 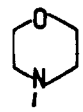 | H |
| OMe | Me | H | H | H | Cl | H | H |
| OMe | Me | H | H | H | H | H | Et |
| OMe | Me | H | H | H | Me | H | Me |
| Me | Ac | H | H | H | H | H | Me |
| Me | Ac | H | H | H | H | OMe | H |
| Me | Ac | H | H | H | Me | OMe | Me |
| OEt | Me | H | H | H | H | H | H |
| OEt | Me | H | H | H | Me | OMe | Me |
| OEt | Me | H | H | H | H | OMe | H |
| OEt | Me | H | H | H | H | H | Me |
| OEt | Me | H | H | H | Me | H | H |
| OBu | Me | H | H | H | H | H | Me |
| OBu | Me | H | H | H | Me | OMe | Me |
| OMe | OMe | H | H | H | H | H | H |
| OMe | OMe | H | H | H | H | OMe | H |
| OMe | OMe | H | H | H | Me | OMe | Me |
| OMe | OMe | H | H | H | Me | H | H |
| OMe | OMe | H | H | H | H | Me | H |
| OMe | OMe | H | H | H | H | H | Me |
| OMe | OMe | H | H | H | H | H | Et |

**Tabelle, Fortsetzung**

A = 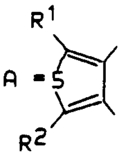 , T = S, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | H | H | H | Cl | 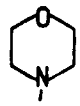 | H |
| OMe | OMe | H | H | H | Cl | OMe | H |
| OMe | Me | H | H | H | Cl | OMe | H |
| OMe | H | H | H | H | H | H | H |
| OMe | H | H | H | H | H | OMe | H |
| OMe | H | H | H | H | Me | OMe | Me |
| OMe | H | H | H | H | H | H | Me |
| OMe | H | H | H | H | H | Me | H |
| OMe | H | H | H | H | Me | H | H |
| OMe | Cl | H | H | H | H | H | H |
| OMe | Cl | H | H | H | Me | OMe | Me |
| OMe | Cl | H | H | H | H | H | Me |
| $PhSO_2$ | $PhSO_2$ | H | H | H | H | H | H |
| $PhSO_2$ | $PhSO_2$ | H | H | H | Me | OMe | Me |
| $PhSO_2$ | $PhSO_2$ | H | H | H | H | H | Me |
| NH-Ph | H | H | H | H | H | H | H |
| NH-Ph | H | H | H | H | Me | OMe | Me |
| NH-Ph | H | H | H | H | H | H | Me |
| NH-Ph | Cl | H | | | H | H | H |
| NH-Ph | Cl | H | | | Me | OMe | Me |
| NH-Ph | Cl | H | | | H | H | Me |

**Tabelle, Fortsetzung**

A = 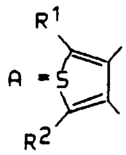 , T = S, $R^9$ = H

| $R^1$ | $R^2$ | $R^3$ | $R^4$ | $R^5$ | $R^6$ | $R^7$ | $R^8$ |
|---|---|---|---|---|---|---|---|
| O-Ph | H | H | H | H | H | H | H |
| O-Ph | H | H | H | H | Me | OMe | H |
| O-Ph | H | H | H | H | H | H | Me |
| O-Ph | Cl | H | H | H | H | H | H |
| Me | Me | H | H | H | H | H | Et |
| Me | Me | H | H | H | H | Et | H |
| Me | Me | H | H | H | Me | H | Me |
| Me | Me | H | H | H | Me | Me | Me |
| Me | Me | H | H | H | Cl | Me | H |
| Me | Me | H | H | H | Cl | OMe | H |
| Me | Me | H | H | H | Cl | H | H |
| Me | Me | H | H | H | Cl | Cl | H |
| Me | Me | H | H | H | Cl | 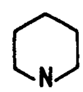 | H |
| Me | Me | H | H | H | Cl | 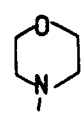 | H |
| Me | Me | H | H | H | H | O-$CH_2$Ph | H |
| Me | Me | H | H | H | H | -O-$CH_2$-$CF_3$ | H |
| Me | Me | H | H | H | H | -O-$CH_2$-$CF_2CF_3$ | H |
| Me | Et | H | H | H | H | H | H |
| Me | Et | H | H | H | Me | OMe | Me |
| Me | Et | H | H | H | H | H | Me |
| Cl | COOMe | H | H | H | H | H | H |

**Tabelle, Fortsetzung**

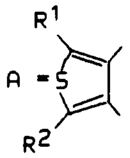 , T = S, $R^9$ = H

| $R^1$ | $R^2$ | $R^3$ | $R^4$ | $R^5$ | $R^6$ | $R^7$ | $R^8$ |
|---|---|---|---|---|---|---|---|
| Cl | COOMe | H | H | H | Me | OMe | Me |
| Cl | COOMe | H | H | H | H | OMe | H |
| Cl | COOMe | H | H | H | H | H | Me |
| H | $CONEt_2$ | H | H | H | H | H | H |
| H | $CONEt_2$ | H | H | H | Me | OMe | Me |
| H | $CONEt_2$ | H | H | H | H | H | Me |
| H | $CONH_2$ | H | H | H | H | H | H |
| H | $CONH_2$ | H | H | H | Me | OMe | Me |
| H | $CONH_2$ | H | H | H | H | H | Me |
| H | CONHEt | H | H | H | H | H | H |
| H | CONHEt | H | H | H | Me | OMe | Me |
| H | CONHEt | H | H | H | H | H | Me |
| $SO_2NMe_2$ | H | H | H | H | H | H | H |
| $SO_2NMe_2$ | H | H | H | H | Me | OMe | Me |
| $SO_2NMe_2$ | H | H | H | H | H | H | Me |
| H | H | H | H | H | H | H | H |
| H | H | H | H | H | Me | H | H |
| H | H | H | H | H | H | Me | H |
| H | H | H | H | H | Me | H | Me |
| H | H | H | H | H | H | H | Me |
| H | H | H | H | H | H | OMe | H |
| H | H | H | H | H | Me | OMe | Me |

**Tabelle, Fortsetzung**

A = 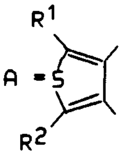 , T = S, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | H | H | H | H | H | Et |
| Me | Me | H | H | H | H | H | H |
| Me | Me | H | H | H | Me | OMe | Me |
| Me | Me | H | H | H | H | H | Me |
| H | H | H | H | H | Cl | 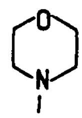 | H |
| H | H | H | H | H | H | $OCH_2CF_3$ | H |
| H | COOEt | H | H | H | H | H | H |
| H | COOEt | H | H | H | Me | OMe | Me |
| H | COOEt | H | H | H | H | H | Me |
| COOMe | COOMe | H | H | H | H | H | H |
| COOMe | COOMe | H | H | H | Me | OMe | Me |
| COOMe | COOMe | H | H | H | H | H | Me |
| $-(CH_2)_4-$ | | H | H | H | H | H | H |
| $-(CH_2)_4-$ | | H | H | H | Me | OMe | Me |
| $-(CH_2)_4-$ | | H | H | H | H | H | Me |
| $-CH_2-O-CH_2-$ | | H | H | H | H | H | H |
| $-CH_2-O-CH_2-$ | | H | H | H | Me | OMe | Me |
| $-CH_2-O-CH_2-$ | | H | H | H | H | H | Me |
| $-CH_2-S-CH_2$ | | H | H | H | H | H | H |
| $-CH_2-S-CH_2-$ | | H | H | H | Me | OMe | H |
| $-CH_2-S-CH_2-$ | | H | H | H | H | H | Me |

**Tabelle, Fortsetzung**

A = 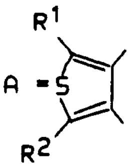 , T = S, $R^9$ = H

| $R^1$ $R^2$ | $R^3$ | $R^4$ | $R^5$ | $R^6$ | $R^7$ | $R^8$ |
|---|---|---|---|---|---|---|
| $-CH_2-SO-CH_2$ | H | H | H | H | H | H |
| $-CH_2-SO-CH_2-$ | H | H | H | Me | OMe | Me |
| $-CH_2-SO-CH_2-$ | H | H | H | Me | H | H |
| $-CH_2-SO-CH_2-$ | H | H | H | H | H | Me |
| -CH=CH-CH=CH- | H | H | H | H | H | H |
| -CH=CH-CH=CH- | H | H | H | Me | OMe | Me |
| -CH=CH-CH=CH- | H | H | H | H | H | Me |

Tabelle, Fortsetzung

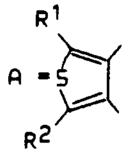

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | H | H | Me | H | H | H |
| H | H | H | H | Me | H | OMe | H |
| H | H | H | H | Me | H | OEt | H |
| H | H | H | H | Me | Me | H | H |
| H | H | H | H | Me | Me | OMe | H |
| H | H | H | H | Me | Me | OMe | Me |
| H | H | H | H | Me | H | H | Me |
| H | H | H | H | Me | H | Me | H |
| H | H | H | H | H | Me | Me | H |
| H | H | H | H | H | Et | H | H |
| H | H | H | H | H | H | Et | H |
| H | H | H | H | H | H | Pr | H |
| H | H | H | H | H | H | H | Pr |
| H | H | H | H | H | H | H | Bu |
| H | H | H | H | H | Me | OEt | H |
| H | H | H | H | H | H | OPr | H |
| H | H | H | H | H | H | OBu | H |
| H | H | H | H | H | H | OHex | H |
| H | H | H | H | H | H | H | Hex |
| H | H | H | H | H | Me | Me | Me |
| H | H | H | H | H | H | O-iPr | H |
| H | H | H | H | H | H | iPr | H |

**Tabelle, Fortsetzung**

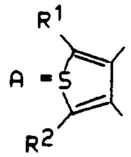 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | H | H | H | H | H | iPr |
| H | H | H | H | H | Cl | H | H |
| H | H | H | H | H | H | Cl | H |
| H | H | H | H | H | H | H | Cl |
| H | H | H | H | H | Cl | H | Me |
| H | H | H | H | H | Cl | Me | H |
| H | H | H | H | H | H | Cl | Me |
| H | H | H | H | H | H | Me | Cl |
| H | H | H | H | H | Cl | 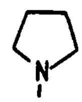 | H |
| H | H | H | H | H | Cl | 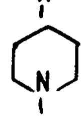 | H |
| H | H | H | H | H | Cl | OEt | H |
| H | H | H | H | H | Cl | OPr | H |
| H | H | H | H | H | Cl | OBu | H |
| H | H | H | H | H | Cl | 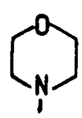 | H |
| H | H | H | H | H | Cl | $O-(CH_2)_2-OMe$ | H |
| H | H | H | H | H | Me | $O-(CH_2)_2OMe$ | H |
| H | H | H | H | H | H | $O-(CH_2)_2-Ph$ | H |
| H | H | H | H | H | H | $O-(CH_2)_3-Ph$ | H |
| H | H | H | H | H | H | $OCH_2CF_3$ | H |
| H | H | H | H | H | H | $OCH_2(CF_2)_2CF_3$ | H |

**Tabelle, Fortsetzung**

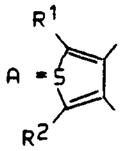 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | H | H | H | H | $OCH_2CF_2CF_3$ | H |
| H | H | H | H | H | H | $OCH_2$-$CF_2$-$CF_2H$ | H |
| H | H | H | H | H | Me | $OCH_2$-$CF_3$ | H |
| H | H | H | H | H | H | $OCH_2CF_3$ | Me |
| H | H | H | H | H | Cl | $OCH_2CF_3$ | H |
| H | H | H | H | H | Me | $OCH_2CF_2CF_3$ | H |
| Me | H | H | H | H | H | H | H |
| Me | H | H | H | H | H | OMe | H |
| Me | H | H | H | H | Me | OMe | H |
| Me | H | H | H | H | Me | OMe | Me |
| Me | H | H | H | H | Me | H | H |
| Me | H | H | H | H | H | Me | H |
| Me | H | H | H | H | H | H | Me |
| Me | H | H | H | H | H | H | Et |
| Me | H | H | H | H | H | O-$CH_2$-Ph | H |
| Me | H | H | H | H | Cl | 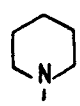 | H |
| Me | H | H | H | H | Cl | 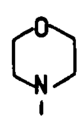 | H |
| Me | H | H | H | H | Cl | H | H |
| Me | H | H | H | H | H | Cl | H |
| Me | H | H | H | H | Cl | Me | H |
| Me | H | H | H | H | H | $OCH_2CF_3$ | H |

Tabelle, Fortsetzung

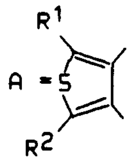 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | H | H | H | H | Me | H | Me |
| Et | H | H | H | H | H | H | H |
| Et | H | H | H | H | H | OMe | H |
| Et | H | H | H | H | Me | H | H |
| Et | H | H | H | H | H | H | Me |
| i-Pr | H | H | H | H | H | H | H |
| i-Pr | H | H | H | H | H | OMe | H |
| i-Pr | H | H | H | H | H | H | Me |
| -CH(OH)-Me | H | H | H | H | H | H | H |
| -CH(OH)-Me | H | H | H | H | H | OMe | H |
| -CH(OH)-Me | H | H | H | H | Me | H | H |
| -CH(OH)-Me | H | H | H | H | H | H | Me |
| -CH(OH)-Me | H | H | H | H | Me | H | Me |
| OMe | Me | H | H | H | H | H | H |
| OMe | Me | H | H | H | Me | OMe | Me |
| OMe | Me | H | H | H | Me | H | H |
| OMe | Me | H | H | H | H | H | Me |
| OMe | Me | H | H | H | H | OMe | H |
| OMe | Me | H | H | H | H | Me | H |

**Tabelle, Fortsetzung**

A = 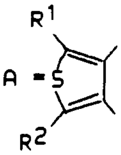 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | Me | H | H | H | Cl | 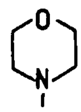 | H |
| OMe | Me | H | H | H | Cl | H | H |
| OMe | Me | H | H | H | H | H | Et |
| OMe | Me | H | H | H | Me | H | Me |
| Me | Ac | H | H | H | H | H | Me |
| Me | Ac | H | H | H | H | OMe | H |
| Me | Ac | H | H | H | Me | OMe | Me |
| OEt | Me | H | H | H | H | H | H |
| OEt | Me | H | H | H | Me | OMe | Me |
| OEt | Me | H | H | H | H | OMe | H |
| OEt | Me | H | H | H | H | H | Me |
| OEt | Me | H | H | H | Me | H | H |
| OBut | Me | H | H | H | H | H | Me |
| OBut | Me | H | H | H | Me | OMe | Me |
| OMe | OMe | H | H | H | H | H | H |
| OMe | OMe | H | H | H | H | OMe | H |
| OMe | OMe | H | H | H | Me | OMe | Me |
| OMe | OMe | H | H | H | Me | H | H |
| OMe | OMe | H | H | H | H | Me | H |
| OMe | OMe | H | H | H | H | H | Me |
| OMe | OMe | H | H | H | H | H | Et |

**Tabelle, Fortsetzung**

A = 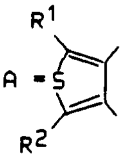 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | H | H | H | Cl | 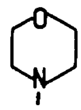 | H |
| OMe | OMe | H | H | H | Cl | OMe | H |
| OMe | Me | H | H | H | Cl | OMe | H |
| OMe | H | H | H | H | H | H | H |
| OMe | H | H | H | H | H | OMe | H |
| OMe | H | H | H | H | Me | OMe | Me |
| OMe | H | H | H | H | H | H | Me |
| OMe | H | H | H | H | H | Me | H |
| OMe | H | H | H | H | Me | H | H |
| OMe | Cl | H | H | H | H | H | H |
| OMe | Cl | H | H | H | Me | OMe | Me |
| OMe | Cl | H | H | H | H | H | Me |
| $PhSO_2$ | $PhSO_2$ | H | H | H | H | H | H |
| $PhSO_2$ | $PhSO_2$ | H | H | H | Me | OMe | Me |
| $PhSO_2$ | $PhSO_2$ | H | H | H | H | H | Me |
| NH-Ph | H | H | H | H | H | H | H |
| NH-Ph | H | H | H | H | Me | OMe | Me |
| NH-Ph | H | H | H | H | H | H | Me |
| NH-Ph | Cl | H | | | H | H | H |
| NH-Ph | Cl | H | | | Me | OMe | Me |
| NH-Ph | Cl | H | | | H | H | Me |

**Tabelle, Fortsetzung**

A = 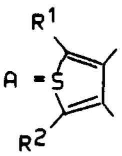 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| O-Ph | H | H | H | H | H | H | H |
| O-Ph | H | H | H | H | Me | OMe | H |
| O-Ph | H | H | H | H | H | H | Me |
| O-Ph | H | H | H | H | H | H | H |
| Me | Me | H | H | H | H | H | Et |
| Me | Me | H | H | H | H | Et | H |
| Me | Me | H | H | H | Me | H | Me |
| Me | Me | H | H | H | Me | Me | Me |
| Me | Me | H | H | H | Cl | Me | H |
| Me | Me | H | H | H | Cl | OMe | H |
| Me | Me | H | H | H | Cl | H | H |
| Me | Me | H | H | H | Cl | Cl | H |
| Me | Me | H | H | H | Cl | 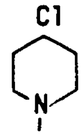 | H |
| Me | Me | H | H | H | Cl | 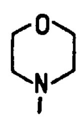 | H |
| Me | Me | H | H | H | H | O-$CH_2$Ph | H |
| Me | Me | H | H | H | H | -O-$CH_2CF_3$ | H |
| Me | Me | H | H | H | H | -O-$CH_2$-$CF_2CF_3$ | H |
| Me | Et | H | H | H | H | H | H |
| Me | Et | H | H | H | Me | OMe | Me |
| Me | Et | H | H | H | H | H | Me |
| Cl | COOMe | H | H | H | H | H | H |

**Tabelle, Fortsetzung**

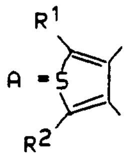 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Cl | COOMe | H | H | H | Me | OMe | Me |
| Cl | COOMe | H | H | H | H | OMe | H |
| Cl | COOMe | H | H | H | H | H | Me |
| H | $CONEt_2$ | H | H | H | H | H | H |
| H | $CONEt_2$ | H | H | H | Me | OMe | Me |
| H | $CONEt_2$ | H | H | H | H | H | Me |
| H | $CONH_2$ | H | H | H | H | H | H |
| H | $CONH_2$ | H | H | H | Me | OMe | Me |
| H | $CONH_2$ | H | H | H | H | H | Me |
| H | CONHEt | H | H | H | H | H | H |
| H | CONHEt | H | H | H | Me | OMe | Me |
| H | CONHEt | H | H | H | H | H | Me |
| $SO_2NMe_2$ | H | H | H | H | H | H | H |
| $SO_2NMe_2$ | H | H | H | H | Me | OMe | Me |
| $SO_2NMe_2$ | H | H | H | H | H | H | Me |
| H | H | H | H | H | H | H | H |
| H | H | H | H | H | Me | H | H |
| H | H | H | H | H | H | Me | H |
| H | H | H | H | H | Me | H | Me |
| H | H | H | H | H | H | H | Me |
| H | H | H | H | H | H | OMe | H |
| H | H | H | H | H | Me | OMe | Me |

**Tabelle, Fortsetzung**

A = 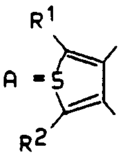 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | H | H | H | H | H | Et |
| Me | Me | H | H | H | H | H | H |
| Me | Me | H | H | H | Me | OMe | Me |
| Me | Me | H | H | H | H | H | Me |
| H | H | H | H | H | Cl | 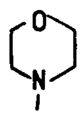 | H |
| H | H | H | H | H | H | $OCH_2CF_3$ | H |
| H | COOEt | H | H | H | H | H | H |
| H | COOEt | H | H | H | Me | OMe | Me |
| H | COOEt | H | H | H | H | H | Me |
| COOMe | COOMe | H | H | H | H | H | H |
| COOMe | COOMe | H | H | H | Me | OMe | Me |
| COOMe | COOMe | H | H | H | H | H | Me |
| $-(CH_2)_4-$ | | H | H | H | H | H | H |
| $-(CH_2)_4-$ | | H | H | H | Me | OMe | Me |
| $-(CH_2)_4-$ | | H | H | H | H | H | Me |
| $-CH_2-O-CH_2-$ | | H | H | H | H | H | H |
| $-CH_2-O-CH_2-$ | | H | H | H | Me | OMe | Me |
| $-CH_2-O-CH_2-$ | | H | H | H | H | H | Me |
| $-CH_2-S-CH_2-$ | | H | H | H | H | H | H |
| $-CH_2-S-CH_2-$ | | H | H | H | Me | OMe | H |
| $-CH_2-S-CH_2-$ | | H | H | H | H | H | Me |

**Tabelle, Fortsetzung**

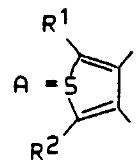 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| $-CH_2-SO-CH_2-$ | | H | H | H | H | H | H |
| $-CH_2-SO-CH_2-$ | | H | H | H | Me | OMe | Me |
| $-CH_2-SO-CH_2-$ | | H | H | H | Me | H | H |
| $-CH_2-SO-CH_2-$ | | H | H | H | H | H | Me |
| -CH=CH-CH=CH- | | H | H | H | H | H | H |
| -CH=CH-CH=CH- | | H | H | H | Me | OMe | Me |
| -CH=CH-CH=CH- | | H | H | H | H | H | Me |
| H | H | H | H | H | Cl | OMe | Cl |
| H | H | H | H | H | Cl | OEt | Cl |
| H | H | H | H | H | Cl | OPr | Cl |
| H | H | H | H | H | Cl | OHex | Cl |
| H | H | H | H | H | Cl | OiPr | Cl |
| H | H | H | H | H | Cl | $OCH_2Ph$ | Cl |
| H | H | H | H | H | Cl | $O(CH_2)_2OMe$ | Cl |
| H | H | H | H | H | Cl | $O(CH_2)_2Ph$ | Cl |
| H | H | H | H | H | Cl | $OCH_2CF_3$ | Cl |
| H | H | H | H | H | Cl | $OCH_2(CF_2)_2CF_3$ | Cl |
| H | H | H | H | H | Cl | $OCH_2CF_2CF_3$ | Cl |
| H | H | H | H | H | Cl | $OCH_2CF_2CF_2H$ | Cl |
| Me | Me | H | H | H | Cl | OMe | Cl |
| Me | Me | H | H | H | Cl | OEt | Cl |

**Tabelle, Fortsetzung**

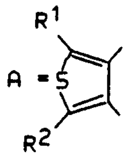 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | Me | H | H | H | Cl | OPr | Cl |
| Me | Me | H | H | H | Cl | OHex | Cl |
| Me | Me | H | H | H | Cl | OiPr | Cl |
| Me | Me | H | H | H | Cl | $OCH_2Ph$ | Cl |
| Me | Me | H | H | H | Cl | $O(CH_2)_2OMe$ | Cl |
| Me | Me | H | H | H | Cl | $O(CH_2)_2Ph$ | Cl |
| Me | Me | H | H | H | Cl | $OCH_2CF_3$ | Cl |
| Me | Me | H | H | H | Cl | $OCH_2(CF_2)_2CF_3$ | Cl |
| Me | Me | H | H | H | Cl | $OCH_2CF_2CF_3$ | Cl |
| Me | Me | H | H | H | Cl | $OCH_2CF_2CF_2H$ | Cl |
| OMe | H | H | H | H | Cl | OMe | Cl |
| OMe | H | H | H | H | Cl | OEt | Cl |
| OMe | H | H | H | H | Cl | OPr | Cl |
| OMe | H | H | H | H | Cl | OHex | Cl |
| OMe | H | H | H | H | Cl | OiPr | Cl |
| OMe | H | H | H | H | Cl | $OCH_2Ph$ | Cl |
| OMe | H | H | H | H | Cl | $O(CH_2)_2OMe$ | Cl |
| OMe | H | H | H | H | Cl | $O(CH_2)_2Ph$ | Cl |
| OMe | H | H | H | H | Cl | $OCH_2CF_3$ | Cl |
| OMe | H | H | H | H | Cl | $OCH_2(CF_2)_2CF_3$ | Cl |
| OMe | H | H | H | H | Cl | $OCH_2CF_2CF_3$ | Cl |
| OMe | H | H | H | H | Cl | $OCH_2CF_2CF_2H$ | Cl |

**Tabelle, Fortsetzung**

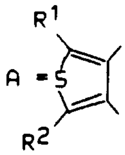 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | OMe | H | H | H | Cl | OMe | Cl |
| Me | OMe | H | H | H | Cl | OEt | Cl |
| Me | OMe | H | H | H | Cl | OPr | Cl |
| Me | OMe | H | H | H | Cl | OHex | Cl |
| Me | OMe | H | H | H | Cl | OiPr | Cl |
| Me | OMe | H | H | H | Cl | $OCH_2Ph$ | Cl |
| Me | OMe | H | H | H | Cl | $O(CH_2)_2OMe$ | Cl |
| Me | OMe | H | H | H | Cl | $O(CH_2)_2Ph$ | Cl |
| Me | OMe | H | H | H | Cl | $OCH_2CF_3$ | Cl |
| Me | OMe | H | H | H | Cl | $OCH_2(CF_2)_2CF_3$ | Cl |
| Me | OMe | H | H | H | Cl | $OCH_2CF_2CF_3$ | Cl |
| Me | OMe | H | H | H | Cl | $OCH_2CF_2CF_2H$ | Cl |
| OMe | OMe | H | H | H | Cl | OMe | Cl |
| OMe | OMe | H | H | H | Cl | OEt | Cl |
| OMe | OMe | H | H | H | Cl | OPr | Cl |
| OMe | OMe | H | H | H | Cl | OHex | Cl |
| OMe | OMe | H | H | H | Cl | OiPr | Cl |
| OMe | OMe | H | H | H | Cl | $OCH_2Ph$ | Cl |
| OMe | OMe | H | H | H | Cl | $O(CH_2)_2OMe$ | Cl |
| OMe | OMe | H | H | H | Cl | $O(CH_2)_2Ph$ | Cl |
| OMe | OMe | H | H | H | Cl | $OCH_2CF_3$ | Cl |
| OMe | OMe | H | H | H | Cl | $OCH_2(CF_2)_2CF_3$ | Cl |

**Tabelle, Fortsetzung**

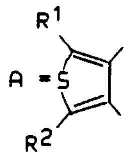 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | H | H | H | Cl | $OCH_2CF_2CF_3$ | Cl |
| OMe | OMe | H | H | H | Cl | $OCH_2CF_2CF_2H$ | Cl |
| H | H | H | H | H | Cl | OMe | H |
| H | H | H | H | H | Cl | OEt | H |
| H | H | H | H | H | Cl | OPr | H |
| H | H | H | H | H | Cl | OHex | H |
| H | H | H | H | H | Cl | OiPr | H |
| H | H | H | H | H | Cl | $OCH_2Ph$ | H |
| H | H | H | H | H | Cl | $O(CH_2)_2OMe$ | H |
| H | H | H | H | H | Cl | $O(CH_2)_2Ph$ | H |
| H | H | H | H | H | Cl | $OCH_2CF_3$ | H |
| H | H | H | H | H | Cl | $OCH_2(CF_2)_2CF_3$ | H |
| H | H | H | H | H | Cl | $OCH_2CF_2CF_3$ | H |
| H | H | H | H | H | Cl | $OCH_2CF_2CF_2H$ | H |
| Me | Me | H | H | H | Cl | OMe | H |
| Me | Me | H | H | H | Cl | OEt | H |
| Me | Me | H | H | H | Cl | OPr | H |
| Me | Me | H | H | H | Cl | OHex | H |
| Me | Me | H | H | H | Cl | OiPr | H |
| Me | Me | H | H | H | Cl | $OCH_2Ph$ | H |
| Me | Me | H | H | H | Cl | $O(CH_2)_2OMe$ | H |
| Me | Me | H | H | H | Cl | $O(CH_2)_2Ph$ | H |

Tabelle, Fortsetzung

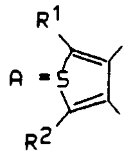 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | Me | H | H | H | Cl | $OCH_2CF_3$ | H |
| Me | Me | H | H | H | Cl | $OCH_2(CF_2)_2CF_3$ | H |
| Me | Me | H | H | H | Cl | $OCH_2CF_2CF_3$ | H |
| Me | Me | H | H | H | Cl | $OCH_2CF_2CF_2H$ | H |
| H | OMe | H | H | H | Cl | OMe | H |
| H | OMe | H | H | H | Cl | OEt | H |
| H | OMe | H | H | H | Cl | OPr | H |
| H | OMe | H | H | H | Cl | OHex | H |
| H | OMe | H | H | H | Cl | OiPr | H |
| H | OMe | H | H | H | Cl | $OCH_2Ph$ | H |
| H | OMe | H | H | H | Cl | $O(CH_2)_2OMe$ | H |
| H | OMe | H | H | H | Cl | $O(CH_2)_2Ph$ | H |
| H | OMe | H | H | H | Cl | $OCH_2CF_3$ | H |
| H | OMe | H | H | H | Cl | $OCH_2(CF_2)_2CF_3$ | H |
| H | OMe | H | H | H | Cl | $OCH_2CF_2CF_3$ | H |
| H | OMe | H | H | H | Cl | $OCH_2CF_2CF_2H$ | H |
| OMe | Me | H | H | H | Cl | OMe | H |
| OMe | Me | H | H | H | Cl | OEt | H |
| OMe | Me | H | H | H | Cl | OPr | H |
| OMe | Me | H | H | H | Cl | OHex | H |
| OMe | Me | H | H | H | Cl | OiPr | H |
| OMe | Me | H | H | H | Cl | $OCH_2Ph$ | H |

Tabelle, Fortsetzung

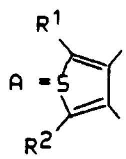

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | Me | H | H | H | Cl | $O(CH_2)_2OMe$ | H |
| OMe | Me | H | H | H | Cl | $O(CH_2)_2Ph$ | H |
| OMe | Me | H | H | H | Cl | $OCH_2CF_3$ | H |
| OMe | Me | H | H | H | Cl | $OCH_2(CF_2)_2CF_3$ | H |
| OMe | Me | H | H | H | Cl | $OCH_2CF_2CF_3$ | H |
| OMe | Me | H | H | H | Cl | $OCH_2CF_2CF_2H$ | H |
| OMe | OMe | H | H | H | Cl | OMe | H |
| OMe | OMe | H | H | H | Cl | OEt | H |
| OMe | OMe | H | H | H | Cl | OPr | H |
| OMe | OMe | H | H | H | Cl | OHex | H |
| OMe | OMe | H | H | H | Cl | OiPr | H |
| OMe | OMe | H | H | H | Cl | $OCH_2Ph$ | H |
| OMe | OMe | H | H | H | Cl | $O(CH_2)_2OMe$ | H |
| OMe | OMe | H | H | H | Cl | $O(CH_2)_2Ph$ | H |
| OMe | OMe | H | H | H | Cl | $OCH_2CF_3$ | H |
| OMe | OMe | H | H | H | Cl | $OCH_2(CF_2)_2CF_3$ | H |
| OMe | OMe | H | H | H | Cl | $OCH_2CF_2CF_3$ | H |
| OMe | OMe | H | H | H | Cl | $OCH_2CF_2CF_2H$ | H |
| H | H | H | H | H | Me | OMe | Cl |
| H | H | H | H | H | Me | OEt | Cl |
| H | H | H | H | H | Me | OPr | Cl |
| H | H | H | H | H | Me | OHex | Cl |

Tabelle, Fortsetzung

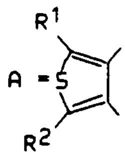 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | H | H | H | Me | OiPr | Cl |
| H | H | H | H | H | Me | $OCH_2Ph$ | Cl |
| H | H | H | H | H | Me | $O(CH_2)_2OMe$ | Cl |
| H | H | H | H | H | Me | $O(CH_2)_2Ph$ | Cl |
| H | H | H | H | H | Me | $OCH_2CF_3$ | Cl |
| H | H | H | H | H | Me | $OCH_2(CF_2)_2CF_3$ | Cl |
| H | H | H | H | H | Me | $OCH_2CF_2CF_3$ | Cl |
| H | H | H | H | H | Me | $OCH_2CF_2CF_2H$ | Cl |
| Me | Me | H | H | H | Me | OMe | Cl |
| Me | Me | H | H | H | Me | OEt | Cl |
| Me | Me | H | H | H | Me | OPr | Cl |
| Me | Me | H | H | H | Me | OHex | Cl |
| Me | Me | H | H | H | Me | OiPr | Cl |
| Me | Me | H | H | H | Me | $OCH_2Ph$ | Cl |
| Me | Me | H | H | H | Me | $O(CH_2)_2OMe$ | Cl |
| Me | Me | H | H | H | Me | $O(CH_2)_2Ph$ | Cl |
| Me | Me | H | H | H | Me | $OCH_2CF_3$ | Cl |
| Me | Me | H | H | H | Me | $OCH_2(CF_2)_2CF_3$ | Cl |
| Me | Me | H | H | H | Me | $OCH_2CF_2CF_3$ | Cl |
| Me | Me | H | H | H | Me | $OCH_2CF_2CF_2H$ | Cl |
| OMe | H | H | H | H | Me | OMe | Cl |
| OMe | H | H | H | H | Me | OEt | Cl |

**Tabelle, Fortsetzung**

A = 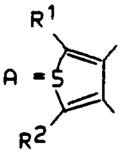 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | H | H | H | H | Me | OPr | Cl |
| OMe | H | H | H | H | Me | OHex | Cl |
| OMe | H | H | H | H | Me | OiPr | Cl |
| OMe | H | H | H | H | Me | $OCH_2Ph$ | Cl |
| OMe | H | H | H | H | Me | $O(CH_2)_2OMe$ | Cl |
| OMe | H | H | H | H | Me | $O(CH_2)_2Ph$ | Cl |
| OMe | H | H | H | H | Me | $OCH_2CF_3$ | Cl |
| OMe | H | H | H | H | Me | $OCH_2(CF_2)_2CF_3$ | Cl |
| OMe | H | H | H | H | Me | $OCH_2CF_2CF_3$ | Cl |
| OMe | H | H | H | H | Me | $OCH_2CF_2CF_2H$ | Cl |
| Me | OMe | H | H | H | Me | OMe | Cl |
| Me | OMe | H | H | H | Me | OEt | Cl |
| Me | OMe | H | H | H | Me | OPr | Cl |
| Me | OMe | H | H | H | Me | OHex | Cl |
| Me | OMe | H | H | H | Me | OiPr | Cl |
| Me | OMe | H | H | H | Me | $OCH_2Ph$ | Cl |
| Me | OMe | H | H | H | Me | $O(CH_2)_2OMe$ | Cl |
| Me | OMe | H | H | H | Me | $O(CH_2)_2Ph$ | Cl |
| Me | OMe | H | H | H | Me | $OCH_2CF_3$ | Cl |
| Me | OMe | H | H | H | Me | $OCH_2(CF_2)_2CF_3$ | Cl |
| Me | OMe | H | H | H | Me | $OCH_2CF_2CF_3$ | Cl |
| Me | OMe | H | H | H | Me | $OCH_2CF_2CF_2H$ | Cl |

**Tabelle, Fortsetzung**

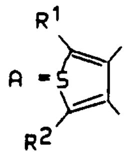

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | H | H | H | Me | OMe | Cl |
| OMe | OMe | H | H | H | Me | OEt | Cl |
| OMe | OMe | H | H | H | Me | OPr | Cl |
| OMe | OMe | H | H | H | Me | OHex | Cl |
| OMe | OMe | H | H | H | Me | OiPr | Cl |
| OMe | OMe | H | H | H | Me | $OCH_2Ph$ | Cl |
| OMe | OMe | H | H | H | Me | $O(CH_2)_2OMe$ | Cl |
| OMe | OMe | H | H | H | Me | $O(CH_2)_2Ph$ | Cl |
| OMe | OMe | H | H | H | Me | $OCH_2CF_3$ | Cl |
| OMe | OMe | H | H | H | Me | $OCH_2(CF_2)_2CF_3$ | Cl |
| OMe | OMe | H | H | H | Me | $OCH_2CF_2CF_3$ | Cl |
| OMe | OMe | H | H | H | Me | $OCH_2CF_2CF_2H$ | Cl |
| H | H | H | H | H | Cl | OMe | Me |
| H | H | H | H | H | Cl | OEt | Me |
| H | H | H | H | H | Cl | OPr | Me |
| H | H | H | H | H | Cl | OHex | Me |
| H | H | H | H | H | Cl | OiPr | Me |
| H | H | H | H | H | Cl | $OCH_2Ph$ | Me |
| H | H | H | H | H | Cl | $O(CH_2)_2OMe$ | Me |
| H | H | H | H | H | Cl | $O(CH_2)_2Ph$ | Me |
| H | H | H | H | H | Cl | $OCH_2CF_3$ | Me |
| H | H | H | H | H | Cl | $OCH_2(CF_2)_2CF_3$ | Me |

Tabelle, Fortsetzung

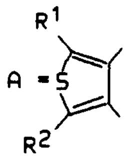 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | H | H | H | Cl | $OCH_2CF_2CF_3$ | Me |
| H | H | H | H | H | Cl | $OCH_2CF_2CF_2H$ | Me |
| Me | Me | H | H | H | Cl | OMe | Me |
| Me | Me | H | H | H | Cl | OEt | Me |
| Me | Me | H | H | H | Cl | OPr | Me |
| Me | Me | H | H | H | Cl | OHex | Me |
| Me | Me | H | H | H | Cl | OiPr | Me |
| Me | Me | H | H | H | Cl | $OCH_2Ph$ | Me |
| Me | Me | H | H | H | Cl | $O(CH_2)_2OMe$ | Me |
| Me | Me | H | H | H | Cl | $O(CH_2)_2Ph$ | Me |
| Me | Me | H | H | H | Cl | $OCH_2CF_3$ | Me |
| Me | Me | H | H | H | Cl | $OCH_2(CF_2)_2CF_3$ | Me |
| Me | Me | H | H | H | Cl | $OCH_2CF_2CF_3$ | Me |
| Me | Me | H | H | H | Cl | $OCH_2CF_2CF_2H$ | Me |
| H | OMe | H | H | H | Cl | OMe | Me |
| H | OMe | H | H | H | Cl | OEt | Me |
| H | OMe | H | H | H | Cl | OPr | Me |
| H | OMe | H | H | H | Cl | OHex | Me |
| H | OMe | H | H | H | Cl | OiPr | Me |
| H | OMe | H | H | H | Cl | $OCH_2Ph$ | Me |
| H | OMe | H | H | H | Cl | $O(CH_2)_2OMe$ | Me |
| H | OMe | H | H | H | Cl | $O(CH_2)_2Ph$ | Me |

**Tabelle, Fortsetzung**

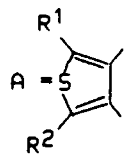

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | OMe | H | H | H | Cl | $OCH_2CF_3$ | Me |
| H | OMe | H | H | H | Cl | $OCH_2(CF_2)_2CF_3$ | Me |
| H | OMe | H | H | H | Cl | $OCH_2CF_2CF_3$ | Me |
| H | OMe | H | H | H | Cl | $OCH_2CF_2CF_2H$ | Me |
| OMe | OMe | H | H | H | Cl | OMe | Me |
| OMe | OMe | H | H | H | Cl | OEt | Me |
| OMe | OMe | H | H | H | Cl | OPr | Me |
| OMe | OMe | H | H | H | Cl | OHex | Me |
| OMe | OMe | H | H | H | Cl | OiPr | Me |
| OMe | OMe | H | H | H | Cl | $OCH_2Ph$ | Me |
| OMe | OMe | H | H | H | Cl | $O(CH_2)_2OMe$ | Me |
| OMe | OMe | H | H | H | Cl | $O(CH_2)_2Ph$ | Me |
| OMe | OMe | H | H | H | Cl | $OCH_2CF_3$ | Me |
| OMe | OMe | H | H | H | Cl | $OCH_2(CF_2)_2CF_3$ | Me |
| OMe | OMe | H | H | H | Cl | $OCH_2CF_2CF_3$ | Me |
| OMe | OMe | H | H | H | Cl | $OCH_2CF_2CF_2H$ | Me |
| OEt | H | H | H | H | Cl | OMe | Me |
| OEt | H | H | H | H | Cl | OEt | Me |
| OEt | H | H | H | H | Cl | OPr | Me |
| OEt | H | H | H | H | Cl | OHex | Me |
| OEt | H | H | H | H | Cl | OiPr | Me |
| OEt | H | H | H | H | Cl | $OCH_2Ph$ | Me |

Tabelle, Fortsetzung

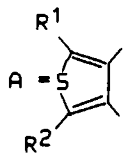

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OEt | H | H | H | H | Cl | $O(CH_2)_2OMe$ | Me |
| OEt | H | H | H | H | Cl | $O(CH_2)_2Ph$ | Me |
| OEt | H | H | H | H | Cl | $OCH_2CF_3$ | Me |
| OEt | H | H | H | H | Cl | $OCH_2(CF_2)_2CF_3$ | Me |
| OEt | H | H | H | H | Cl | $OCH_2CF_2CF_3$ | Me |
| OEt | H | H | H | H | Cl | $OCH_2CF_2CF_2H$ | Me |
| H | H | H | H | H | Me | OMe | Br |
| H | H | H | H | H | Me | OEt | Br |
| H | H | H | H | H | Me | $OCH_2Ph$ | Br |
| H | H | H | H | H | Me | $OCH_2CF_3$ | Br |
| Me | H | H | H | H | Me | OMe | Br |
| Me | Me | H | H | H | Me | OMe | Br |
| Me | Me | H | H | H | Me | OEt | Br |
| Me | Me | H | H | H | Me | OiPr | Br |
| H | OMe | H | H | H | Me | OMe | Br |
| H | OMe | H | H | H | Me | OEt | Br |
| H | OMe | H | H | H | Me | OiPr | Br |
| H | OMe | H | H | H | Me | $OCH_2Ph$ | Br |
| H | OMe | H | H | H | Me | $OCH_2CF_3$ | Br |
| H | OMe | H | H | H | Me | $OCH_2CH_2CF_3$ | Br |
| Me | OMe | H | H | H | Me | OMe | Br |
| OMe | OMe | H | H | H | Me | OMe | Br |

**Tabelle, Fortsetzung**

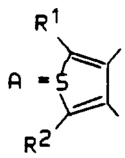

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | H | H | H | Me | OPr | Br |
| OMe | OMe | H | H | H | Me | $OCH_2Ph$ | Br |
| OMe | OMe | H | H | H | Me | $OCH_2CF_3$ | Br |
| H | H | H | H | H | Cl | OMe | Br |
| H | H | H | H | H | Cl | OPr | Br |
| H | H | H | H | H | Cl | $OCH_2Ph$ | Br |
| Me | H | H | H | H | Br | OEt | Br |
| Me | Me | H | H | H | Br | OMe | Br |
| Me | Me | H | H | H | Br | $OCH_2Ph$ | Br |
| H | OMe | H | H | H | Br | OMe | Cl |
| H | OMe | H | H | H | Br | OPr | Cl |
| H | OMe | H | H | H | Br | $OCH_2Ph$ | Cl |
| H | OMe | H | H | H | Br | $OCH_2CF_3$ | Cl |
| Me | OMe | H | H | H | Br | OMe | Cl |
| OMe | OMe | H | H | H | Br | OMe | Cl |
| OMe | OMe | H | H | H | Br | OEt | Cl |
| H | H | H | H | H | Br | $OCH_2Ph$ | H |
| H | H | H | H | H | Br | $OCH_2CF_3$ | H |
| H | H | H | H | H | Br | $OCH_2CF_2CF_3$ | H |
| Me | Me | H | H | H | Br | OMe | H |
| Me | Me | H | H | H | Br | $OCH_2Ph$ | H |
| OMe | H | H | H | H | Br | OMe | H |

Tabelle, Fortsetzung

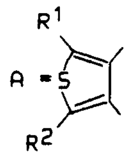 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | H | H | H | H | Br | $OCH_2Ph$ | H |
| OMe | H | H | H | H | Br | $OCH_2CF_3$ | H |
| OMe | H | H | H | H | Br | $OCH_2CF_2CF_3$ | H |
| Me | OMe | H | H | H | Br | OEt | H |
| Me | OMe | H | H | H | Br | $OCH_2CF_2CF_3$ | H |
| OMe | OMe | H | H | H | Br | OMe | H |
| OMe | OMe | H | H | H | Br | OEt | H |
| OMe | OMe | H | H | H | Br | OiPr | H |
| OMe | OMe | H | H | H | Br | $OCH_2Ph$ | H |
| OMe | OMe | H | H | H | Br | $OCH_2CF_3$ | H |
| H | H | H | H | H | Br | OMe | Me |
| H | H | H | H | H | Br | OEt | Me |
| H | H | H | H | H | Br | OiPr | Me |
| H | H | H | H | H | Br | $OCH_2Ph$ | Me |
| H | H | H | H | H | Br | $OCH_2CF_2CF_3$ | Me |
| Me | Me | H | H | H | Br | OMe | Me |
| Me | Me | H | H | H | Br | OEt | Me |
| Me | Me | H | H | H | Br | $OCH_2CF_3$ | Me |
| Me | Me | H | H | H | Br | $OCH_2CF_2CF_3$ | Me |
| OMe | H | H | H | H | Br | OMe | Me |
| OMe | H | H | H | H | Br | OiPr | Me |
| OMe | H | H | H | H | Br | $OCH_2Ph$ | Me |

**Tabelle, Fortsetzung**

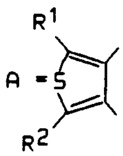 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | H | H | H | H | Br | $OCH_2CF_3$ | Me |
| OMe | H | H | H | H | Br | OMe | Me |
| OMe | OMe | H | H | H | Br | OMe | Me |
| OMe | OMe | H | H | H | Br | $OCH_2Ph$ | Me |
| H | H | $CH_2OAc$ | H | H | Me | OMe | Cl |
| H | H | $CH_2OAc$ | H | H | Me | OEt | Cl |
| H | H | $CH_2OAc$ | H | H | Me | $OCH_2Ph$ | Cl |
| H | H | $CH_2OAc$ | H | H | Me | $OCH_2CF_3$ | Cl |
| Me | H | $CH_2OAc$ | H | H | Me | OMe | Cl |
| Me | Me | $CH_2OAc$ | H | H | Me | OMe | Cl |
| Me | Me | $CH_2OAc$ | H | H | Me | OEt | Cl |
| Me | Me | $CH_2OAc$ | H | H | Me | OiPr | Cl |
| H | OMe | $CH_2OAc$ | H | H | Me | OMe | Cl |
| H | OMe | $CH_2OAc$ | H | H | Me | OEt | Cl |
| H | OMe | $CH_2OAc$ | H | H | Me | OiPr | Cl |
| H | OMe | $CH_2OAc$ | H | H | Me | $OCH_2Ph$ | Cl |
| H | OMe | $CH_2OAc$ | H | H | Me | $OCH_2CF_3$ | Cl |
| H | OMe | $CH_2OAc$ | H | H | Me | $OCH_2CH_2CF_3$ | Cl |
| Me | OMe | $CH_2OAc$ | H | H | Me | OMe | Cl |
| OMe | OMe | $CH_2OAc$ | H | H | Me | OMe | Cl |
| OMe | OMe | $CH_2OAc$ | H | H | Me | Opr | Cl |
| OMe | OMe | $CH_2OAc$ | H | H | Me | $OCH_2Ph$ | Cl |

Tabelle, Fortsetzung

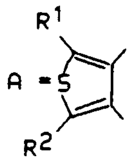

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | $CH_2OAc$ | H | H | Me | $OCH_2CF_3$ | Cl |
| H | H | $CH_2OAc$ | H | H | Cl | OMe | Cl |
| H | H | $CH_2OAc$ | H | H | Cl | OPr | Cl |
| H | H | $CH_2OAc$ | H | H | Cl | $OCH_2Ph$ | Cl |
| Me | H | $CH_2OAc$ | H | H | Cl | OEt | Cl |
| Me | Me | $CH_2OAc$ | H | H | Cl | OMe | Cl |
| Me | Me | $CH_2OAc$ | H | H | Cl | $OCH_2Ph$ | Cl |
| H | OMe | $CH_2OAc$ | H | H | Cl | OMe | Cl |
| H | OMe | $CH_2OAc$ | H | H | Cl | OPr | Cl |
| H | OMe | $CH_2OAc$ | H | H | Cl | $OCH_2Ph$ | Cl |
| H | OMe | $CH_2OAc$ | H | H | Cl | $OCH_2CF_3$ | Cl |
| Me | OMe | $CH_2OAc$ | H | H | Cl | OMe | Cl |
| OMe | OMe | $CH_2OAc$ | H | H | Cl | OMe | Cl |
| OMe | OMe | $CH_2OAc$ | H | H | Cl | OEt | Cl |
| H | H | $CH_2OAc$ | H | H | Cl | $OCH_2Ph$ | H |
| H | H | $CH_2OAc$ | H | H | Cl | $OCH_2CF_3$ | H |
| H | H | $CH_2OAc$ | H | H | Cl | $OCH_2CF_2CF_3$ | H |
| Me | Me | $CH_2OAc$ | H | H | Cl | OMe | H |
| Me | Me | $CH_2OAc$ | H | H | Cl | $OCH_2Ph$ | H |
| OMe | H | $CH_2OAc$ | H | H | Cl | OMe | H |
| OMe | H | $CH_2OAc$ | H | H | Cl | $OCH_2Ph$ | H |

**Tabelle, Fortsetzung**

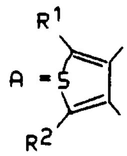 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | H | $CH_2OAc$ | H | H | Cl | $OCH_2CF_2CF_3$ | H |
| Me | OMe | $CH_2OAc$ | H | H | Cl | OEt | H |
| Me | OMe | $CH_2OAc$ | H | H | Cl | $OCH_2CF_2CF_3$ | H |
| OMe | OMe | $CH_2OAc$ | H | H | Cl | OMe | H |
| OMe | OMe | $CH_2OAc$ | H | H | Cl | OEt | H |
| OMe | OMe | $CH_2OAc$ | H | H | Cl | OiPr | H |
| OMe | OMe | $CH_2OAc$ | H | H | Cl | $OCH_2Ph$ | H |
| OMe | OMe | $CH_2OAc$ | H | H | Cl | $OCH_2CF_3$ | H |
| H | H | $CH_2OAc$ | H | H | Cl | OMe | Me |
| H | H | $CH_2OAc$ | H | H | Cl | OEt | Me |
| H | H | $CH_2OAc$ | H | H | Cl | OiPr | Me |
| H | H | $CH_2OAc$ | H | H | Cl | $OCH_2Ph$ | Me |
| H | H | $CH_2OAc$ | H | H | Cl | $OCH_2CF_2CF_3$ | Me |
| Me | Me | $CH_2OAc$ | H | H | Cl | OMe | Me |
| Me | Me | $CH_2OAc$ | H | H | Cl | OEt | Me |
| Me | Me | $CH_2OAc$ | H | H | Cl | $OCH_2CF_3$ | Me |
| Me | Me | $CH_2OAc$ | H | H | Cl | $OCH_2CF_2CF_3$ | Me |
| OMe | H | $CH_2OAc$ | H | H | Cl | OMe | Me |
| OMe | H | $CH_2OAc$ | H | H | Cl | OiPr | Me |
| OMe | H | $CH_2OAc$ | H | H | Cl | $OCH_2Ph$ | Me |
| OMe | H | $CH_2OAc$ | H | H | Cl | $OCH_2CF_3$ | Me |
| OMe | Me | $CH_2OAc$ | H | H | Cl | OMe | Me |

**Tabelle, Fortsetzung**

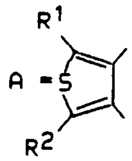 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | $CH_2OAc$ | H | H | Cl | OMe | Me |
| OMe | OMe | $CH_2OAc$ | H | H | Cl | $OCH_2Ph$ | Me |
| OMe | OMe | $CH_2OAc$ | H | H | Cl | OiPr | Cl |
| OMe | OMe | $CH_2OAc$ | H | H | Cl | $OCH_2Ph$ | Cl |
| OMe | OMe | $CH_2OAc$ | H | H | Cl | $OCH_2CF_3$ | Cl |
| H | H | $CH_2OAc$ | H | H | Me | OMe | Me |
| H | H | $CH_2OAc$ | H | H | Me | $OCH_2Ph$ | Me |
| Me | Me | $CH_2OAc$ | H | H | Me | OMe | Me |
| Me | Me | $CH_2OAc$ | H | H | Me | $OCH_2CF_3$ | Me |
| OMe | H | $CH_2OAc$ | H | H | Me | OMe | Me |
| OMe | H | $CH_2OAc$ | H | H | Me | Opr | Me |
| OMe | Me | $CH_2OAc$ | H | H | Me | OMe | Me |
| OMe | Me | $CH_2OAc$ | H | H | Me | $O(CH_2)_2OMe$ | Me |
| OMe | OMe | $CH_2OAc$ | H | H | Me | OMe | Me |
| OMe | OMe | $CH_2OAc$ | H | H | Me | $OCH_2CF_3$ | Me |
| H | H | $CH_2OAc$ | H | H | Cl | OMe | H |
| H | H | $CH_2OAc$ | H | H | Cl | OEt | H |
| H | H | $CH_2OAc$ | H | H | Cl | OiPr | H |
| OMe | OMe | $CH_2OAc$ | H | H | Cl | $OCH_2CF_3$ | Me |
| H | H | $CH_2OAc$ | H | H | H | Me | H |
| Me | H | $CH_2OAc$ | H | H | H | iPr | H |

**Tabelle, Fortsetzung**

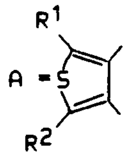

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | Me | $CH_2OAc$ | H | H | H | Me | H |
| Me | Me | $CH_2OAc$ | H | H | Me | iPr | H |
| OMe | H | $CH_2OAc$ | H | H | H | Me | H |
| OMe | H | $CH_2OAc$ | H | H | Me | Me | H |
| OMe | H | $CH_2OAc$ | H | H | Me | iPr | H |
| OMe | Me | $CH_2OAc$ | H | H | Me | Me | H |
| OMe | OMe | $CH_2OAc$ | H | H | H | Me | H |
| OMe | OMe | $CH_2OAc$ | H | H | Me | Me | H |
| OMe | OMe | $CH_2OAc$ | H | H | H | Me | Me |
| H | H | $CH_2OAc$ | H | H | Me | Me | Me |
| H | H | $CH_2OAc$ | H | H | Cl | Me | H |
| Me | Me | $CH_2OAc$ | H | H | Me | Me | Me |
| Me | Me | $CH_2OAc$ | H | H | Cl | Me | H |
| Me | Me | $CH_2OAc$ | H | H | H | Me | Cl |
| OMe | H | $CH_2OAc$ | H | H | Me | Me | Me |
| OMe | H | $CH_2OAc$ | H | H | Cl | Me | H |
| OMe | OMe | $CH_2OAc$ | H | H | Me | Me | Me |
| OMe | OMe | $CH_2OAc$ | H | H | Cl | Me | H |
| OMe | OMe | $CH_2OAc$ | H | H | Me | Et | Me |
| H | H | $CH_2OAc$ | H | H | Cl | Me | Cl |
| H | H | $CH_2OAc$ | H | H | Cl | Me | Me |
| H | H | $CH_2OAc$ | H | H | Cl | Et | Me |

**Tabelle, Fortsetzung**

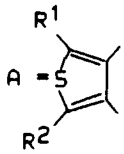 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | Me | $CH_2OAc$ | H | H | Cl | Me | Me |
| Et | Et | $CH_2OAc$ | H | H | Me | Me | Cl |
| OMe | H | $CH_2OAc$ | H | H | Cl | Me | Cl |
| OMe | H | $CH_2OAc$ | H | H | Cl | Me | Me |
| OMe | Me | $CH_2OAc$ | H | H | Me | Me | Cl |
| OMe | OMe | $CH_2OAc$ | H | H | Cl | Me | Me |
| OMe | OMe | $CH_2OAc$ | H | H | Me | Me | Cl |
| OEt | OEt | $CH_2OAc$ | H | H | Me | Me | Cl |
| H | H | $CH_2OAc$ | H | H | Me | 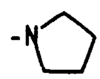 | H |
| Me | Me | $CH_2OAc$ | H | H | Me | 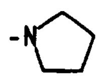 | H |
| OMe | H | $CH_2OAc$ | H | H | Me | 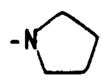 | H |
| OEt | H | $CH_2OAc$ | H | H | H | $-NMe_2$ | Me |
| OMe | OMe | $CH_2OAc$ | H | H | $CH_3$ | $-NMe_2$ | H |
| OEt | OEt | $CH_2OAc$ | H | H | H | $-NMe_2$ | Me |
| Me | H | $CH_2OAc$ | H | H | Cl | 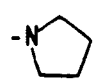 | H |
| Me | Me | $CH_2OAc$ | H | H | Cl | $-NMe_2$ | H |
| OMe | H | $CH_2OAc$ | H | H | Cl | $-NMe_2$ | H |
| OMe | OMe | $CH_2OAc$ | H | H | Cl | $-NMe_2$ | H |
| OMe | OMe | $CH_2OAc$ | H | H | Cl | 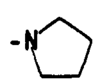 | H |

**Tabelle, Fortsetzung**

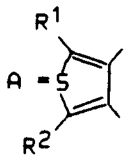 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | $CH_2OAc$ | H | H | Cl | $-NMe_2$ | Cl |
| Me | H | $CH_2OAc$ | H | H | Cl | 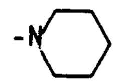 | Cl |
| Et | H | $CH_2OAc$ | H | H | Cl | $-NMe_2$ | $CH_3$ |
| Me | Me | $CH_2OAc$ | H | H | Cl | 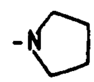 | Cl |
| OMe | H | $CH_2OAc$ | H | H | Cl | $-NMe_2$ | Cl |
| $OCH_2Ph$ | H | $CH_2OAc$ | H | H | Cl | 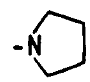 | Cl |
| OEt | H | $CH_2OAc$ | H | H | Cl | 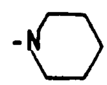 | Cl |
| OMe | H | $CH_2OAc$ | H | H | Cl | $-NMe_2$ | Me |
| OMe | H | $CH_2OAc$ | H | H | Cl | $NMe_2$ | Cl |
| OMe | OMe | $CH_2OAc$ | H | H | Cl | 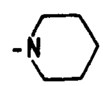 | Cl |
| OMe | OMe | $CH_2OAc$ | H | H | Cl | 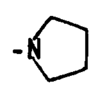 | Me |
| OEt | OEt | $CH_2OAc$ | H | H | Me | 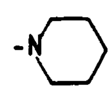 | Cl |
| H | H | $CH(CH_3)OAc$ | H | H | Me | OMe | Cl |
| H | H | $CH(CH_3)OAc$ | H | H | Me | OEt | Cl |
| H | H | $CH(CH_3)OAc$ | H | H | Me | $OCH_2Ph$ | Cl |
| H | H | $CH(CH_3)OAc$ | H | H | Me | $OCH_2CF_3$ | Cl |

**Tabelle, Fortsetzung**

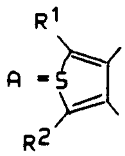

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | H | $CH(CH_3)OAc$ | H | H | Me | OMe | Cl |
| Me | Me | $CH(CH_3)OAc$ | H | H | Me | OMe | Cl |
| Me | Me | $CH(CH_3)OAc$ | H | H | Me | OEt | Cl |
| Me | Me | $CH(CH_3)OAc$ | H | H | Me | OiPr | Cl |
| H | OMe | $CH(CH_3)OAc$ | H | H | Me | OMe | Cl |
| H | OMe | $CH(CH_3)OAc$ | H | H | Me | OEt | Cl |
| H | OMe | $CH(CH_3)OAc$ | H | H | Me | OiPr | Cl |
| H | OMe | $CH(CH_3)OAc$ | H | H | Me | $OCH_2Ph$ | Cl |
| H | OMe | $CH(CH_3)OAc$ | H | H | Me | $OCH_2CF_3$ | Cl |
| H | OMe | $CH(CH_3)OAc$ | H | H | Me | $OCH_2CH_2CF_3$ | Cl |
| Me | OMe | $CH(CH_3)OAc$ | H | H | Me | OMe | Cl |
| OMe | OMe | $CH(CH_3)OAc$ | H | H | Me | OMe | Cl |
| OMe | OMe | $CH(CH_3)OAc$ | H | H | Me | Opr | Cl |
| OMe | OMe | $CH(CH_3)OAc$ | H | H | Me | $OCH_2Ph$ | Cl |
| OMe | OMe | $CH(CH_3)OAc$ | H | H | Me | $OCH_2CF_3$ | Cl |
| H | H | $CH(CH_3)OAc$ | H | H | Cl | OMe | Cl |
| H | H | $CH(CH_3)OAc$ | H | H | Cl | OPr | Cl |
| H | H | $CH(CH_3)OAc$ | H | H | Cl | $OCH_2Ph$ | Cl |
| Me | H | $CH(CH_3)OAc$ | H | H | Cl | OEt | Cl |
| Me | Me | $CH(CH_3)OAc$ | H | H | Cl | OMe | Cl |
| Me | Me | $CH(CH_3)OAc$ | H | H | Cl | $OCH_2Ph$ | Cl |
| H | OMe | $CH(CH_3)OAc$ | H | H | Cl | OMe | Cl |

**Tabelle, Fortsetzung**

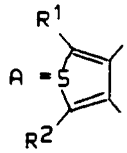 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | OMe | $CH(CH_3)OAc$ | H | H | Cl | OPr | Cl |
| H | OMe | $CH(CH_3)OAc$ | H | H | Cl | $OCH_2Ph$ | Cl |
| H | OMe | $CH(CH_3)OAc$ | H | H | Cl | $OCH_2CF_3$ | Cl |
| Me | OMe | $CH(CH_3)OAc$ | H | H | Cl | OMe | Cl |
| OMe | OMe | $CH(CH_3)OAc$ | H | H | Cl | OMe | Cl |
| OMe | OMe | $CH(CH_3)OAc$ | H | H | Cl | OEt | Cl |
| H | H | $CH(CH_3)OAc$ | H | H | Cl | $OCH_2Ph$ | H |
| H | H | $CH(CH_3)OAc$ | H | H | Cl | $OCH_2CF_3$ | H |
| H | H | $CH(CH_3)OAc$ | H | H | Cl | $OCH_2CF_2CF_3$ | H |
| Me | Me | $CH(CH_3)OAc$ | H | H | Cl | OMe | H |
| Me | Me | $CH(CH_3)OAc$ | H | H | Cl | $OCH_2Ph$ | H |
| OMe | H | $CH(CH_3)OAc$ | H | H | Cl | OMe | H |
| OMe | H | $CH(CH_3)OAc$ | H | H | Cl | $OCH_2Ph$ | H |
| OMe | H | $CH(CH_3)OAc$ | H | H | Cl | $OCH_2CF_3$ | H |
| OMe | H | $CH(CH_3)OAc$ | H | H | Cl | $OCH_2CF_2CF_3$ | H |
| Me | OMe | $CH(CH_3)OAc$ | H | H | Cl | OEt | H |
| Me | OMe | $CH(CH_3)OAc$ | H | H | Cl | $OCH_2CF_2CF_3$ | H |
| OMe | OMe | $CH(CH_3)OAc$ | H | H | Cl | OMe | H |
| OMe | OMe | $CH(CH_3)OAc$ | H | H | Cl | OEt | H |
| OMe | OMe | $CH(CH_3)OAc$ | H | H | Cl | OiPr | H |
| OMe | OMe | $CH(CH_3)OAc$ | H | H | Cl | $OCH_2Ph$ | H |
| OMe | OMe | $CH(CH_3)OAc$ | H | H | Cl | $OCH_2CF_3$ | H |

**Tabelle, Fortsetzung**

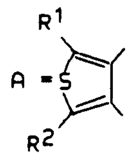

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | $CH(CH_3)OAc$ | H | H | Cl | OMe | Me |
| H | H | $CH(CH_3)OAc$ | H | H | Cl | OEt | Me |
| H | H | $CH(CH_3)OAc$ | H | H | Cl | OiPr | Me |
| H | H | $CH(CH_3)OAc$ | H | H | Cl | $OCH_2Ph$ | Me |
| H | H | $CH(CH_3)OAc$ | H | H | Cl | $OCH[_2CF_2CF_3$ | Me |
| Me | Me | $CH(CH_3)OAc$ | H | H | Cl | OMe | Me |
| Me | Me | $CH(CH_3)OAc$ | H | H | Cl | OEt | Me |
| Me | Me | $CH(CH_3)OAc$ | H | H | Cl | $OCH_2CF_3$ | Me |
| Me | Me | $CH(CH_3)OAc$ | H | H | Cl | $OCH_2CF_2CF_3$ | Me |
| OMe | H | $CH(CH_3)OAc$ | H | H | Cl | OMe | Me |
| OMe | H | $CH(CH_3)OAc$ | H | H | Cl | OiPr | Me |
| OMe | H | $CH(CH_3)OAc$ | H | H | Cl | $OCH_2Ph$ | Me |
| OMe | H | $CH(CH_3)OAc$ | H | H | Cl | $OCH_2CF_3$ | Me |
| OMe | Me | $CH(CH_3)OAc$ | H | H | Cl | OMe | Me |
| OMe | OMe | $CH(CH_3)OAc$ | H | H | Cl | OMe | Me |
| OMe | OMe | $CH(CH_3)OAc$ | H | H | Cl | $OCH_2Ph$ | Me |
| OMe | OMe | $CH(CH_3)OAc$ | H | H | Cl | OiPr | Cl |
| OMe | OMe | $CH(CH_3)OAc$ | H | H | Cl | $OCH_2Ph$ | Cl |
| OMe | OMe | $CH(CH_3)OAc$ | H | H | Cl | $OCH_2CF_3$ | Cl |
| H | H | $CH(CH_3)OAc$ | H | H | Me | OMe | Me |
| H | H | $CH(CH_3)OAc$ | H | H | Me | $OCH_2Ph$ | Me |
| Me | Me | $CH(CH_3)OAc$ | H | H | Me | OMe | Me |

**Tabelle, Fortsetzung**

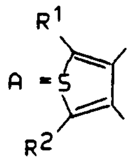 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | Me | $CH(CH_3)OAc$ | H | H | Me | $OCH_2CF_3$ | Me |
| OMe | H | $CH(CH_3)OAc$ | H | H | Me | OMe | Me |
| OMe | H | $CH(CH_3)OAc$ | H | H | Me | OPr | Me |
| OMe | Me | $CH(CH_3)OAc$ | H | H | Me | OMe | Me |
| OMe | Me | $CH(CH_3)OAc$ | H | H | Me | $O(CH_2)_2OMe$ | Me |
| OMe | OMe | $CH(CH_3)OAc$ | H | H | Me | OMe | Me |
| OMe | OMe | $CH(CH_3)OAc$ | H | H | Me | $OCH_2CF_3$ | Me |
| H | H | $CH(CH_3)OAc$ | H | H | Cl | OMe | H |
| H | H | $CH(CH_3)OAc$ | H | H | Cl | OEt | H |
| H | H | $CH(CH_3)OAc$ | H | H | Cl | OiPr | H |
| OMe | OMe | $CH(CH_3)OAc$ | H | H | Cl | $OCH_2CF_3$ | Me |
| H | H | $CH(CH_3)OAc$ | H | H | H | Me | H |
| Me | H | $CH(CH_3)OAc$ | H | H | H | iPr | H |
| Me | Me | $CH(CH_3)OAc$ | H | H | Me | Me | H |
| Me | Me | $CH(CH_3)OAc$ | H | H | Me | iPr | H |
| OMe | H | $CH(CH_3)OAc$ | H | H | H | Me | H |
| OMe | H | $CH(CH_3)OAc$ | H | H | Me | Me | H |
| OMe | H | $CH(CH_3)OAc$ | H | H | Me | iPr | H |
| OMe | Me | $CH(CH_3)OAc$ | H | H | Me | Me | H |
| OMe | OMe | $CH(CH_3)OAc$ | H | H | H | Me | H |
| OMe | OMe | $CH(CH_3)OAc$ | H | H | Me | Me | H |

**Tabelle, Fortsetzung**

A = 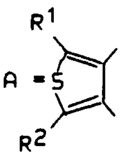 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | $CH(CH_3)OAc$ | H | H | H | Me | Me |
| H | H | $CH(CH_3)OAc$ | H | H | Me | Me | Me |
| H | H | $CH(CH_3)OAc$ | H | H | Cl | Me | H |
| Me | Me | $CH(CH_3)OAc$ | H | H | Me | Me | Me |
| Me | Me | $CH(CH_3)OAc$ | H | H | Cl | Me | H |
| Me | Me | $CH(CH_3)OAc$ | H | H | H | Me | Cl |
| OMe | H | $CH(CH_3)OAc$ | H | H | Me | Me | Me |
| OMe | H | $CH(CH_3)OAc$ | H | H | Cl | Me | H |
| OMe | OMe | $CH(CH_3)OAc$ | H | H | Me | Me | Me |
| OMe | OMe | $CH(CH_3)OAc$ | H | H | Cl | Me | H |
| OMe | OMe | $CH(CH_3)OAc$ | H | H | Me | Et | Me |
| H | H | $CH(CH_3)OAc$ | H | H | Cl | Me | Cl |
| H | H | $CH(CH_3)OAc$ | H | H | Cl | Me | Me |
| H | H | $CH(CH_3)OAc$ | H | H | Cl | Et | Me |
| Me | Me | $CH(CH_3)OAc$ | H | H | Cl | Me | Me |
| Et | Et | $CH(CH_3)OAc$ | H | H | Me | Me | Cl |
| OMe | H | $CH(CH_3)OAc$ | H | H | Cl | Me | Cl |
| OMe | H | $CH(CH_3)OAc$ | H | H | Cl | Me | Me |
| OMe | Me | $CH(CH_3)OAc$ | H | H | Me | Me | Cl |
| OMe | OMe | $CH(CH_3)OAc$ | H | H | Cl | Me | Me |
| OMe | OMe | $CH(CH_3)OAc$ | H | H | Me | Me | Cl |
| OEt | OEt | $CH(CH_3)OAc$ | H | H | Me | Me | Cl |

**Tabelle, Fortsetzung**

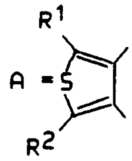 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | CH(CH$_3$)OAc | H | H | Me | 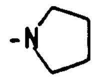 | H |
| Me | Me | CH(CH$_3$)OAc | H | H | Me | 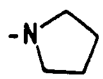 | H |
| OMe | H | CH(CH$_3$)OAc | H | H | Me | 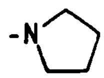 | H |
| OEt | H | CH(CH$_3$)OAc | H | H | H | -NMe$_2$ | Me |
| OMe | OMe | CH(CH$_3$)OAc | H | H | CH$_3$ | -NMe$_2$ | H |
| OEt | OEt | CH(CH$_3$)OAc | H | H | H | -NMe$_2$ | Me |
| Me | H | CH(CH$_3$)OAc | H | H | Cl | 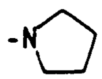 | H |
| Me | Me | CH(CH$_3$)OAc | H | H | Cl | -NMe$_2$ | H |
| OMe | H | CH(CH$_3$)OAc | H | H | Cl | -NMe$_2$ | H |
| OMe | OMe | CH(CH$_3$)OAc | H | H | Cl | -NMe$_2$ | H |
| OMe | OMe | CH(CH$_3$)OAc | H | H | Cl | 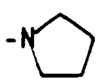 | H |
| H | H | CH(CH$_3$)OAc | H | H | Cl | -NMe$_2$ | Cl |
| Me | H | CH(CH$_3$)OAc | H | H | Cl | 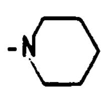 | Cl |
| Et | H | CH(CH$_3$)OAc | H | H | Cl | -NMe$_2$ | CH$_3$ |
| Me | Me | CH(CH$_3$)OAc | H | H | Cl | 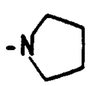 | Cl |
| OMe | H | CH(CH$_3$)OAc | H | H | Cl | -NMe$_2$ | Cl |

**Tabelle, Fortsetzung**

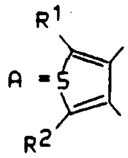 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| $OCH_2Ph$ | H | $CH(CH_3)OAc$ | H | H | Cl | 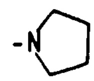 | Cl |
| OEt | H | $CH(CH_3)OAc$ | H | H | Cl | 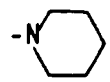 | Cl |
| OMe | H | $CH(CH_3)OAc$ | H | H | Cl | $-NMe_2$ | Me |
| OMe | H | $CH(CH_3)OAc$ | H | H | Cl | $NMe_2$ | Cl |
| OMe | OMe | $CH(CH_3)OAc$ | H | H | Cl | 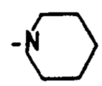 | Cl |
| OMe | OMe | $CH(CH_3)OAc$ | H | H | Cl | 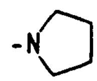 | Me |
| OEt | OEt | $CH(CH_3)OAc$ | H | H | Me | 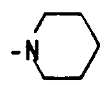 | Cl |
| H | H | $CH(CH_3)-O-CO-[CH_2]_4CH_3$ | H | H | Me | OMe | Cl |
| H | H | " | H | H | Me | OEt | Cl |
| H | H | " | H | H | Me | $OCH_2Ph$ | Cl |
| H | H | " | H | H | Me | $OCH_2CF_3$ | Cl |
| Me | H | " | H | H | Me | OMe | Cl |
| Me | Me | " | H | H | Me | OMe | Cl |
| Me | Me | " | H | H | Me | OEt | Cl |
| Me | Me | " | H | H | Me | OiPr | Cl |
| H | OMe | " | H | H | Me | OMe | Cl |
| H | OMe | " | H | H | Me | OEt | Cl |
| H | OMe | " | H | H | Me | OiPr | Cl |
| H | OMe | " | H | H | Me | $OCH_2Ph$ | Cl |

**Tabelle, Fortsetzung**

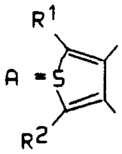 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | OMe | $CH(CH_3)-O-CO-[CH_2]_4CH_3$ | H | H | Me | $OCH_2CF_3$ | Cl |
| H | OMe | " | H | H | Me | $OCH_2CH_2CF_3$ | Cl |
| Me | OMe | " | H | H | Me | OMe | Cl |
| OMe | OMe | " | H | H | Me | OMe | Cl |
| OMe | OMe | " | H | H | Me | Opr | Cl |
| OMe | OMe | " | H | H | Me | $OCH_2Ph$ | Cl |
| OMe | OMe | " | H | H | Me | $OCH_2CF_3$ | Cl |
| H | H | " | H | H | Cl | OMe | Cl |
| H | H | " | H | H | Cl | OPr | Cl |
| H | H | " | H | H | Cl | $OCH_2Ph$ | Cl |
| Me | H | " | H | H | Cl | OEt | Cl |
| Me | Me | " | H | H | Cl | OMe | Cl |
| Me | Me | " | H | H | Cl | $OCH_2Ph$ | Cl |
| H | OMe | " | H | H | Cl | OMe | Cl |
| H | OMe | " | H | H | Cl | OPr | Cl |
| H | OMe | " | H | H | Cl | $OCH_2Ph$ | Cl |
| H | OMe | " | H | H | Cl | $OCH_2CF_3$ | Cl |
| Me | OMe | " | H | H | Cl | OMe | Cl |
| OMe | OMe | " | H | H | Cl | OMe | Cl |
| OMe | OMe | " | H | H | Cl | OEt | Cl |
| H | H | " | H | H | Cl | $OCH_2Ph$ | H |
| H | H | " | H | H | Cl | $OCH_2CF_3$ | H |

**Tabelle, Fortsetzung**

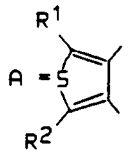 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | $CH(CH_3)-O-CO-[CH_2]_4CH_3$ | H | H | Cl | $OCH_2CF_2CF_3$ | H |
| Me | Me | " | H | H | Cl | OMe | H |
| Me | Me | " | H | H | Cl | $OCH_2Ph$ | H |
| OMe | H | " | H | H | Cl | OMe | H |
| OMe | H | " | H | H | Cl | $OCH_2Ph$ | H |
| OMe | H | " | H | H | Cl | $OCH_2CF_3$ | H |
| OMe | H | " | H | H | Cl | $OCH_2CF_2CF_3$ | H |
| Me | OMe | " | H | H | Cl | OEt | H |
| Me | OMe | " | H | H | Cl | $OCH_2CF_2CF_3$ | H |
| OMe | OMe | " | H | H | Cl | OMe | H |
| OMe | OMe | " | H | H | Cl | OEt | H |
| OMe | OMe | " | H | H | Cl | OiPr | H |
| OMe | OMe | " | H | H | Cl | $OCH_2Ph$ | H |
| OMe | OMe | " | H | H | Cl | $OCH_2CF_3$ | H |
| H | H | " | H | H | Cl | OMe | Me |
| H | H | " | H | H | Cl | OEt | Me |
| H | H | " | H | H | Cl | OiPr | Me |
| H | H | " | H | H | Cl | $OCH_2Ph$ | Me |
| H | H | " | H | H | Cl | $OCH_2CF_2CF_3$ | Me |
| Me | Me | " | H | H | Cl | OMe | Me |
| Me | Me | " | H | H | Cl | OEt | Me |
| Me | Me | " | H | H | Cl | $OCH_2CF_3$ | Me |

Tabelle, Fortsetzung

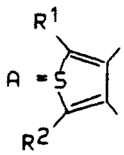

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | Me | $CH(CH_3)-O-CO-(CH_2)_4CH_3$ | H | H | Cl | $OCH_2CF_2CF_3$ | Me |
| OMe | H | " | H | H | Cl | OMe | Me |
| OMe | H | " | H | H | Cl | OiPr | Me |
| OMe | H | " | H | H | Cl | $OCH_2Ph$ | Me |
| OMe | H | " | H | H | Cl | $OCH_2CF_3$ | Me |
| OMe | Me | " | H | H | Cl | OMe | Me |
| OMe | OMe | " | H | H | Cl | OMe | Me |
| OMe | OMe | " | H | H | Cl | $OCH_2Ph$ | Me |
| OMe | OMe | " | H | H | Cl | OiPr | Cl |
| OMe | OMe | " | H | H | Cl | $OCH_2Ph$ | Cl |
| OMe | OMe | " | H | H | Cl | $OCH_2CF_3$ | Cl |
| H | H | " | H | H | Me | OMe | Me |
| H | H | " | H | H | Me | $OCH_2Ph$ | Me |
| Me | Me | " | H | H | Me | OMe | Me |
| Me | Me | " | H | H | Me | $OCH_2CF_3$ | Me |
| OMe | H | " | H | H | Me | OMe | Me |
| OMe | H | " | H | H | Me | Opr | Me |
| OMe | Me | " | H | H | Me | OMe | Me |
| OMe | Me | " | H | H | Me | $O(CH_2)_2OMe$ | Me |
| OMe | OMe | " | H | H | Me | OMe | Me |
| OMe | OMe | " | H | H | Me | $OCH_2CF_3$ | Me |
| H | H | " | H | H | Cl | OMe | H |

**Tabelle, Fortsetzung**

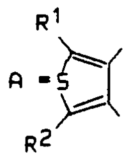

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | $CH(CH_3)-O-CO-(CH_2)_4CH_3$ | H | H | Cl | OEt | H |
| H | H | " | H | H | Cl | OiPr | H |
| OMe | OMe | " | H | H | Cl | $OCH_2CF_3$ | H |
| H | H | " | H | H | H | Me | H |
| Me | H | " | H | H | H | iPr | H |
| Me | Me | " | H | H | H | Me | H |
| Me | Me | " | H | H | Me | iPr | H |
| OMe | H | " | H | H | H | Me | H |
| OMe | H | " | H | H | Me | Me | H |
| OMe | H | " | H | H | Me | iPr | H |
| OMe | Me | " | H | H | Me | Me | H |
| OMe | OMe | " | H | H | H | Me | H |
| OMe | OMe | " | H | H | Me | Me | H |
| OMe | OMe | " | H | H | H | Me | Me |
| H | H | " | H | H | Me | Me | Me |
| H | H | " | H | H | Cl | Me | H |
| Me | Me | " | H | H | Me | Me | Me |
| Me | Me | " | H | H | Cl | Me | H |
| Me | Me | " | H | H | H | Me | Cl |
| OMe | H | " | H | H | Me | Me | Me |
| OMe | H | " | H | H | Cl | Me | H |
| OMe | OMe | " | H | H | Me | Me | Me |

Tabelle, Fortsetzung

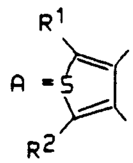 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | $CH(CH_3)-O-CO-(CH_2)_4CH_3$ | H | H | Cl | Me | H |
| OMe | OMe | " | H | H | Me | Et | Me |
| H | H | " | H | H | Cl | Me | Cl |
| H | H | " | H | H | Cl | Me | Me |
| H | H | " | H | H | Cl | Et | Me |
| Me | Me | " | H | H | Cl | Me | Me |
| Et | Et | " | H | H | Me | Me | Cl |
| OMe | H | " | H | H | Cl | Me | Cl |
| OMe | H | " | H | H | Cl | Me | Me |
| OMe | Me | " | H | H | Me | Me | Cl |
| OMe | OMe | " | H | H | Cl | Me | Me |
| OMe | OMe | " | H | H | Me | Me | Cl |
| OEt | OEt | " | H | H | Me | Me | Cl |
| H | H | " | H | H | Me | 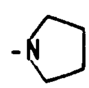 | H |
| Me | Me | " | H | H | Me | 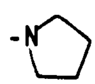 | H |
| OMe | H | " | H | H | Me | 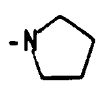 | H |
| OEt | H | " | H | H | H | $-NMe_2$ | Me |
| OMe | OMe | " | H | H | $CH_3$ | $-NMe_2$ | H |
| OEt | OEt | " | H | H | H | $-NMe_2$ | Me |

**Tabelle, Fortsetzung**

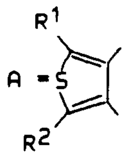 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | H | $CH(CH_3)-O-CO-(CH_2)_4CH_3$ | H | H | Cl | 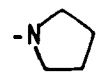 | H |
| Me | Me | " | H | H | Cl | $-NMe_2$ | H |
| OMe | H | " | H | H | Cl | $-NMe_2$ | H |
| OMe | OMe | " | H | H | Cl | $-NMe_2$ | H |
| OMe | OMe | " | H | H | Cl | 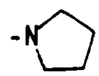 | H |
| H | H | " | H | H | Cl | $-NMe_2$ | Cl |
| Me | H | " | H | H | Cl | 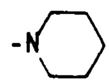 | Cl |
| Et | H | " | H | H | Cl | $-NMe_2$ | $CH_3$ |
| Me | Me | " | H | H | Cl | 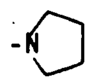 | Cl |
| OMe | H | " | H | H | Cl | $-NMe_2$ | Cl |
| $OCH_2Ph$ | H | " | H | H | Cl | 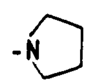 | Cl |
| OEt | H | " | H | H | Cl | 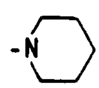 | Cl |
| OMe | H | " | H | H | Cl | $-NMe_2$ | Me |
| OMe | H | " | H | H | Cl | $NMe_2$ | Cl |
| OMe | OMe | " | H | H | Cl | 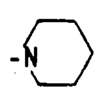 | Cl |
| OMe | OMe | " | H | H | Cl | 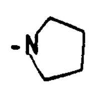 | Me |

**Tabelle, Fortsetzung**

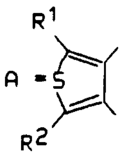 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OEt | OEt | $CH(CH_3)-O-CO-(CH_2)_4CH_3$ | H | H | Me | - 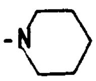 | Cl |
| H | H | CH(Pr)OAc | H | H | Me | OMe | Cl |
| H | H | " | H | H | Me | OEt | Cl |
| H | H | " | H | H | Me | $OCH_2Ph$ | Cl |
| H | H | " | H | H | Me | $OCH_2CF_3$ | Cl |
| Me | H | " | H | H | Me | OMe | Cl |
| Me | Me | " | H | H | Me | OMe | Cl |
| Me | Me | " | H | H | Me | OEt | Cl |
| Me | Me | " | H | H | Me | OiPr | Cl |
| H | OMe | " | H | H | Me | OMe | Cl |
| H | OMe | " | H | H | Me | OEt | Cl |
| H | OMe | " | H | H | Me | OiPr | Cl |
| H | OMe | " | H | H | Me | $OCH_2Ph$ | Cl |
| H | OMe | " | H | H | Me | $OCH_2CF_3$ | Cl |
| H | OMe | " | H | H | Me | $OCH_2CH_2CF_3$ | Cl |
| Me | OMe | " | H | H | Me | OMe | Cl |
| OMe | OMe | " | H | H | Me | OMe | Cl |
| OMe | OMe | " | H | H | Me | Opr | Cl |
| OMe | OMe | " | H | H | Me | $OCH_2Ph$ | Cl |
| OMe | OMe | " | H | H | Me | $OCH_2CF_3$ | Cl |
| H | H | " | H | H | Cl | OMe | Cl |
| H | H | " | H | H | Cl | OPr | Cl |

**Tabelle, Fortsetzung**

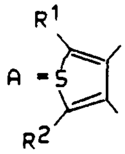

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | CH(Pr)OAc | H | H | Cl | $OCH_2Ph$ | Cl |
| Me | H | " | H | H | Cl | OEt | Cl |
| Me | Me | " | H | H | Cl | OMe | Cl |
| Me | Me | " | H | H | Cl | $OCH_2Ph$ | Cl |
| H | OMe | " | H | H | Cl | OMe | Cl |
| H | OMe | " | H | H | Cl | OPr | Cl |
| H | OMe | " | H | H | Cl | $OCH_2Ph$ | Cl |
| H | OMe | " | H | H | Cl | $OCH_2CF_3$ | Cl |
| Me | OMe | " | H | H | Cl | OMe | Cl |
| OMe | OMe | " | H | H | Cl | OMe | Cl |
| OMe | OMe | " | H | H | Cl | OEt | Cl |
| H | H | " | H | H | Cl | $OCH_2Ph$ | H |
| H | H | " | H | H | Cl | $OCH_2CF_3$ | H |
| H | H | " | H | H | Cl | $OCH_2CF_2CF_3$ | H |
| Me | Me | " | H | H | Cl | OMe | H |
| Me | Me | " | H | H | Cl | $OCH_2Ph$ | H |
| OMe | H | " | H | H | Cl | OMe | H |
| OMe | H | " | H | H | Cl | $OCH_2Ph$ | H |
| OMe | H | " | H | H | Cl | $OCH_2CF_3$ | H |
| OMe | H | " | H | H | Cl | $OCH_2CF_2CF_3$ | H |
| Me | OMe | " | H | H | Cl | OEt | H |
| Me | OMe | " | H | H | Cl | $OCH_2CF_2CF_3$ | H |

**Tabelle, Fortsetzung**

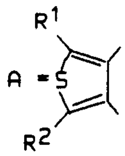 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | CH(Pr)OAc | H | H | Cl | OMe | H |
| OMe | OMe | " | H | H | Cl | OEt | H |
| OMe | OMe | " | H | H | Cl | OiPr | H |
| OMe | OMe | " | H | H | Cl | $OCH_2Ph$ | H |
| OMe | OMe | " | H | H | Cl | $OCH_2CF_3$ | H |
| H | H | " | H | H | Cl | OMe | Me |
| H | H | " | H | H | Cl | OEt | Me |
| H | H | " | H | H | Cl | OiPr | Me |
| H | H | " | H | H | Cl | $OCH_2Ph$ | Me |
| H | H | " | H | H | Cl | $OCH_2CF_2CF_3$ | Me |
| Me | Me | " | H | H | Cl | OMe | Me |
| Me | Me | " | H | H | Cl | OEt | Me |
| Me | Me | " | H | H | Cl | $OCH_2CF_3$ | Me |
| Me | Me | " | H | H | Cl | $OCH_2CF_2CF_3$ | Me |
| OMe | H | " | H | H | Cl | OMe | Me |
| OMe | H | " | H | H | Cl | OiPr | Me |
| OMe | H | " | H | H | Cl | $OCH_2Ph$ | Me |
| OMe | H | " | H | H | Cl | $OCH_2CF_3$ | Me |
| OMe | H | " | H | H | Cl | OMe | Me |
| OMe | H | " | H | H | Cl | OMe | Me |
| OMe | OMe | " | H | H | Cl | $OCH_2Ph$ | Me |
| OMe | OMe | " | H | H | Cl | OiPr | Cl |

**Tabelle, Fortsetzung**

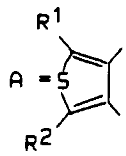 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | CH(Pr)OAc | H | H | Cl | $OCH_2Ph$ | Cl |
| OMe | OMe | " | H | H | Cl | $OCH_2CF_3$ | Cl |
| H | H | " | H | H | Me | OMe | Me |
| H | H | " | H | H | Me | $OCH_2Ph$ | Me |
| Me | Me | " | H | H | Me | OMe | Me |
| Me | Me | " | H | H | Me | $OCH_2CF_3$ | Me |
| OMe | H | " | H | H | Me | OMe | Me |
| OMe | H | " | H | H | Me | Opr | Me |
| OMe | Me | " | H | H | Me | OMe | Me |
| OMe | Me | " | H | H | Me | $O(CH_2)_2OMe$ | Me |
| OMe | OMe | " | H | H | Me | OMe | Me |
| OMe | OMe | " | H | H | Me | $OCH_2CF_3$ | Me |
| H | H | " | H | H | Cl | OMe | H |
| H | H | " | H | H | Cl | OEt | H |
| H | H | " | H | H | Cl | OiPr | H |
| OMe | OMe | " | H | H | Cl | $OCH_2CF_3$ | H |
| H | H | " | H | H | H | Me | H |
| Me | H | " | H | H | H | iPr | H |
| Me | Me | " | H | H | H | Me | H |
| Me | Me | " | H | H | Me | iPr | H |
| OMe | H | " | H | H | H | Me | H |
| OMe | H | " | H | H | Me | Me | H |

**Tabelle, Fortsetzung**

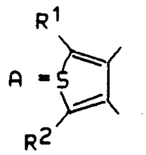 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | H | CH(Pr)OAc | H | H | Me | iPr | H |
| OMe | Me | " | H | H | Me | Me | H |
| OMe | OMe | " | H | H | H | Me | H |
| OMe | OMe | " | H | H | Me | Me | H |
| OMe | OMe | " | H | H | H | Me | Me |
| H | H | " | H | H | Me | Me | Me |
| H | H | " | H | H | Cl | Me | H |
| Me | Me | " | H | H | Me | Me | Me |
| Me | Me | " | H | H | Cl | Me | H |
| Me | Me | " | H | H | H | Me | Cl |
| OMe | H | " | H | H | Me | Me | Me |
| OMe | H | " | H | H | Cl | Me | H |
| OMe | OMe | " | H | H | Me | Me | Me |
| OMe | OMe | " | H | H | Cl | Me | H |
| OMe | OMe | " | H | H | Me | Et | Me |
| H | H | " | H | H | Cl | Me | Cl |
| H | H | " | H | H | Cl | Me | Me |
| H | H | " | H | H | Cl | Et | Me |
| Me | Me | " | H | H | Cl | Me | Me |
| Et | Et | " | H | H | Me | Me | Cl |
| OMe | H | " | H | H | Cl | Me | Cl |
| OMe | H | " | H | H | Cl | Me | Me |

**Tabelle, Fortsetzung**

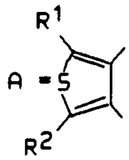 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | Me | CH(Pr)OAc | H | H | Me | Me | Cl |
| OMe | OMe | " | H | H | Cl | Me | Me |
| OMe | OMe | " | H | H | Me | Me | Cl |
| OEt | OEt | " | H | H | Me | Me | Cl |
| H | H | " | H | H | Me | 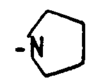 | H |
| Me | Me | " | H | H | Me | 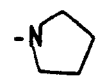 | H |
| OMe | H | " | H | H | Me | 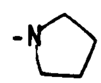 | H |
| OEt | H | " | H | H | H | $-NMe_2$ | Me |
| OMe | OMe | " | H | H | $CH_3$ | $-NMe_2$ | H |
| OEt | OEt | " | H | H | H | $-NMe_2$ | Me |
| Me | H | " | H | H | Cl | 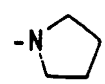 | H |
| Me | Me | " | H | H | Cl | $-NMe_2$ | H |
| OMe | H | " | H | H | Cl | $-NMe_2$ | H |
| OMe | OMe | " | H | H | Cl | $-NMe_2$ | H |
| OMe | OMe | " | H | H | Cl | 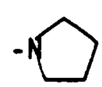 | H |
| H | H | " | H | H | Cl | $-NMe_2$ | Cl |
| Me | H | " | H | H | Cl | 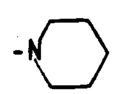 | Cl |

**Tabelle, Fortsetzung**

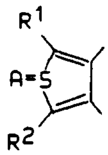 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Et | H | CH(Pr)OAc | H | H | Cl | $-NMe_2$ | $CH_3$ |
| Me | Me | " | H | H | Cl | 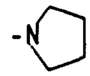 | Cl |
| OMe | H | " | H | H | Cl | $-NMe_2$ | Cl |
| $OCH_2Ph$ | H | " | H | H | Cl | 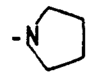 | Cl |
| OEt | H | " | H | H | Cl | 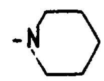 | Cl |
| OMe | H | " | H | H | Cl | $-NMe_2$ | Me |
| OMe | H | " | H | H | Cl | $-NMe_2$ | Cl |
| OMe | OMe | " | H | H | Cl | 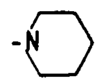 | Cl |
| OMe | OMe | " | H | H | Cl | 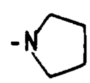 | Me |
| OEt | OEt | " | H | H | Me | 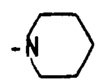 | Cl |
| H | H | $CO_2$-CH(Me)OAc | H | H | Me | OMe | Cl |
| H | H | " | H | H | Me | OEt | Cl |
| H | H | " | H | H | Me | $OCH_2Ph$ | Cl |
| H | H | " | H | H | Me | $OCH_2CF_3$ | Cl |
| Me | H | " | H | H | Me | OMe | Cl |
| Me | Me | " | H | H | Me | OMe | Cl |
| Me | Me | " | H | H | Me | OEt | Cl |
| Me | Me | " | H | H | Me | OiPr | Cl |

**Tabelle, Fortsetzung**

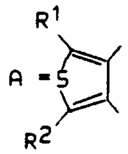 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | OMe | $CO_2$-CH(Me)OAc | H | H | Me | OMe | Cl |
| H | OMe | " | H | H | Me | OEt | Cl |
| H | OMe | " | H | H | Me | OiPr | Cl |
| H | OMe | " | H | H | Me | $OCH_2Ph$ | Cl |
| H | OMe | " | H | H | Me | $OCH_2CF_3$ | Cl |
| H | OMe | " | H | H | Me | $OCH_2CH_2CF_3$ | Cl |
| Me | OMe | " | H | H | Me | OMe | Cl |
| OMe | OMe | " | H | H | Me | OMe | Cl |
| OMe | OMe | " | H | H | Me | Opr | Cl |
| OMe | OMe | " | H | H | Me | $OCH_2Ph$ | Cl |
| OMe | OMe | " | H | H | Me | $OCH_2CF_3$ | Cl |
| H | H | " | H | H | Cl | OMe | Cl |
| H | H | " | H | H | Cl | OPr | Cl |
| H | H | " | H | H | Cl | $OCH_2Ph$ | Cl |
| Me | H | " | H | H | Cl | OEt | Cl |
| Me | Me | " | H | H | Cl | OMe | Cl |
| Me | Me | " | H | H | Cl | $OCH_2Ph$ | Cl |
| H | OMe | " | H | H | Cl | OMe | Cl |
| H | OMe | " | H | H | Cl | OPr | Cl |
| H | OMe | " | H | H | Cl | $OCH_2Ph$ | Cl |
| H | OMe | " | H | H | Cl | $OCH_2CF_3$ | Cl |
| Me | OMe | " | H | H | Cl | OMe | Cl |

**Tabelle, Fortsetzung**

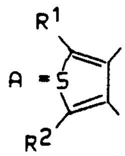

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | $CO_2CH(Me)OAc$ | H | H | Cl | OMe | Cl |
| OMe | OMe | " | H | H | Cl | OEt | Cl |
| H | H | " | H | H | Cl | $OCH_2Ph$ | H |
| H | H | " | H | H | Cl | $OCH_2CF_3$ | H |
| H | H | " | H | H | Cl | $OCH_2CF_2CF_3$ | H |
| Me | Me | " | H | H | Cl | OMe | H |
| Me | Me | " | H | H | Cl | $OCH_2Ph$ | H |
| OMe | H | " | H | H | Cl | OMe | H |
| OMe | H | " | H | H | Cl | $OCH_2Ph$ | H |
| OMe | H | " | H | H | Cl | $OCH_2CF_3$ | H |
| OMe | H | " | H | H | Cl | $OCH_2CF_2CF_3$ | H |
| Me | OMe | " | H | H | Cl | OEt | H |
| Me | OMe | " | H | H | Cl | $OCH_2CF_2CF_3$ | H |
| OMe | OMe | " | H | H | Cl | OMe | H |
| OMe | OMe | " | H | H | Cl | OEt | H |
| OMe | OMe | " | H | H | Cl | OiPr | H |
| OMe | OMe | " | H | H | Cl | $OCH_2Ph$ | H |
| OMe | OMe | " | H | H | Cl | $OCH_2CF_3$ | H |
| H | H | " | H | H | Cl | OMe | Me |
| H | H | " | H | H | Cl | OEt | Me |
| H | H | " | H | H | Cl | OiPr | Me |
| H | H | " | H | H | Cl | $OCH_2Ph$ | Me |

Tabelle, Fortsetzung

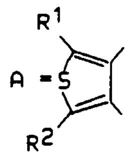

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | $CO_2$-CH(Me)OAc | H | H | Cl | $OCH_2CF_2CF_3$ | Me |
| Me | Me | " | H | H | Cl | OMe | Me |
| Me | Me | " | H | H | Cl | OEt | Me |
| Me | Me | " | H | H | Cl | $OCH_2CF_3$ | Me |
| Me | Me | " | H | H | Cl | $OCH_2CF_2CF_3$ | Me |
| OMe | H | " | H | H | Cl | OMe | Me |
| OMe | H | " | H | H | Cl | OiPr | Me |
| OMe | H | " | H | H | Cl | $OCH_2Ph$ | Me |
| OMe | H | " | H | H | Cl | $OCH_2CF_3$ | Me |
| OMe | H | " | H | H | Cl | OMe | Me |
| OMe | H | " | H | H | Cl | OMe | Me |
| OMe | OMe | " | H | H | Cl | $OCH_2Ph$ | Me |
| OMe | OMe | " | H | H | Cl | OiPr | Cl |
| OMe | OMe | " | H | H | Cl | $OCH_2Ph$ | Cl |
| OMe | OMe | " | H | H | Cl | $OCH_2CF_3$ | Cl |
| H | H | " | H | H | Me | OMe | Me |
| H | H | " | H | H | Me | $OCH_2Ph$ | Me |
| Me | Me | " | H | H | Me | OMe | Me |
| Me | Me | " | H | H | Me | $OCH_2CF_3$ | Me |
| OMe | H | " | H | H | Me | OMe | Me |
| OMe | H | " | H | H | Me | Opr | Me |
| OMe | Me | " | H | H | Me | OMe | Me |

**Tabelle, Fortsetzung**

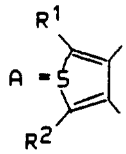

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | Me | $CO_2$-CH(Me)OAc | H | H | Me | $O(CH_2)_2OMe$ | Me |
| OMe | OMe | " | H | H | Me | OMe | Me |
| OMe | OMe | " | H | H | Me | $OCH_2CF_3$ | Me |
| H | H | " | H | H | Cl | OMe | H |
| H | H | " | H | H | Cl | OEt | H |
| H | H | " | H | H | Cl | OiPr | H |
| OMe | OMe | " | H | H | Cl | $OCH_2CF_3$ | H |
| H | H | " | H | H | H | Me | H |
| Me | H | " | H | H | H | iPr | H |
| Me | Me | " | H | H | H | Me | H |
| Me | Me | " | H | H | Me | iPr | H |
| OMe | H | " | H | H | H | Me | H |
| OMe | H | " | H | H | Me | Me | H |
| OMe | H | " | H | H | Me | iPr | H |
| OMe | Me | " | H | H | Me | Me | H |
| OMe | OMe | " | H | H | H | Me | H |
| OMe | OMe | " | H | H | Me | Me | H |
| OMe | OMe | " | H | H | H | Me | Me |
| H | H | " | H | H | Me | Me | Me |
| H | H | " | H | H | Cl | Me | H |
| Me | Me | " | H | H | Me | Me | Me |
| Me | Me | " | H | H | Cl | Me | H |

**Tabelle, Fortsetzung**

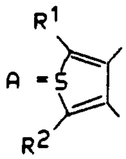 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | Me | $CO_2$-CH(Me)OAc | H | H | H | Me | Cl |
| OMe | H | " | H | H | Me | Me | Me |
| OMe | H | " | H | H | Cl | Me | H |
| OMe | OMe | " | H | H | Me | Me | Me |
| OMe | OMe | " | H | H | Cl | Me | H |
| OMe | OMe | " | H | H | Me | Et | Me |
| H | H | " | H | H | Cl | Me | Cl |
| H | H | " | H | H | Cl | Me | Me |
| H | H | " | H | H | Cl | Et | Me |
| Me | Me | " | H | H | Cl | Me | Me |
| Et | Et | " | H | H | Me | Me | Cl |
| OMe | H | " | H | H | Cl | Me | Cl |
| OMe | H | " | H | H | Cl | Me | Me |
| OMe | Me | " | H | H | Me | Me | Cl |
| OMe | OMe | " | H | H | Cl | Me | Me |
| OMe | OMe | " | H | H | Me | Me | Cl |
| OEt | OEt | " | H | H | Me | Me | Cl |
| H | H | " | H | H | Me | 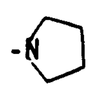 | H |
| Me | Me | " | H | H | Me | 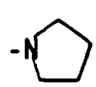 | H |
| OMe | H | " | H | H | Me | 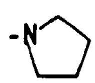 | H |

**Tabelle, Fortsetzung**

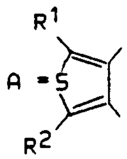 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OEt | H | $CO_2$-CH(Me)OAc | H | H | H | $-NMe_2$ | Me |
| OMe | OMe | " | H | H | $CH_3$ | $-NMe_2$ | H |
| OEt | OEt | " | H | H | H | $-NMe_2$ | Me |
| Me | H | " | H | H | Cl | 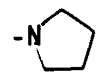 | H |
| Me | Me | " | H | H | Cl | $-NMe_2$ | H |
| OMe | H | " | H | H | Cl | $-NMe_2$ | H |
| OMe | OMe | " | H | H | Cl | $-NMe_2$ | H |
| OMe | OMe | " | H | H | Cl | 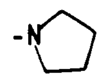 | H |
| H | H | " | H | H | Cl | $-NMe_2$ | Cl |
| Me | H | " | H | H | Cl | 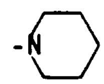 | Cl |
| Et | H | " | H | H | Cl | $-NMe_2$ | $CH_3$ |
| Me | Me | " | H | H | Cl | 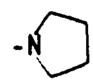 | Cl |
| OMe | H | " | H | H | Cl | $-NMe_2$ | Cl |
| $OCH_2Ph$ | H | " | H | H | Cl | 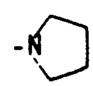 | Cl |
| OEt | H | " | H | H | Cl | 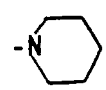 | Cl |
| OMe | H | " | H | H | Cl | $-NMe_2$ | Me |

**Tabelle, Fortsetzung**

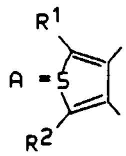 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | H | $CO_2CH(Me)OAc$ | H | H | Cl | $-NMe_2$ | Cl |
| OMe | OMe | " | H | H | Cl | 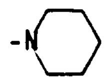 | Cl |
| OMe | OMe | " | H | H | Cl | 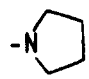 | Me |
| OEt | OEt | " | H | H | Me | 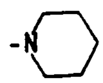 | Cl |
| H | H | $CO_2$-CH(Me)-O-CO-Pent | H | H | Me | OMe | Cl |
| H | H | " | H | H | Me | OEt | Cl |
| H | H | " | H | H | Me | $OCH_2Ph$ | Cl |
| H | H | " | H | H | Me | $OCH_2CF_3$ | Cl |
| Me | H | " | H | H | Me | OMe | Cl |
| Me | Me | " | H | H | Me | OMe | Cl |
| Me | Me | " | H | H | Me | OEt | Cl |
| Me | Me | " | H | H | Me | OiPr | Cl |
| H | OMe | " | H | H | Me | OMe | Cl |
| H | OMe | " | H | H | Me | OEt | Cl |
| H | OMe | " | H | H | Me | OiPr | Cl |
| H | OMe | " | H | H | Me | $OCH_2Ph$ | Cl |
| H | OMe | " | H | H | Me | $OCH_2CF_3$ | Cl |
| H | OMe | " | H | H | Me | $OCH_2CH_2CF_3$ | Cl |
| Me | OMe | " | H | H | Me | OMe | Cl |

Pent= n-Pentyl

**Tabelle, Fortsetzung**

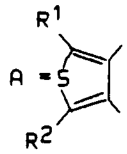

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | $CO_2$-CH(Me)-O-CO-Pent | H | H | Me | OMe | Cl |
| OMe | OMe | " | H | H | Me | Opr | Cl |
| OMe | OMe | " | H | H | Me | $OCH_2Ph$ | Cl |
| OMe | OMe | " | H | H | Me | $OCH_2CF_3$ | Cl |
| H | H | " | H | H | Cl | OMe | Cl |
| H | H | " | H | H | Cl | OPr | Cl |
| H | H | " | H | H | Cl | $OCH_2Ph$ | Cl |
| Me | H | " | H | H | Cl | OEt | Cl |
| Me | Me | " | H | H | Cl | OMe | Cl |
| Me | Me | " | H | H | Cl | $OCH_2Ph$ | Cl |
| H | OMe | " | H | H | Cl | OMe | Cl |
| H | OMe | " | H | H | Cl | OPr | Cl |
| H | OMe | " | H | H | Cl | $OCH_2Ph$ | Cl |
| H | OMe | " | H | H | Cl | $OCH_2CF_3$ | Cl |
| Me | OMe | " | H | H | Cl | OMe | Cl |
| OMe | OMe | " | H | H | Cl | OMe | Cl |
| OMe | OMe | " | H | H | Cl | OEt | Cl |
| H | H | " | H | H | Cl | $OCH_2Ph$ | H |
| H | H | " | H | H | Cl | $OCH_2CF_3$ | H |
| H | H | " | H | H | Cl | $OCH_2CF_2CF_3$ | H |
| Me | Me | " | H | H | Cl | OMe | H |

Pent= n-Pentyl

**Tabelle, Fortsetzung**

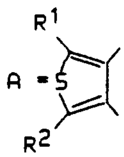 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | Me | $CO_2$-CH(Me)-O-CO-Pent | H | H | Cl | $OCH_2Ph$ | H |
| OMe | H | " | H | H | Cl | OMe | H |
| OMe | H | " | H | H | Cl | $OCH_2Ph$ | H |
| OMe | H | " | H | H | Cl | $OCH_2CF_3$ | H |
| OMe | H | " | H | H | Cl | $OCH_2CF_2CF_3$ | H |
| Me | OMe | " | H | H | Cl | OEt | H |
| Me | OMe | " | H | H | Cl | $OCH_2CF_2CF_3$ | H |
| OMe | OMe | " | H | H | Cl | OMe | H |
| OMe | OMe | " | H | H | Cl | OEt | H |
| OMe | OMe | " | H | H | Cl | OiPr | H |
| OMe | OMe | " | H | H | Cl | $OCH_2Ph$ | H |
| OMe | OMe | " | H | H | Cl | $OCH_2CF_3$ | H |
| H | H | " | H | H | Cl | OMe | Me |
| H | H | " | H | H | Cl | OEt | Me |
| H | H | " | H | H | Cl | OiPr | Me |
| H | H | " | H | H | Cl | $OCH_2Ph$ | Me |
| H | H | " | H | H | Cl | $OCH_2CF_2CF_3$ | Me |
| Me | Me | " | H | H | Cl | OMe | Me |
| Me | Me | " | H | H | Cl | OEt | Me |
| Me | Me | " | H | H | Cl | $OCH_2CF_3$ | Me |
| Me | Me | " | H | H | Cl | $OCH_2CF_2CF_3$ | Me |

Pent= n-Pentyl

**Tabelle, Fortsetzung**

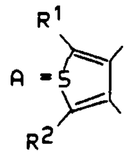

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | H | $CO_2$-CH(Me)-O-CO-Pent | H | H | Cl | OMe | Me |
| OMe | H | " | H | H | Cl | OiPr | Me |
| OMe | H | " | H | H | Cl | $OCH_2Ph$ | Me |
| OMe | H | " | H | H | Cl | $OCH_2CF_3$ | Me |
| OMe | Me | " | H | H | Cl | OMe | Me |
| OMe | OMe | " | H | H | Cl | OMe | Me |
| OMe | OMe | " | H | H | Cl | $OCH_2Ph$ | Me |
| OMe | OMe | " | H | H | Cl | OiPr | Cl |
| OMe | OMe | " | H | H | Cl | $OCH_2Ph$ | Cl |
| OMe | OMe | " | H | H | Cl | $OCH_2CF_3$ | Cl |
| H | H | " | H | H | Me | OMe | Me |
| H | H | " | H | H | Me | $OCH_2Ph$ | Me |
| Me | Me | " | H | H | Me | OMe | Me |
| Me | Me | " | H | H | Me | $OCH_2CF_3$ | Me |
| OMe | H | " | H | H | Me | OMe | Me |
| OMe | H | " | H | H | Me | Opr | Me |
| OMe | Me | " | H | H | Me | OMe | Me |
| OMe | Me | " | H | H | Me | $O(CH_2)_2OMe$ | Me |
| OMe | OMe | " | H | H | Me | OMe | Me |
| OMe | OMe | " | H | H | Me | $OCH_2CF_3$ | Me |
| H | H | " | H | H | Cl | OMe | H |

Pent= n-Pentyl

**Tabelle, Fortsetzung**

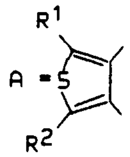

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | $CO_2CH(Me)$-O-CO-Pent | H | H | Cl | OEt | H |
| H | H | " | H | H | Cl | OiPr | H |
| OMe | OMe | " | H | H | Cl | $OCH_2CF_3$ | H |
| H | H | " | H | H | H | Me | H |
| H | H | " | H | H | H | Me | H |
| Me | H | " | H | H | H | iPr | H |
| Me | Me | " | H | H | H | Me | H |
| Me | Me | " | H | H | Me | iPr | H |
| OMe | H | " | H | H | H | Me | H |
| OMe | H | " | H | H | Me | Me | H |
| OMe | H | " | H | H | Me | iPr | H |
| OMe | Me | " | H | H | Me | Me | H |
| OMe | OMe | " | H | H | H | Me | H |
| OMe | OMe | " | H | H | Me | Me | H |
| OMe | OMe | " | H | H | H | Me | Me |
| H | H | " | H | H | Me | Me | Me |
| H | H | " | H | H | Cl | Me | H |
| Me | Me | " | H | H | Me | Me | Me |
| Me | Me | " | H | H | Cl | Me | H |
| Me | Me | " | H | H | H | Me | Cl |
| OMe | H | " | H | H | Me | Me | Me |

Pent= n-Pentyl

**Tabelle, Fortsetzung**

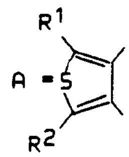 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | H | $CO_2$-CH(Me)-O-CO-Pent | H | H | Cl | Me | H |
| OMe | OMe | " | H | H | Me | Me | Me |
| OMe | OMe | " | H | H | Cl | Me | H |
| OMe | OMe | " | H | H | Me | Et | Me |
| H | H | " | H | H | Cl | Me | Cl |
| H | H | " | H | H | Cl | Me | Me |
| H | H | " | H | H | Cl | Et | Me |
| Me | Me | " | H | H | Cl | Me | Me |
| Et | Et | " | H | H | Me | Me | Cl |
| OMe | H | " | H | H | Cl | Me | Cl |
| OMe | H | " | H | H | Cl | Me | Me |
| OMe | Me | " | H | H | Me | Me | Cl |
| OMe | OMe | " | H | H | Cl | Me | Me |
| OMe | OMe | " | H | H | Me | Me | Cl |
| OEt | OEt | " | H | H | Me | Me | Cl |
| H | H | " | H | H | Me | 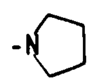 | H |
| Me | Me | " | H | H | Me | 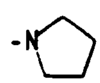 | H |
| OMe | H | " | H | H | Me | 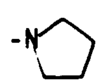 | H |
| OEt | H | " | H | H | H | -$NMe_2$ | Me |

Pent= n-Pentyl

**Tabelle, Fortsetzung**

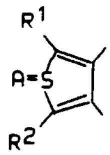 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | $CO_2$-CH(Me)-O-CO-Pent | H | H | $CH_3$ | $-NMe_2$ | H |
| OEt | OEt | " | H | H | H | $-NMe_2$ | Me |
| Me | H | " | H | H | Cl | 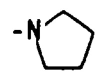 | H |
| Me | Me | " | H | H | Cl | $-NMe_2$ | H |
| OMe | H | " | H | H | Cl | $-NMe_2$ | H |
| OMe | OMe | " | H | H | Cl | $-NMe_2$ | H |
| OMe | OMe | " | H | H | Cl | 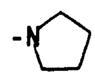 | H |
| H | H | " | H | H | Cl | $-NMe_2$ | Cl |
| Me | H | " | H | H | Cl | 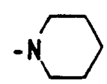 | Cl |
| Et | H | " | H | H | Cl | $-NMe_2$ | $CH_3$ |
| Me | Me | " | H | H | Cl | 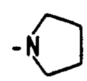 | Cl |
| OMe | H | " | H | H | Cl | $-NMe_2$ | Cl |
| $OCH_2Ph$ | H | " | H | H | Cl | 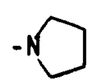 | Cl |
| OEt | H | " | H | H | Cl | 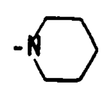 | Cl |
| OMe | H | " | H | H | Cl | $-NMe_2$ | Me |
| OMe | H | " | H | H | Cl | $-NMe_2$ | Cl |

Pent= n-Pentyl

Tabelle, Fortsetzung

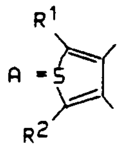 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | $CO_2CH(Me)$-O-CO-Pent | H | H | Cl | 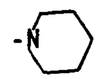 | Cl |
| OMe | OMe | " | H | H | Cl | 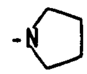 | Me |
| OEt | OEt | " | H | H | Me | 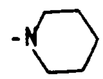 | Cl |
| H | H | Moc | H | H | Me | OMe | Cl |
| H | H | " | H | H | Me | OEt | Cl |
| H | H | " | H | H | Me | $OCH_2Ph$ | Cl |
| H | H | " | H | H | Me | $OCH_2CF_3$ | Cl |
| Me | H | " | H | H | Me | OMe | Cl |
| Me | Me | " | H | H | Me | OMe | Cl |
| Me | Me | " | H | H | Me | OEt | Cl |
| Me | Me | " | H | H | Me | OiPr | Cl |
| H | OMe | " | H | H | Me | OMe | Cl |
| H | OMe | " | H | H | Me | OEt | Cl |
| H | OMe | " | H | H | Me | OiPr | Cl |
| H | OMe | " | H | H | Me | $OCH_2Ph$ | Cl |
| H | OMe | " | H | H | Me | $OCH_2CF_3$ | Cl |
| H | OMe | " | H | H | Me | $OCH_2CH_2CF_3$ | Cl |
| Me | OMe | " | H | H | Me | OMe | Cl |
| OMe | OMe | " | H | H | Me | OMe | Cl |
| OMe | OMe | Ddz | H | H | Me | Opr | Cl |

Pent= n-Pentyl

**Tabelle, Fortsetzung**

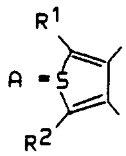

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | Ddz | H | H | Me | $OCH_2Ph$ | Cl |
| OMe | OMe | " | H | H | Me | $OCH_2CF_3$ | Cl |
| H | H | " | H | H | Cl | OMe | Cl |
| H | H | " | H | H | Cl | OPr | Cl |
| H | H | " | H | H | Cl | $OCH_2Ph$ | Cl |
| Me | H | " | H | H | Cl | OEt | Cl |
| Me | Me | " | H | H | Cl | OMe | Cl |
| Me | Me | " | H | H | Cl | $OCH_2Ph$ | Cl |
| H | OMe | " | H | H | Cl | OMe | Cl |
| H | OMe | " | H | H | Cl | OPr | Cl |
| H | OMe | " | H | H | Cl | $OCH_2Ph$ | Cl |
| H | OMe | " | H | H | Cl | $OCH_2CF_3$ | Cl |
| Me | OMe | " | H | H | Cl | OMe | Cl |
| OMe | OMe | " | H | H | Cl | OMe | Cl |
| OMe | OMe | " | H | H | Cl | OEt | Cl |
| H | H | Moc | H | H | Cl | $OCH_2Ph$ | H |
| H | H | " | H | H | Cl | $OCH_2CF_3$ | H |
| H | H | " | H | H | Cl | $OCH_2CF_2CF_3$ | H |
| Me | Me | " | H | H | Cl | OMe | H |
| Me | Me | " | H | H | Cl | $OCH_2Ph$ | H |
| OMe | H | " | H | H | Cl | OMe | H |
| OMe | H | " | H | H | Cl | $OCH_2Ph$ | H |

Tabelle, Fortsetzung

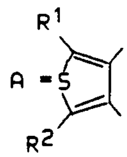 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | H | Moc | H | H | Cl | $OCH_2CF_3$ | H |
| OMe | H | " | H | H | Cl | $OCH_2CF_2CF_3$ | H |
| Me | OMe | " | H | H | Cl | OEt | H |
| Me | OMe | " | H | H | Cl | $OCH_2CF_2CF_3$ | H |
| OMe | OMe | " | H | H | Cl | OMe | H |
| OMe | OMe | " | H | H | Cl | OEt | H |
| OMe | OMe | " | H | H | Cl | OiPr | H |
| OMe | OMe | " | H | H | Cl | $OCH_2Ph$ | H |
| OMe | OMe | " | H | H | Cl | $OCH_2CF_3$ | H |
| H | H | Ddz | H | H | Cl | OMe | Me |
| H | H | " | H | H | Cl | OEt | Me |
| H | H | " | H | H | Cl | OiPr | Me |
| H | H | " | H | H | Cl | $OCH_2Ph$ | Me |
| H | H | " | H | H | Cl | $OCH_2CF_2CF_3$ | Me |
| Me | Me | " | H | H | Cl | OMe | Me |
| Me | Me | " | H | H | Cl | OEt | Me |
| Me | Me | " | H | H | Cl | $OCH_2CF_3$ | Me |
| Me | Me | " | H | H | Cl | $OCH_2CF_2CF_3$ | Me |
| OMe | H | " | H | H | Cl | OMe | Me |
| OMe | H | " | H | H | Cl | OiPr | Me |
| OMe | H | " | H | H | Cl | $OCH_2Ph$ | Me |
| OMe | H | " | H | H | Cl | $OCH_2CF_3$ | Me |

**Tabelle, Fortsetzung**

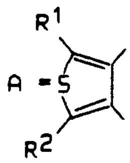

, T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | Me | Ddz | H | H | Cl | OMe | Me |
| OMe | OMe | " | H | H | Cl | OMe | Me |
| OMe | OMe | " | H | H | Cl | $OCH_2Ph$ | Me |
| OMe | OMe | Moc | H | H | Cl | OiPr | Cl |
| OMe | OMe | " | H | H | Cl | $OCH_2Ph$ | Cl |
| OMe | OMe | " | H | H | Cl | $OCH_2CF_3$ | Cl |
| H | H | " | H | H | Me | OMe | Me |
| H | H | " | H | H | Me | $OCH_2Ph$ | Me |
| Me | Me | " | H | H | Me | OMe | Me |
| Me | Me | " | H | H | Me | $OCH_2CF_3$ | Me |
| OMe | H | " | H | H | Me | OMe | Me |
| OMe | H | " | H | H | Me | Opr | Me |
| OMe | Me | " | H | H | Me | OMe | Me |
| OMe | Me | " | H | H | Me | $O(CH_2)_2OMe$ | Me |
| OMe | OMe | " | H | H | Me | OMe | Me |
| OMe | OMe | " | H | H | Me | $OCH_2CF_3$ | Me |
| H | H | " | H | H | Cl | OMe | H |
| H | H | " | H | H | Cl | OEt | H |
| H | H | " | H | H | Cl | OiPr | H |
| OMe | OMe | Ddz | H | H | Cl | $OCH_2CF_3$ | H |
| H | H | " | H | H | H | Me | H |

**Tabelle, Fortsetzung**

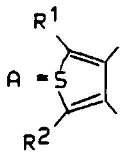 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| Me | H | Ddz | H | H | H | iPr | H |
| Me | Me | " | H | H | H | Me | H |
| Me | Me | " | H | H | Me | iPr | H |
| OMe | H | " | H | H | H | Me | H |
| OMe | H | " | H | H | Me | Me | H |
| OMe | H | " | H | H | Me | iPr | H |
| OMe | Me | " | H | H | Me | Me | H |
| OMe | OMe | " | H | H | H | Me | H |
| OMe | OMe | " | H | H | Me | Me | H |
| OMe | OMe | " | H | H | H | Me | Me |
| H | H | " | H | H | Me | Me | Me |
| H | H | " | H | H | Cl | Me | H |
| Me | Me | " | H | H | Me | Me | Me |
| Me | Me | " | H | H | Cl | Me | H |
| Me | Me | Moc | H | H | H | Me | Cl |
| OMe | H | " | H | H | Me | Me | Me |
| OMe | H | " | H | H | Cl | Me | H |
| OMe | OMe | " | H | H | Me | Me | Me |
| OMe | OMe | " | H | H | Cl | Me | H |
| OMe | OMe | " | H | H | Me | Et | Me |
| H | H | " | H | H | Cl | Me | Cl |
| H | H | " | H | H | Cl | Me | Me |

**Tabelle, Fortsetzung**

A = 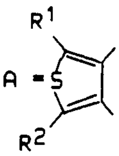 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| H | H | Moc | H | H | Cl | Et | Me |
| Me | Me | " | H | H | Cl | Me | Me |
| Et | Et | " | H | H | Me | Me | Cl |
| OMe | H | " | H | H | Cl | Me | Cl |
| OMe | H | " | H | H | Cl | Me | Me |
| OMe | Me | " | H | H | Me | Me | Cl |
| OMe | OMe | " | H | H | Cl | Me | Me |
| OMe | OMe | " | H | H | Me | Me | Cl |
| OEt | OEt | Ddz | H | H | Me | Me | Cl |
| H | H | " | H | H | Me | 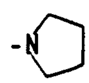 | H |
| Me | Me | " | H | H | Me | 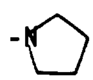 | H |
| OMe | H | " | H | H | Me | 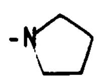 | H |
| OEt | H | " | H | H | H | $-NMe_2$ | Me |
| OMe | OMe | " | H | H | $CH_3$ | $-NMe_2$ | H |
| OEt | OEt | " | H | H | H | $-NMe_2$ | Me |
| Me | H | " | H | H | Cl | 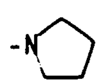 | H |
| Me | Me | " | H | H | Cl | $-NMe_2$ | H |
| OMe | H | " | H | H | Cl | $-NMe_2$ | H |
| OMe | OMe | " | H | H | Cl | $-NMe_2$ | H |

**Tabelle, Fortsetzung**

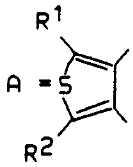 , T = SO, $R^9$ = H

| $R^1$ | $R^2$ | $R^3$ | $R^4$ | $R^5$ | $R^6$ | $R^7$ | $R^8$ |
|---|---|---|---|---|---|---|---|
| OMe | OMe | Ddz | H | H | Cl | 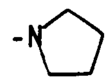 | H |
| H | H | " | H | H | Cl | $-NMe_2$ | Cl |
| Me | H | " | H | H | Cl | 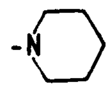 | Cl |
| Et | H | " | H | H | Cl | $-NMe_2$ | $CH_3$ |
| Me | Me | " | H | H | Cl | 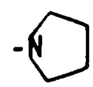 | Cl |
| OMe | H | Moc | H | H | Cl | $-NMe_2$ | Cl |
| $OCH_2Ph$ | H | " | H | H | Cl | 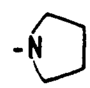 | Cl |
| OEt | H | " | H | H | Cl | 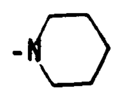 | Cl |
| OMe | H | " | H | H | Cl | $-NMe_2$ | Me |
| OMe | H | " | H | H | Cl | $-NMe_2$ | Cl |
| OMe | OMe | " | H | H | Cl | 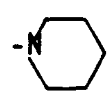 | Cl |
| OMe | OMe | " | H | H | Cl | 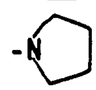 | Me |
| OEt | OEt | " | H | H | Me | 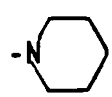 | Cl |

Die neuen Verbindungen der Formel I und ihre Salze besitzen wertvolle pharmakologische Eigenschaften.

Sie hemmen deutlich die Magensäuresekretion und weisen darüber hinaus eine ausgezeichnete Magen- und Darmschutzwirkung auf.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen)

verstanden, die beispielsweise durch Mikroorganismen, Bakterientoxine, Medikamente (z.B. Antiphlogistika und Antirheumatika), Chemikalien (z.B. Ethanol), Magensäure oder Streßsituationen verursacht werden können.

Aufgrund ihrer ausgezeichneten Eigenschaften sind die substituierten Thienoimidazole der Formel I und ihre pharmakologisch verträglichen Salze für den Einsatz in der Human- und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden.

Es wurde gefunden, daß auch die Colon-$K^+$-ATPase (vgl. [Gustin, Goodman, J. Biol. Chem. 256 [1981] 10651-10656 in vitro stark durch Verbindungen gehemmt wird, die beim Behandeln der erfindungsgemäßen Verbindungen der Formel I mit Säure (z.B. mit Natriumacetat/HCl-Puffer mit einem pH-Wert von etwa 4-5,5) entstehen. Solche Umwandlungsprodukte können sich auch in vivo bei der Passage der Verbindungen der Formel I durch den Magen-Darm-Frakt bilden. In welchem Umfang sie gebildet werden, hängt vom Substitutionsmuster und vom pH ab.

Der Colon-$K^+$-ATPase wird ein entscheidender Einfluß auf das Elektrolytgleichgewicht an der Darmschleimhaut zugeschrieben. Colon-$K^+$-ATPase-Hemmer, wie die oben genannten, können daher in dieses Gleichgewicht eingreifen und zu Behandlung von Krankheiten mit gestörtem Elektrolytgleichgewicht dienen.

Die Erfindung betrifft daher auch die Verwendung von Verbindungen der Formel I bzw. deren Säure-Umwandlungsprodukte bei der Behandlung von Durchfallerkrankungen. Beispiele solcher Krankheiten sind entzündliche Darmerkrankungen, wie Cholera, Paratyphus, Reisediarrhoe oder andere Formen der sekretorischen Diarrhoe aber auch andere Darmerkrankungen wie Colitis ulcerosa und regionale Enteritis.

Die Erfindung betrifft weiterhin Umwandlungsprodukte, die beim Behandeln von Verbindungen der Formel I mit Säure gebildet werden.

Die Erfindung betrifft daher weiter die erfindungsgemäßen Verbindungen der Formel I zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere Verbindungen der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt vorteilhafterweise zwischen 0,1 und 96% beträgt.

Welche Hilfsstoffe für die gewünschte Arzneimittelformulierungen geeignet sind, ist dem Fachmann aufgrund seines Wissens geläufig. Neben Lösemitteln, Gelbildern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können oral oder parenteral appliziert werden, wobei die orale Applikation bevorzugt ist.

Im allgemeinen hat es sich in der Humanmedizin als vorteilhaft erwiesen, den oder die Wirkstoffe bei oraler Gabe in einer Tagesdosis von etwa 0,01 bis etwa 20 mg/kg Körpergewicht, gegebenenfalls in Form mehrerer, vorzugsweise 1 bis 4 Einzelgaben zur Erzielung des gewünschten Ergebnisses zu verabreichen. Bei der parenteralen Applikation können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der obengenannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Tranquilizer, wie Benzodiazepine, beispielsweise Diazepam; Spasmolytika, wie z.B. Bietamiverin, Camylofin; Anticholinergica, wie z.B. Oxyphencylimin, Phencarbamid; Lokalanaesthetika, wie z.B. Tetracain, Procain; gegebenenfalls auch Gastrinantagonisten, Fermente, Vitamine oder Aminosäuren enthalten.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi

arabicum, Magnesia, Magnesiumcarbonat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propanol oder Glycerin, daneben auch Zuckerlösungen wie Glucose- oder Mannitlösungen, oder auch eine Mischung aus den verschiedenen genannten Lösungsmitteln.

Die nachfolgenden Beispiele sollen die erfindungsgemäßen Verfahrensweisen erläutern.

Die angegebenen Schmelz- und Zersetzungspunkte sind nicht korrigiert oder standardisiert.

Beispiel 1:

2-(4-Methoxy-2-picolylmercapto)-1H-thieno[3,4-d]imidazol-Di-hydrochlorid

1,6 g 2-Mercapto-thieno[3,4-d]imidazol und 2 g 4-Methoxypicolylchlorid-Hydrochlorid werden in 50 ml Ethanol etwa eine Stunde auf 60 ° C erwärmt und weitere 40 Stunden bei Raumtemperatur gerührt. Nach dem Abfiltrieren schlämmt man die kristalline Substanz in Aceton auf, rührt eine Stunde bei Raumtemperatur, saugt die Kristalle ab und trocknet an der Luft.
Farblose Kristalle, Fp. 330 ° C.

Beispiel 2:

2-(4-Methoxy-2-picolylmercapto)-1H-thieno[3,4-d]imidazol

2,1 g 2-(4-Methoxy-2-Picolylmercapto)-1H-thieno[3,4-d]imidazol Dihydrochlorid werden nach dem Suspendieren in 100 ml Methanol mit 1,9 g Triethylamin versetzt. Man rührt die erhaltene Lösung etwa eine Stunde bei Raumtemperatur, destilliert das Lösungsmittel ab. Nach Zugabe von 50 ml Wasser rührt man etwa eine Stunde bei Raumtemperatur, saugt die Kristalle ab und kristallisiert nach dem Trocknen aus Ethanol in Gegenwart von Aktivkohle um.
Farblose Kristalle, Fp. 172-175 ° C.

Beispiel 3:

2-(4-Methoxy-2-picolylsulfinyl)-1H-thieno[3,4-d]imidazol

0,9 g 2-(4-Methoxy-2-picolylmercapto-1H-thieno[3,4-d]imidazol werden bei Raumtemperatur mit 50 ml Methylenchlorid und nach dem Abkühlen auf 0 ° C sodann portionsweise mit 0,64 g 3-Chlorperbenzoesäure versetzt. Man rührt etwa 5 Minuten unter Beibehaltung der Kühlung und nach Zugabe von 20 ml gesättigter wäßriger Natriumbicarbonatlösung weitere 10 Minuten bei Raumtemperatur. Nach Abtrennen der organischen Phase und Trocknen über Natriumsulfat wird das Lösungsmittel abdestilliert, der Rückstand mit einem Gemisch aus Diisopropylether und Aceton gerührt und die Kristalle abfiltriert und getrocknet.
Farblose Kristalle, Fp. 142-144 ° C.

Beispiel 4:

2-(4-Methoxycarbonyl-2-picolylmercapto)-1H-thieno[3,4-d]imidazol-Dihydrochlorid

Die Titelverbindung erhält man analog der in Beispiel 1 angegebenen Vorschrift aus 2-Mercapto-6-methoxycarbonylthieno-[3,4-d]imidazol und 4-Methoxy-2-picolylchlorid-Hydrochlorid.
Farblose Kristalle, Fp. 210-213 ° C.

Beispiel 5:

6-Methoxycarbonyl-2-(4-methoxy-2-picolylmercapto)-1H-thieno[3,4-d]imidazol

Die Titelverbindung erhält man analog der in Beispiel 2 angegebenen Vorschrift aus der Verbindung von Beispiel 4.
Farblose Kristalle, Fp. 156-160 ° C.

Beispiel 6:

2-(2-Picolylmercapto)-1H-thieno[3,4-d]imidazol-Dihydrochlorid

Die Titelverbindung erhält man analog der in Beispiel 1 angegebenen Vorschrift aus 2-Picolylchlorid-Hydrochlorid und 2-Mercapto-1H-thieno[3,4-d]imidazol in Isopropanol als Lösungsmittel.
Farblose Kristalle, Fp. 154-162 ° C.

Beispiel 7:

4-Methoxycarbonyl-2-(2-picolylmercapto)-1H-thieno[3,4-d]imidazol-Hydrat-Hydrochlorid

Die Titelverbindungen erhält man analog der in Beispiel 6 angegebenen Vorschrift aus 2-Mercapto-4-methoxycarbonyl-1H-thieno[3,4-d]imidazol und 2-Picolylchlorid-Hydrochlorid.
Farblose Kristalle, Fp. 204-208 ° C.

Beispiel 8:

2-(5-Methyl-2-picolylsulfinyl)-1H-thieno[3,4-d]imidazol Natriumsalz

0,036 g Ätznatron werden in 15 ml Methanol gelöst und die Lösung nach dem Zusetzen von 0,24 g 2-(5-Methyl-2-picolylsulfinyl)-1H-thieno[3,4-d]imidazol 30 Minuten bei Raumtemperatur gerührt. Nach dem Abdestillieren des Lösungsmittels unter vermindertem Druck läßt man aus Essigester kristallisieren und filtriert ab.
Farblose Kristalle, Fp. 320 ° C.

Beispiel 9:

2-(5-Methyl-2-picolylsulfonyl)-1H-thieno[3,4-d]imidazol

Zu einer Zweiphasenmischung bestehend aus 20 ml Methylenchlorid, 20 ml gesättigter wäßriger Natriumcarbonat-Lösung und 1 g 2-(5-Methyl-2-picolylmercapto)-1H-thieno[3,4-d]imidazol wird bei 0 ° C eine Lösung von 1,34 g 3-Chlorperbenzoesäure in 25 ml Methylenchlorid tropfenweise zugegeben. Man rührt 30 Minuten bei 0 - 5 ° C, trennt die organische Phase ab und destilliert das Lösungsmittel nach Trocknen über Calciumchlorid ab. Der dunkle Rückstand wird an Kieselgel durch Säulenchromatographie mittels Essigsäureethylester/Methanol = 8:1 als mobiler Phase gereinigt und aus Essigester kristallisiert.
Farblose Kristalle, Fp. 163 ° C

Beispiel 11:

3-Chlor-4-methoxy-2-picolin-N-oxid:

Zu einer Natriummethylatlösung, dargestellt aus 0,51 g Natrium und 20 ml Methanol, gibt man bei -10 ° C 3,5 g 3,4-Dichlor-2-picolin-N-oxid in 20 ml wasserfreiem Methanol. Man läßt langsam auf Raumtemperatur erwärmen und erhitzt sodann 1 Stunde am Rückfluß. Nun destilliert man das Lösungsmittel unter vermindertem Druck ab, versetzt den Rückstand mit Wasser, extrahiert mit Dichlormethan und vertreibt das Lösungsmittel.
Farblose Kristalle aus Diisopropylether, Fp. 94 - 97 ° C.

Beispiel 12:

3-Chlor-2-hydroxymethyl-4-methoxypyridin:

5,8 g 3-Chlor-4-methoxy-2-picolin-N-oxid werden in 8 ml Eisessig gelöst und unter Rühren bei 90 ° C

mit 14 ml Acetanhydrid versetzt. Man erhitzt 2 Stunden auf 110-115° C läßt sodann auf 80° C abkühlen und tropft 25 ml Methanol zu. Anschließend wird unter vermindertem Druck das Lösungsmittel abdestilliert, der Rückstand sodann mit 20 ml Wasser und 8 g Ätznatron in kleinen Portionen versetzt und diese Mischung 2 Stunden zum Rückfluß erhitzt. Nach dem Abkühlen extrahiert man mit Dichlormethan, vertreibt das Lösungsmittel und bringt den Rückstand mit Diethylether zur Kristallisation.
Feststoff, Fp. 103-105° C.

Beispiel 13:

3-Chlor-2-chlormethyl-4-methoxypyridin-Hydrochlorid:

Zu einer Mischung aus 2,6 g 3-Chlor-2-hydroxymethyl-4-methoxypyridin und 30 ml Dichlormethan tropft man bei -10 bis -15° C eine Lösung aus 3,5 ml Thionylchlorid in 25 ml Dichlormethan und rührt sodann 2 Stunden bei Raumtemperatur. Man vertreibt das Lösungsmittel und bringt den Rückstand mit Diethylether zur Kristallisation.
Farblose Kristalle, Fp. 145-146° C.

Beispiel 15:

1- Ethoxycarbonyl -2-(4-methoxy-2-picolylmercapto)-1H-thieno[3,4-d]imidazol

Unter Stickstoff werden 1,4 g (5,0 mmol) 2-(4-Methoxy-2-picolylmercapto)-1H-thieno[3,4-d]imidazol in 15 ml wasserfreiem Dimethylformamid gelöst, 270 mg (6 mmol) einer 60 %igen öligen NaH-Suspension portionsweise hinzugefügt und 10 Minuten auf 30-40° C erwärmt. Man gibt nun bei 25° C 0,5 ml (5 mmol) Chlorameisensäureethylester (95%) hinzu, wobei sich die Temperatur auf ca. 36° C erhöht. 30 Minuten später wird das kristalline Produkt abgesaugt, 2 mal mit Diethylether gewaschen.
Fp. 154-156° C (Zers.)

Beispiel 16:

1- Ethoxycarbonyl -2-(4-methoxy-2-picolylsulfinyl)-1H-thieno[3,4-d]imidazol

Zu 750 mg (2,1 mmol) 1- Ethoxycarbonyl -2-(4-methoxy-2-picolylmercapto)-1H-thieno[3,4-d]imidazol in 30 ml Methylenchlorid und 25 ml 0,5 n wäßriger Natriumhydrogencarbonat-Lösung werden unter Rühren zunächst 420 mg (2,1 mMol), dann nochmals 210 mg (1.05 mmol) 3-Chlorperbenzoesäure in $CH_2Cl_2$ hinzugetropft. Die organische Phase wird über $MgSO_4$ getrocknet, im Vakuum eingeengt und der Rückstand aus Essigester kristallisiert.
Fp. 143° C (Zers.)

Beispiel 17:

1- Vinyloxycarbonyl -2- (5-methyl-2-picolylmercapto)-1H-thieno[3,4-d]imidazol

Analog Beispiel 15 erhält man aus 2,1 g ( 8 mmol) 2-(5-Methyl-2-picolylmercapto)-1H-thieno[3,4-d]-imidazol und 0,85 g (0,72 ml, 8 mmol) Chlorameisensäurevinylester 1,5 g Rohprodukt, welches an $SiO_2$ -($CH_2Cl_2$/MeOH 50:1) chromatographiert wird. 1,1 g Titelverbindung, die aus Diisopropylether kristallisieren, werden erhalten.
Fp. 78 - 80° C.

Beispiel 18:

1- Vinyloxycarbonyl -2-(5-methyl-2-picolylsulfinyl)-1H-thieno[3,4-d]imidazol

Analog Beispiel 16 werden 0,5 g (1,5 mmol) 1- Vinyloxycarbonyl -2-(5-methyl-2-picolylmercapto)-1H-thieno[3,4-d] imidazol mit m-Chlorperbenzoesäure oxidiert, allerdings im 2-Phasengemisch aus Methylenchlorid und wäßriger $KH_2PO_4$/$Na_2HPO_4$-Pufferlösung (pH = 7,5).
Chromatographie erfolgt mit $CH_2Cl_2$/$CH_3OH$ (30:1) an $SiO_2$ Fp. 162° C

Beispiel 19:

1- Benzyloxycarbonyl -2-(4-methoxy-2-picolylmercapto)-1H-thieno[3,4-d]imidazol

1,4 g (5 mmol)2-(4-Methoxy-2-picolylmercapto)-1H-thieno[3,4-d] imidazol werden analog Beispiel 15 mit 0,8 ml (5 mmol) Chlorameisensäurebenzylester (90 - 95 %) umgesetzt. Es werden 2,2g öliges Rohprodukt erhalten, welches mit Toluol/Essigester (1:5) an Kieselgel (35 - 70 µ) chromatographiert wird. Das Produkt kristallisiert aus Diethylether.
Fp. 102 - 104 ° C.

Beispiel 20:

1-Benzyloxycarbonyl)-2-(5-methyl-2-picolylmercapto)-1H-thieno[3,4-d]imidazol

2-(5-Methyl-2-picolylmercapto)-1H-thieno[3,4-d]imidazol wird analog Beispiel 19 umgesetzt. Das DMF wird im Vakuum abdestilliert, der Rückstand in $CH_2Cl_2$ aufgenommen, mit Wasser ausgeschüttelt und über $MgSO_4$ getrocknet. Nach Einengen kristallisiert die Titelverbindung aus Essigester.
Fp. 103 - 104 ° C

Beispiel 21:

1-(4-Methoxybenzyloxycarbonyl)-2-(5-methyl-2-picolylmercapto)-1H-thieno[3,4-d]imidazol

Zu 1,3 g ( 5mmol) 2-(5-Methyl-2-picolylmercapto)-1H-thieno[3,4-d]imidazol, gelöst in 15 ml wasserfreiem DMF, werden unter Stickstoff 275 mg (6 mmol) Natriumhydrid gegeben. Nachdem 10 min auf 40-50 ° C erwärmt wurde, gibt man bei Raumtemperatur 1,92 g (7,5 mmol) 4-Methoxybenzyl-phenyl-carbonat (hergestellt aus 4-Methoxybenzylalkohol und Chlorameisensäurephenylester) zu, erwärmt 10 Minuten auf 30-40 ° C und rührt 1 Stunde bei Raumtemperatur. Das Lösungsmittel wird im Vakuum abdestilliert und der Rückstand mit Wasser versetzt. Die ölig-harzige Fällung wird in $CH_2Cl_2$ aufgenommen, die Lösung über $MgSO_4$ getrocknet und das Lösungsmittel abgedampft. Der Rückstand kristallisiert aus Diethylether und wird aus Isopropanol umkristallisiert.
Fp. 120 - 121 ° C.

Beispiel 22:

1-(4-Methoxybenzyloxycarbonyl)-2-(5-methyl-2-picolylsulfinyl)-1H-thieno[3,4-d]imidazol

850 mg (2 mmol) der Titelverbindung aus Beispiel 21 werden in 50 ml $CH_2Cl_2$ gelöst, mit 50 ml wäßriger $Na_2HPO_4/KH_2PO_4$-Pufferlösung (pH 7,5; 7,4 ml $KH_2PO_4$-Lösung (45,35 g/l) + 42,5 ml $Na_2HPO_4$-Lösung (59,5 g/1)) versetzt. Unter starkem Rühren tropft man bei Raumtemperatur 500 mg (2,5 mmol) m-Chlorperbenzoesäure, gelöst in $CH_2Cl_2$, zu. Die organische Phase wird über $MgSO_4$ getrocknet, eingeengt und der Rückstand mit Essigester an Kieselgel chromatographiert. Die Titelverbindung kristallisiert aus Isopropanol.
Fp. 119 - 120 ° C.

Beispiel 23:

1- tert. Butoxycarbonyl -2-(5 methyl-2-picolylmercapto)-1H-thieno[3,4-d]imidazol

2 g (7,7 mmol) 2-(5-Methyl-2-picolylmercapto)-1H-thieno-[3,4-d] imidazol werden in 25 ml DMF gelöst, mit 1,2 ml Triethylamin und 1,85 g (8,5 mmol) Di-tert.-butyl-dicarbonat versetzt. Nach 2 Stunden werden weitere 3 g des Dicarbonats zugefügt und 4 Stunden bei 70 ° C gerührt. Nachdem DMF weitgehend abgedampft wurde, nimmt man den Rückstand in $CH_2Cl_2$ auf, schüttelt mit Wasser, trocknet über $MgSO_4$ und engt ein. Der Rückstand kann aus Diisopropylether oder Petrolether kristallisiert werden.
Fp. 115 - 117 ° C.

Beispiel 24:

1- tert.-Butoxycarbonyl -2-(5-methyl-2-picolylsulfinyl)-1H-thieno[3,4-d]imidazol

1,1 g (3 mmol) der Titelverbindung aus Beispiel 23 werden in 50 ml $CH_2Cl_2$gelöst und mit 50 ml der $KH_2PO_4$/$Na_2HPO_4$-Pufferlösung aus Beispiel 22 versetzt. Portionsweise tropft man bei 10 °C insgesamt 900 mg (4,5 mmol) m-Chlorperbenzoesäure in $CH_2Cl_2$ zu, bis das Edukt vollständig verbraucht ist.

Die organische Phase wird abgetrennt, mit Wasser gewaschen, getrocknet und eingeengt.

Der Rückstand wird zunächst mit Essigester an Kieselgel chromatographiert. Man kristallisiert die entsprechenden Fraktionen aus Diethylether/Petrolether und erhält die Titelverbindung.

Fp. 98 °C (Zers.)

Beispiel 25:

1- tert.-Butoxycarbonyl -2-(5-methyl-2-picolylsulfonyl)-1H-thieno[3,4-d]imidazol

Die Titelverbindung erhält man, indem bei der säulenchromatographischen Reiniging in Beispiel 24 mit Methanol/Essigester (1:20) weitereluiert wird.

Fp. 127 °C (Zers.)

Beispiel 26:

1- tert.-Butoxycarbonyl -2-(4-methoxy-2-picolylmercapto)-1H-thieno[3,4-d]imidazol

1,1 g (4,0 mMol) 2-(4-Methoxy-2-picolylmercapto)-1H-thieno[3,4-d]imidazol werden in 15 ml wasserfreiem DMF gelöst, mit 0,6 ml Triethylamin und 0,96 g (ca. 4,5 mmol) Di-tert.-butyl-dicarbonat versetzt. Nach 2 Stunden Rühren bei Raumtemperatur werden nochmals 0,32 g (1,5 mmol) Ditert.-butyl-dicarbonat hinzugefügt.

Das ausgefallene Produkt wird abgesaugt; die Lösung mit Wasser versetzt, mit $CH_2Cl_2$ extrahiert, die organische Phase über $MgSO_4$ getrocknet und im Vakuum eingeengt. Der ölige Rückstand kristallisiert aus Diethylether.

Fp. 152 °C (Zers.)

Beispiel 27:

1-(p-Nitrophenyl-oxycarbonyl)-2-(5 methyl-2-picolylmercapto)-1H-thieno[3,4-d]imidazol

Die Titelverbindung wird analog Beispiel 17 aus 2-(5-Methyl-2-picolylmercapto)-1H-thieno[3,4-d]imidazol und Chlorameisensäure-p-nitrophenylester hergestellt. Nach Aufarbeitung und Chromatographie mit Toluol/Essigester (1:1) an Kieselgel kristallisiert man die entsprechenden Fraktionen aus Essigester und erhält die Titelverbindung.

Fp. 165 - 168 °C

Beispiel 28:

1- Hydroxymethyl -2-(4-methoxy-2-picolylmercapto)-1H-thieno[3,4-d]imidazol

Unter Stickstoffatmosphäre werden zu 1,6 g (5,8 mmol) 2-(4-Methoxy-2-picolylmercapto)-1H-thieno[3,4-d]imidazol gelöst in 50 ml Acetonitril, 0,7 ml 37%ige wäßrige Formaldehyd-Lösung in 3 ml Acetonitril hinzugetropft. Anschließend wird 15 Minuten bei 70 °C gerührt. Die im Vakuum konzentrierte Lösung wird mit Wasser und gesättigter wäßriger NaCl-Lösung gewaschen und über $MgSO_4$ getrocknet. Der nach Eindampfen erhaltene Rückstand ergibt nach Behandeln mit Diisopropylether ein halbkristallines Rohprodukt, das aus Essigester kristallisiert.

Fp. 125 - 127 °C.

Beispiel 29:

1- Acetoxymethyl -2-(4-methoxy-2-picolylmercapto)-1H-thieno[3,4-d]imidazol

1,3 g (4,2 mmol) der Titelverbindung von Beispiel 28 werden in 25 ml wasserfreiem Pyridin gelöst und

mit 50 mg 4-Dimethylaminopyridin versetzt. Unter Stickstoffatmosphäre und Rühren tropft man 6,3 ml Acetanhydrid zu und rührt eine Stunde bei Raumtemperatur. Anschließend gießt man auf Eiswasser, extrahiert mit Methylenchlorid, trocknet über $MgSO_4$ und engt die organische Phase im Vakuum ein. Der kristalline Feststoff wird aus Ethanol umkristallisiert.
Fp. 111 - 113 °C

Beispiel 30

1- Hydroxymethyl -2-(5-methyl-2-picolylmercapto)-1H-thieno[3,4-d]imidazol

Die Titelverbindung wird analog Beispiel 28 aus 2-(5-Methyl-2-picolylercapto)-1H-thieno[3,4-d]imidazol hergestellt.

Beispiel 31:

1- Acetoxymethyl -2-(5-methyl-2-picolyl-mercapto)-1H-thieno[3,4-d]imidazol

Die Titelverbindung wird analog Beispiel 29 aus der Titelverbindung des Beispiels 30 erhalten. Das erhaltene Rohprodukt wird chromatographisch an Kieselgel (Essigsäureethylester/Toluol = 2:1) gereinigt und kristallisiert beim Anreiben spontan aus Diisopropylether.
Farblose Kristalle, Fp. 87 - 89 °C.

Beispiel 32:

1- Acetoxymethyl -2-(5-methyl-2-picolylsulfinyl)-1H-thieno[3,4-d]imidazol

0,67 g (2 mmol) der Titelverbindung aus Beispiel 31 werden in 30 ml wasserfreiem $CH_2Cl_2$ gelöst und unter Stickstoffatmosphäre mit 0,6 ml (2 mmol) Titantetraisopropylat versetzt. Sodann werden bei 0 °C 0,6 ml (2 mmol) einer 3 M Lösung von tert.-Butylhydroperoxid in Toluol zugetropft. Nach 30 Minuten läßt man auf Raumtemperatur kommen, rührt weitere 20 Stunden, versetzt mit Wasser, filtriert vom ausgefallenen weißen Feststoff ab, trocknet die organische Phase über $MgSO_4$, destilliert das Lösungsmittel im Vakuum ab und chromatographiert das Rohprodukt mit Toluol/Essigester (1:5) an Kieselgel. Die Titelverbindung ($R_f$ = 0,18) kristallisiert farblos aus Diisopropylether.
Fp. 104 - 106 °C.

Beispiel 33:

1- Hydroxymethyl -2-(4-piperidino-3-chlor-2-picolylmercapto)-1H-thieno[3,4-d]imidazol

Die Titelverbindung wird analog Beispiel 28 aus 2-(4-Piperidino-3-chlor-2-picolylmercapto)-1H-thieno-[3,4-d]imidazol erhalten.
Fp. 132 - 134 °C.

Beispiel 34:

1- Acetoxymethyl -2-(4-piperidino-3-chlor-2-picolylmercapto)-1H-thieno[3,4-d]imidazol

Die Titelverbindung wird analog Beispiel 28 aus der Titelverbindung des Beispiels 33 erhalten. Das Rohprodukt wird mit Toluol/Essigester (1:1) an Kieselgel chromatographiert.
Fp. 169 -170 °C.

Beispiel 35:

1-(4-Methoxybenzyl)-2-(5-methyl-2-picolyl-mercapto)-1H-thieno[3,4-d]imidazol

Aus 2,6 g (10 mmol) 2-(5-Methyl-2-picolylmercapto)-1H-thieno[3,4-d]imidazol wird analog Beispiel 15 das entsprechende Natriumsalz hergestellt und mit 1,7 g (11 mmol) 4-Methoxybenzylchlorid alkyliert. Nach Aufarbeitung wird mit $CH_2Cl_2$/Methanol (50:1) an Kieselgel chromatographiert. Die entsprechenden Fraktionen werden aus Diethylether umkristallisiert und man erhält die Titelverbindung.
Fp. 114 -116 °C.

Beispiel 36:

1-(4-Methoxybenzyl)-2-(5-methyl-2-picolylsulfonyl)-1H-thieno[3,4-d]imidazol

Die Titelverbindung aus Beispiel 35 wird in $CH_2Cl_2$, $Na_2HPO_4/KH_2PO_4$-Puffer (wie in Beispiel 22 beschrieben) mit 2 Äquivalenten m-Chlorperbenzoesäure oxidiert. Das Rohprodukt wird chromatographisch (Kieselgel, Toluol/Essigester 1:4) gereinigt. Die Titelverbindung kristallisiert aus wenig Diisopropylether.
Fp. 148 - 150 °C.

Beispiel 37:

1-Acetyl-2-(5-methyl-2-picolylmercapto)-1H-thieno [3,4-d]imidazol

Die Titelverbindung erhält man analog Beispiel 29 durch Umsetzung von 2-(5-Methyl-2-picolyl-mercapto)-1H-thieno[3,4-d]imidazol mit Pyridin/Acetanhydrid/Dimethylaminopyridin. Nach Aufarbeitung wird das Rohprodukt in wenig $CH_2Cl_2$ gelöst und mit Toluol/Essigester (1:1) an Kieselgel chromatographiert. Aus entsprechenden Fraktionen kristallisiert die Titelverbindung.
Fp. 139 - 141 °C.

Beispiel 38:

1-(1-Acetoxy-ethoxy-carbonyl)-2-(5-methyl-2-picolylmercapto)-1H-thieno[3,4-d] imidazol

Analog Beispiel 15 wird aus 2,6 g (10 mmol) 2-(5-Methyl-2-picolyl-mercapto)-1H-thieno[3,4-d]imidazol das Natriumsalz hergestellt. Bei -10 °C tropft man eine Lösung von 2,7 g (10 mmol) Acetoxy-1-ethyl-(p-nitrophenyl)carbonat in DMF zu. Man erwärmt auf Raumtemperatur und engt die Reaktionsmischung nach 2 Stunden im Vakuum ein, versetzt mit Wasser, extrahiert mit $CH_2Cl_2$, trocknet über $MgSO_4$ und engt ein.

Der Rückstand wird mit Toluol/Essigester (3:1) an Kieselgel chromatographiert. Die Titelverbindung kristallisiert aus entsprechenden Fraktionen.
Fp. 111 - 113 °C.
Daneben wird in geringer Menge die Titelverbindung des Beispiels 36 erhalten.

Die Verbindungen der nachstehenden Tabelle 2 werden in analoger Weise hergestellt.

Tabelle 2

| Beispiel Nr. | A | T | $R^3$ | $R^4$ | $R^5$ | $R^6$ | $R^7$ | $R^8$ | $R^9$ | | Fp. |
|---|---|---|---|---|---|---|---|---|---|---|---|
| 39. | 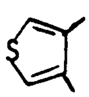 | S | H | H | H | H | $O-CH_2-C_6H_5$ | H | H | x 2HCl | 177°C (Zers.) |
| 40. | " | S | H | H | H | H | $-O-CH_2-C_6H_5$ | H | H | | 186°C (Zers.) |
| 41. | " | S | H | H | H | H | $OC_2H_5$ | H | H | x 2HCl | 206°C |

Tabelle (Fortsetzung)

| Bei-spiel Nr. | A | T | $R^3$ | $R^4$ | $R^5$ | $R^6$ | $R^7$ | $R^8$ | $R^9$ | | Fp. |
|---|---|---|---|---|---|---|---|---|---|---|---|
| 42. | 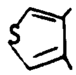 | S | H | H | H | H | $CH_3$ | H | H | x 2HCl | 204°C |
| 43. | " | S | H | H | H | H | H | $CH_3$ | H | x 2HBr | 218°C |
| 44. | " | S | H | H | H | H | H | $CH_3$ | H | | 136°C |
| 45. | " | S | H | H | H | $CH_3$ | H | $CH_3$ | H | x 2HCl | 207°C (Zers.) |
| 46. | " | SO | H | H | H | $CH_3$ | H | $CH_3$ | H | | 156°C (Zers.) |
| 47. | " | S | H | H | H | $CH_3$ | $OCH_3$ | $CH_3$ | H | | 108-112°C |
| 48. | " | S | H | H | H | $CH_3$ | $OCH_3$ | H | H | | 174-177°C |
| 49. | " | SO | H | H | H | $CH_3$ | $OCH_3$ | $CH_3$ | H | | 190°C (Zers.) |
| 50. | " | SO | H | H | H | $CH_3$ | $OCH_3$ | H | H | | 145°C (Zers.) |
| 51. | " | S | H | H | H | H | H | H | H | | 126-129°C |
| 52. | " | SO | H | H | H | H | H | H | H | | 148-149°C |
| 53. | " | SO | H | H | H | H | $OC_2H_5$ | H | H | | 135°C (Zers.) |
| 54. | " | S | H | H | H | $CH_3$ | H | H | H | | >320°C (Zers.) |
| 55. | " | SO | H | H | H | $CH_3$ | H | H | H | | ab 155°C (Zers.) |
| 56. | " | SO | H | H | H | H | H | $CH_3$ | H | | 120-123°C |
| 57. | 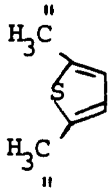 | S | H | H | H | H | H | H | H | x 2HCl | 330°C |
| 58. | " | SO | H | H | H | H | H | H | H | | 155-157°C |
| 59. | " | S | H | H | H | H | $OCH_3$ | H | H | | 217-222°C |
| 60. | " | SO | H | H | H | H | $OCH_3$ | H | H | | 170-174°C |
| 61. | 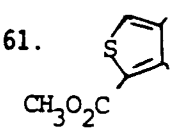 | SO | H | H | H | H | $OCH_3$ | H | H | | 142°C |
| 62. | 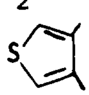 | S | H | H | H | H | 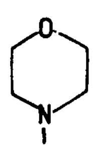 | H | H | x 2HCl | >320°C |
| 63. | 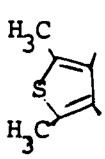 | S | H | H | H | H | H | $CH_3$ | H | x 2HCl | 250°C |

Tabelle (Fortsetzung)

| Beispiel Nr. | A | T | $R^3$ | $R^4$ | $R^5$ | $R^6$ | $R^7$ | $R^8$ | $R^9$ | Fp. |
|---|---|---|---|---|---|---|---|---|---|---|
| 64. | 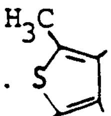 | S | H | H | H | H | H | $CH_3$ | H | 164°C (Zers.) |
| 65. | 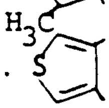 | S | H | H | $-CH_2-CH_2-$ | | H | H | H | 147°C |
| 66. | " | SO | H | H | $-CH_2-CH_2-$ | | H | H | H | 93°C (Zers.) |
| 67. | " | S | H | H | H | H | H | $C_2H_5$ | H | x 2HCl 183°C (Zers.) |
| 68. | " | SO | H | H | H | H | H | $C_2H_5$ | H | >85°C (Zers.) |
| 69. | " | S | H | H | H | H | $-O-CH_2-C_6H_5$ | H | H | x 2HCl 181°C (Zers.) |
| 70. | " | SO | H | H | H | H | $-O-CH_2-C_6H_5$ | H | H | 174°C (Zers.) |
| 71. | " | S | H | H | H | H | $-O-(CH_2)_2-O-CH_3$ | H | H | x 2HCl 170°C (Zers.) |
| 72 | " | S | H | H | H | H | $-O-(CH_2)_2-O-CH_3$ | H | H | 114°C |
| 73 | " | SO | H | H | H | H | $-O-(CH_2)_2-OCH_3$ | H | H | 105°C |
| 74 | " | S | H | H | H | H | H | H | $CH_3$ | x2HCl>300°C |
| 75 | " | SO | H | H | H | H | H | H | $CH_3$ | 125°C |
| 76 | " | S | H | H | H | Cl | 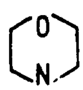 | H | H | x 2HCl 194°C (Zers.) |
| 77 | " | S | H | H | H | Cl | " | H | H | >90°C |
| 78 | " | S | H | H | H | Cl | $OCH_3$ | H | H | x 2HCl>250°C |
| 79 | " | S | H | H | H | Cl | $OCH_3$ | H | H | 156°C |
| 80 | " | SO | H | H | H | Cl | $OCH_3$ | H | H | ab 160°C (Zers.) |

$H_3CO_2C$

Tabelle (Fortsetzung)

| Beispiel Nr. | A | T | $R^3$ | $R^4$ | $R^5$ | $R^6$ | $R^7$ | $R^8$ | $R^9$ | Fp. |
|---|---|---|---|---|---|---|---|---|---|---|
| 82 | 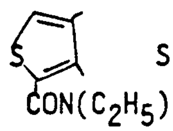 | S | H | H | H | H | H | H | H | 102°C |
| 83 | 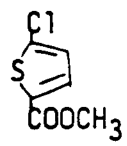 | S | H | H | H | H | $OCH_3$ | H | H x 2HCl | 168°C |

**Patentansprüche**

**Patentansprüche für folgende Vertragsstaaten : BE, CH, DE, FR, GB, IT, LI, LU, NL, SE**

**1.** Verbindung der Formel I

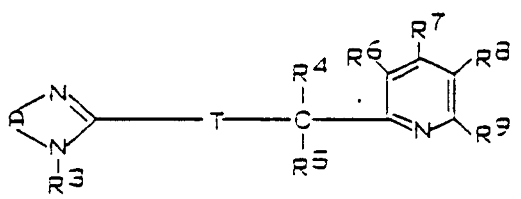

(I)

in welcher

A für

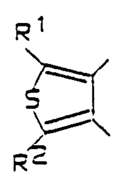

steht,

T -S-, -SO- oder $-SO_2-$ bedeutet,

$R^1$ und $R^2$ gleich oder verschieden sind un Wasserstoff, Halogen Cyano, Nitro, Trifluormethyl, $(C_1-C_6)$-Alkyl, $(C_1-C_6)$-Hydroxyalkyl, $(C_1-C_6)$-Alkoxy, $(C_1-C_4)$-Fluoralkoxy, $-OCF_2Cl$, $-O-CF_2-CHFCl$, $(C_1-C_6)$-Alkylmercapto, $(C_1-C_6)$-Alkylsulinyl, $(C_1-C_6)$-Alkylsulfonyl, $(C_1-C_6)$-Alkylcarbonyl, $(C_1-C_6)$-Alkoxycarbonyl, Carbamoyl, $N(C_1-C_4)$-Alkylcarbamoyl, N,N-Di$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, $N(C_1-C_4)$-Alkylsulfamoyl oder N,N-Di-$(C_1-C_4)$-alkylsulfamoyl bedeuten,

$R^3$ Wasserstoff, Alkanoyl, $(C_1-C_6)$-Alkylcarbamoyl oder eine andere physiologisch verträgliche, vorzugsweise im sauren Medium und/oder unter physiologischen Bedingungen abspaltbare $N^{im}$-Schutzgruppe bedeutet,

$R^4$ und $R^5$ gleich oder verschieden sind und Wasserstoff oder $(C_1-C_3)$-Alkyl bedeuten,

$R^6$, $R^7$, $R^8$ und $R^9$ gleich oder verschieden sind und Wasserstoff, Halogen, $(C_1-C_{12})$-Alkyl, $(C_1-C_{12})$-Alkoxy, -O-$CH_2$-$C_fH_{(2f+1-g)}F_g$, -NR'R", $(C_1-C_{12})$-Alkoxy-$(C_1-C_{12})$-alkyl, $(C_1-C_{12})$-Alkoxy-$(C_1-C_{12})$-alkoxy, $(C_7-C_{11})$-Aralkyloxy, $(C_1-C_{12})$-Alkylmercapto, $(C_1-C_{12})$-Alkylsulfinyl oder $(C_1-C_{12})$-Alkylsulfonyl bedeuten, oder

$R^5$ und $R^6$ gemeinsam für -$[CH_2]_i$- stehen,

R' und R" gleich oder verschieden sind und Wasserstoff oder $(C_1-C_4)$-Alkyl bedeuten oder

R' und R" gemeinsam für -$[CH_2]_h$- stehen, worin eine $CH_2$-Gruppe durch O, S, N-$(C_1-C_4)$-Alkanoylimino oder N-$(C_1-C_4)$-Alkoxylcarbonylimino ersetzt sein kann,

f = 1, 2, 3 oder 4 ist,

g = 1 bis (2f + 1) ist,

h = 4, 5 oder 6 ist,

i = 1, 2 oder 3 ist und

n = 3 oder 4 ist,

sowie deren physiologisch verträglichen Salze.

**2.** Verbindung der Formel I gemäß Anspruch 1, in welcher $R^9$ Wasserstoff bedeutet.

**3.** Verbindung der Formel I gemäß einem der Ansprüche 1 oder 2, in welcher T für -SO- steht.

**4.** Verbindung der Formel I gemäß einem der Ansprüche 1 bis 3, in welcher

$R^1$ und $R^2$ gleich oder verschieden sind und Wasserstoff, $(C_1-C_3)$-Alkyl, Halogen, $(C_1-C_4)$-Alkoxy, oder $(C_1-C_4)$-Alkoxycarbonyl bedeuten,

$R^3$ wie im Anspruch 1 definiert ist,

$R^4$ und $R^5$ jeweils Wasserstoff bedeuten und

$R^6$, $R^7$, $R^8$ und $R^9$ gleich oder verschieden sind und Wasserstoff, Halogen, $(C_1-C_3)$-Alkyl, $(C_1-C_4)$-Alkoxy, Benzyloxy oder $(C_1-C_7)$Alkoxy-$(C_1-C_3)$-alkyl bedeuten.

**5.** Verbindung der Formel I gemäß einem der Ansprüche 1 bis 4, in welcher

$R^1$ und $R^2$ gleich oder verschieden sind und Wasserstoff oder $(C_1-C_3)$-Alkyl bedeuten,

$R^3$ wie in Anspruch 1 definiert ist,

$R^4$ und $R^5$ jeweils Wasserstoff bedeuten,

$R^6$ und $R^8$ gleich oder verschieden sind und Wasserstoff, Chlor, Methyl oder Ethyl bedeuten,

$R^9$ Wasserstoff bedeutet und

$R^7$ Wasserstoff, $(C_1-C_4)$-Alkoxy, $(C_1-C_3)$-Alkyl oder Benzyloxy bedeutet.

**6.** 2-[4-(2,2,3,3,4,4,4-Heptafluorbutyloxy)-2-picolylsulfinyl]-1H-thieno[3,4-d]imidazol oder dessen physiologisch verträglichen Salze.

**7.** Verfahren zur Herstellung einer Verbindung der Formel 1 gemäß einem der Ansprüche 1 bis 6, dadurch gekennzeichnet, daß man

a) eine Verbindung der Formel II

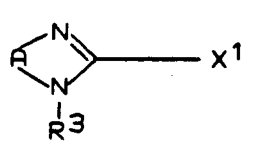

(II)

in welcher A, $R^1$, $R^2$ und $R^3$ wie im Anspruch 1 definiert sind und

$X^1$ i. eine Abgangsgruppe oder

ii. -SH, $-S^-$ oder $-SO_2^-$ bedeutet,

umsetzt mit einer Verbindung der Formel III

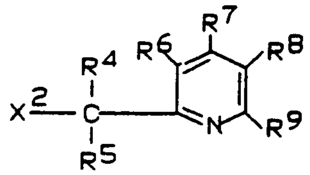

(III)

in welcher $R^4$, $R^5$, $R^6$, $R^7$, $R^8$ und $R^9$ wie im Anspruch 1 definiert sind und
$X^2$ im oben genannten Fall i. -SH, $-S^-$ oder $-SO_2^-$ und
im oben genannten Fall ii. eine Abgangsgruppe bedeutet, oder
b) eine Verbindung der Formel IV,

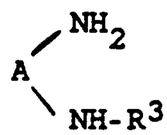

(IV)

in welcher A, $R^1$, $R^2$ und $R^3$ wie im Anspruch 1 definiert sind, umsetzt mit einer Verbindung der Formel V

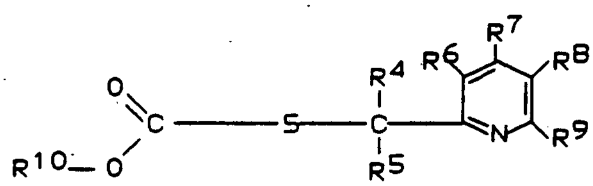

(V)

in welcher $R^4$, $R^5$, $R^6$, $R^7$, $R^8$ und $R^9$ wie im Anspruch 1 definiert sind und $R^{10}$ für eine veresternde Gruppe steht,

i. in einer Verbindung der Formel I (eine) gegebenenfalls vorhandene -S-Gruppe(n) gewünschtenfalls zu(r) -SO oder $-SO_2$-Gruppe(n) oxidiert,

ii. in einer Verbindung der Formel I (eine) gegebenenfalls vorhandene -SO-Gruppe(n) gewünschtenfalls zu(r) $-SO_2$-Gruppe(n) oxidiert,

iii. eine Verbindung der Formel I, worin $R^3$ für Wasserstoff steht, gewünschtenfalls acyliert, alkyliert oder aralkyliert,

iv. eine Verbindung der Formel I, worin $R^3$ nicht Wasserstoff bedeutet, gewünschtenfalls verseift und

v. eine Verbindung der Formel I gewünschtenfalls in ihr physiologisch verträgliches Salz überführt,

wobei zwei oder mehr der Maßnahmen i.-iv. auch in einer anderen als der angegebenen Reihenfolge ausgeführt werden können.

**8.** Verbindung gemäß einem der Ansprüche 1 bis 6 zur Anwendung als Heilmittel.

**9.** Verbindung gemäß einem der Ansprüche 1 bis 6 zur Anwendung als Magensäuresekretionshemmer.

**10.** Verbindung gemäß einem der Ansprüche 1 bis 6 zur Anwendung als Heilmittel bei der Behandlung entzündlicher Darmerkrankungen.

**11.** Verbindung gemäß einem der Ansprüche 1 bis 6 zur Anwendung als Heilmittel bei der Behandlung der Diarrhoe.

**12.** Mittel enthaltend eine Verbindung gemäß einem der Ansprüche 1 bis 6.

**13.** Verfahren zur Herstellung eines Mittels gemäß Anspruch 12 dadurch gekennzeichnet, daß man eine

Verbindung gemäß einem der Ansprüche 1 bis 6 in eine geeignete Darreichungsform bringt.

**Patentansprüche für folgende Vertragsstaaten : AT, ES, GR**

1. Verfahren zur Herstellung einer Verbindung der Formel I

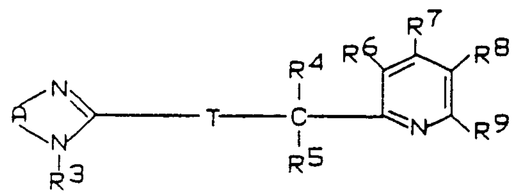

(I)

in welcher

| | |
|---|---|
| A | für 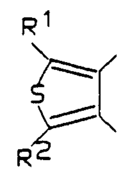 steht, |
| T | -S-, -SO- oder $-SO_2-$ bedeutet, |
| $R^1$ und $R^2$ | gleich oder verschieden sind und Wasserstoff, Halogen, Cyano, Nitro, Trifluormethyl, $(C_1-C_6)$-Alkyl, $(C_1-C_6)$-Hydroxyalkyl, $(C_1-C_6)$-Alkoxy, $(C_1-C_4)$-Fluoralkoxy, $-OCF_2Cl$, $-O-CF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl bedeuten, |
| $R^3$ | Wasserstoff, Alkanoyl, $(C_1-C_6)$-Alkylcarbamoyl oder eine andere physiologisch verträgliche, vorzugsweise im sauren Medium und/oder unter physiologischen Bedingungen abspaltbare $N^{im}$-Schutzgruppe bedeutet, |
| $R^4$ und $R^5$ | gleich oder verschieden sind und Wasserstoff oder $(C_1-C_3)$-Alkyl bedeuten, |
| $R^6$, $R^7$, $R^8$ und $R^9$ | gleich oder verschieden sind und Wasserstoff, Halogen, $(C_1-C_{12})$-Alkyl, $(C_1-C_{12})$-Alkoxy, $-O-CH_2-C_fH_{(2f+1-g)}F_g$, -NR'R", $(C_1-C_{12})$-Alkoxy-$(C_1-C_{12})$-alkyl, $(C_1-C_{12})$-Alkoxy-$(C_1-C_{12})$-alkoxy, $(C_7-C_{11})$-Aralkyloxy, $(C_1-C_{12})$-Alkylmercapto, $(C_1-C_{12})$-Alkylsulfinyl oder $(C_1-C_{12})$-Alkylsulfonyl bedeuten, oder |
| $R^5$ und $R^6$ | gemeinsam für $-[CH_2]_i-$ stehen, |
| R' und R" | gleich oder verschieden sind und Wasserstoff oder $(C_1-C_4)$-Alkyl bedeuten oder |
| R' und R" | gemeinsam für $-[CH_2]_h-$ stehen, worin eine $CH_2$-Gruppe durch O, S, N-$(C_1-C_4)$-Alkanoylimino oder N$(C_1-C_4)$-Alkoxylcarbonylimino ersetzt sein kann, |
| f = | 1, 2, 3 oder 4 ist, |
| g = | 1 bis (2f + 1) ist, |
| h = | 4, 5 oder 6 ist, |
| i = | 1, 2 oder 3 ist und |
| n = | 3 oder 4 ist, |

oder deren physiologisch verträglichen Salzen,

a) eine Verbindung der Formel II

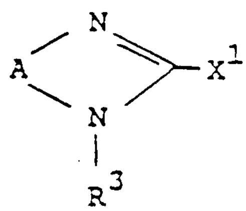

(II)

in welcher A, $R^1$, $R^2$ und $R^3$ wie im Anspruch definiert sind und

$X^1$ i. eine Abgangsgruppe oder

ii. -SH, $-S^-$ oder $-SO_2^-$ bedeutet,

umsetzt mit einer Verbindung der Formel III

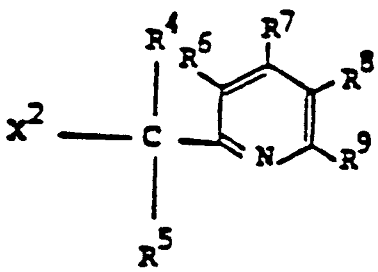

(III)

in welcher $R^4$, $R^5$, $R^6$, $R^7$, $R^8$ und $R^9$ wie oben definiert sind und

$X^2$ im oben genannten Fall i. -SH, $-S^-$ oder $-SO_2^-$ und

im oben genannten Fall ii. eine Abgangsgruppe bedeutet, oder

b) eine Verbindung der Formel IV,

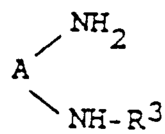

(IV)

in welcher A, $R^1$, $R^2$ und $R^3$ wie oben definiert sind, umsetzt mit einer Verbindung der Formel V

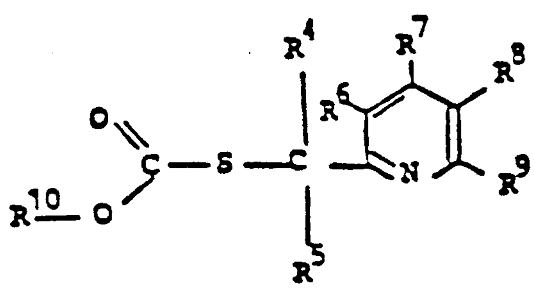

(V).

in welcher $R^4$, $R^5$, $R^6$, $R^7$, $H^8$ und $R^9$ wie oben definiert sind und $R^{10}$ für eine veresternde Gruppe steht,

i. in einer Verbindung der Formel I (eine) gegebenenfalls vorhandene -S-Gruppe(n) gewünschtenfalls zu(r) -SO- oder $-SO_2$-Gruppe(n) oxidiert,

ii. in einer Verbindung der Formel I (eine) gegebenenfalls vorhandene -SO-Gruppe(n) gewünschtenfalls zu(r) $-SO_2$-Gruppe(n) oxidiert,

iii. eine Verbindung der Formel I, worin $R^3$ für Wasserstoff steht, gewünschtenfalls acyliert, alkyliert oder aralkyliert,

iv. eine Verbindung der Formel I, worin $R^3$ nicht Wasserstoff bedeutet, gewünschtenfalls verseift

und

v. eine Verbindung der Formel I gewünschtenfalls in ihr physiologisch verträgliches Salz überführt,

wobei zwei oder mehr der Maßnahmen i.-iv. auch in einer anderen als der angegebenen Reihenfolge ausgeführt werden können.

**2.** Verfahren gemäß Anspruch 1, dadurch gekennzeichnet, daß eine Verbindung der Formel I hergestellt wird, in welcher $R^9$ Wasserstoff bedeutet.

**3.** Verfahren gemäß einem der Ansprüche 1 oder 2, dadurch gekennzeichnet, daß eine Verbindung der Formel I hergestellt wird, in welcher T für -SO- steht.

**4.** Verfahren gemäß einem der Ansprüche 1 bis 3, dadurch gekennzeichnet, daß eine Verbindung der Formel I hergestellt wird, in welcher

| | |
|---|---|
| $R^1$ und $R^2$ | gleich oder verschieden sind und Wasserstoff, $(C_1-C_3)$-Alkyl, Halogen, $(C_1-C_4)$-Alkoxy oder $(C_1-C_4)$-Alkoxycarbonyl bedeuten, |
| $R^3$ | wie im Anspruch 1 definiert ist, |
| $R^4$ und $R^5$ | jeweils Wasserstoff bedeuten und |
| $R^6$,$R^7$,$R^8$ und $R^9$ | gleich oder verschien sind und Wasserstoff, Halogen, $(C_1-C_3)$Alkyl, $(C_1-C_4)$-Alkoxy, Benzyloxy oder $(C_1-C_7)$-Alkoxy-$(C_1-C_3)$-alkyl bedeuten. |

**5.** Verfahren gemäß einem der Ansprüche 1 bis 4, in welcher

| | |
|---|---|
| $R^1$ und $R^2$ | gleich oder verschieden sind und Wasserstoff oder $(C_1-C_3)$-Alkyl bedeuten, |
| $R^3$ | wie in Anspruch 1 definiert ist, |
| $R^4$ und $R^5$ | jeweils Wasserstoff bedeuten, |
| $R^6$ und $R^8$ | gleich oder verschieden sind und Wasserstoff, Chlor, Methyl oder Ethyl bedeuten, |
| $R^9$ | Wasserstoff bedeutet und |
| $R^7$ | Wasserstoff, $(C_1-C_4)$-Alkoxy, $(C_1-C_3)$-Alkyl oder Benzyloxy bedeutet. |

**6.** Verfahren gemäß Anspruch 1 zur Herstellung von 2-[4-(2,2,3,3,4,4,4-Heptafluorbutyloxy)-2-picolylsulfinyl]-1H-thieno[3,4-d]imidazol oder dessen physiologisch verträglichen Salzen.

**7.** Verfahren zur Herstellung eines Mittels enthaltend eine Verbindung hergestellt gemäß einem der Ansprüche 1 bis 6, dadurch gekennzeichnet, daß man diese Verbindung in eine geeignete Darreichungsform bringt.

## Claims

**Claims for the following Contracting States : BE, CH/LI, DE, FR, GB, IT, LU, NL, SE**

**1.** A compound of the formula I

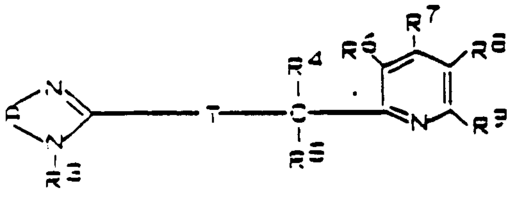

(I)

in which

A is

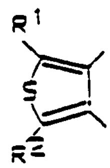

| | |
|---|---|
| T | is -S-, -SO- or $SO_2$-, |
| $R^1$ and $R^2$ | are identical or different and are hydrogen, halogen, cyano, nitro, trifluoromethyl, ($C_1$-$C_6$)-alkyl, ($C_1$-$C_6$)-hydroxyalkyl, ($C_1$-$C_6$)-alkoxy, ($C_1$-$C_4$)-fluoroalkoxy, -$OCF_2Cl$, -O-$CF_2$-CHFCl, ($C_1$-$C_6$)-alkylmercapto, ($C_1$-$C_6$)-alkylsulfinyl, ($C_1$-$C_6$)-alkylsulfonyl, ($C_1$-$C_6$)-alkylcarbonyl, ($C_1$-$C_6$)-alkoxycarbonyl, carbamoyl, N-($C_1$-C4)-alkylcarbamoyl, N,N-di-($C_1$-$C_4$) alkylcarbamoyl, ($C_1$-$C_6$)-alkylcarbonyloxy, ($C_3$-$C_8$)-cycloalkyl, phenyl, benzyl, phenoxy, benzyloxy, anilino, N-methylanilino, phenylmercapto, phenylsulfonyl, phenylsulfinyl, sulfamoyl, N-($C_1$-$C_4$)-alkylsulfamoyl or N,N,-di-($C_1$-$C_4$)-alkylsulfamoyl, |
| $R^3$ | is hydrogen, alkanoyl, ($C_1$-$C_6$)-alkylcarbamoyl or another physiologically tolerated $N^{im}$ protective group which can be eliminated preferably in an acid medium and/or under physiological conditions, |
| $R^4$ and $R^5$ | are identical or different and are hydrogen or ($C_1$-$C_3$)-alkyl, |
| $R^6$, $R^7$, $R^8$ and $R^9$ | are identical or different and are hydrogen, halogen, ($C_1$-$C_{12}$)-alkyl, ($C_1$-$C_{12}$)-alkoxy, -O-$CH_2$-$C_fH_{(2f+1-g)}F_g$,-NR'R", ($C_1$-$C_{12}$)-alkoxy-($C_1$-$C_{12}$)-alkyl, ($C_1$-$C_{12}$)-alkoxy-($C_1$-$C_{12}$)-alkoxy, ($C_7$-$C_{11}$)-aralkyloxy, ($C_1$-$C_{12}$)-alkylmercapto, ($C_1$-$C_{12}$)-alkylsulfinyl or ($C_1$-$C_{12}$)-alkylsulfonyl, or |
| $R^5$ and $R^6$ | together are -$[CH_2]_i$-, |
| R' and R" | are identical or different and are hydrogen or ($C_1$-$C_4$)-alkyl, or |
| R' and R" | together are -$[CH_2]_h$-, in which one $CH_2$-group can be replaced by O, S, N-($C_1$-$C_4$)-alkanoylimino or N-($C_1$-$C_4$)-alkoxycarbonylimino, |
| f | = 1, 2, 3 or 4, |
| g | = 1 to (2f+1), |
| h | = 4, 5 or 6, |
| i | = 1, 2 or 3 and |
| n | = 3 or 4, |

and its physiologically tolerated salts.

**2.** A compound of the formula I as claimed in claim 1, in which $R^9$ is hydrogen.

**3.** A compound of the formula I as claimed in claim 1 or 2, in which T is -SO-.

**4.** A compound of the formula I as claimed in one of claims 1 to 3, in which

| | |
|---|---|
| $R^1$ and $R^2$ | are identical or different and are hydrogen, ($C_1$-$C_3$)-alkyl, halogen, ($C_1$-$C_4$)-alkoxy or ($C_1$-$C_4$)-alkoxycarbonyl, |
| $R^3$ | is as defined in Claim 1, |
| $R^4$ and $R^5$ | are each hydrogen and |
| $R^6$, $R^7$, $R^8$ and $R^9$ | are identical or different and are hydrogen, halogen, ($C_1$-$C_3$)-alkyl, ($C_1$-$C_4$)-alkoxy, benzyloxy or ($C_1$-$C_7$)-alkoxy-($C_1$-$C_3$)-alkyl. |

**5.** A compound of the formula I as claimed in one of claims 1 to 4, in which

| | |
|---|---|
| $R^1$ and $R^2$ | are identical or different and are hydrogen or ($C_1$-$C_3$)-alkyl, |
| $R^3$ | is as defined in claim 1, |
| $R^4$ and $R^5$ | are each hydrogen, |
| $R^6$ and $R^8$ | are identical or different and are hydrogen, chlorine, methyl or ethyl, |
| $R^9$ | is hydrogen and |
| $R^7$ | is hydrogen, ($C_1$-$C_4$)-alkoxy, ($C_1$-$C_3$)-alkyl or benzyloxy. |

**6.** 2-[4-(2,2,3,3,4,4,4-heptaflurobutyloxy)-2-picolylsulfinyl]-1H-thieno[3,4-d]imidazole or the physiologically tolerated salts thereof.

**7.** A process for the preparation of a compound of the formula I as claimed in one of claims 1 to 6 which comprises

a) reaction of a compound of the formula II

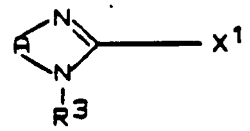

(II)

in which A, $R^1$, $R^2$ and $R^3$ are as defined in claim 1, and

$X^1$ is i. a leaving group or

ii. -SH, -S' or $-SO_2^-$,

with a compound of the formula III

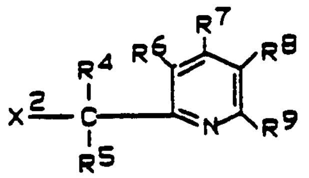

(III)

in which $R^4$, $R^5$, $R^6$, $R^7$, $R^8$ and $R^9$ are as defined in claim 1 and

$X^2$ in the abovementioned case i. is -SH, -S' or $-SO_2^-$, and

in the abovementioned case ii. is a leaving group or

b) reaction of a compound of the formula IV

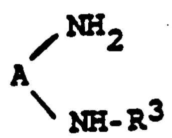

(IV)

in which A, $R^1$, $R^2$ and $R^3$ are as defined in claim 1, with a compound of the formula V

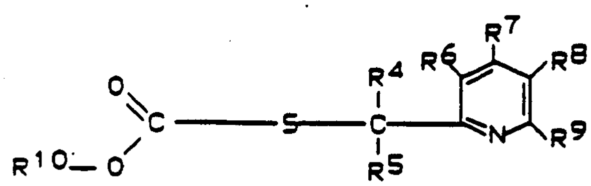

(V)

in which $R^4$, $R^5$, $R^6$, $R^7$, $R^8$ and $R^9$ are as defined in claim 1 and $R^{10}$ is an esterifying group, and

i. if desired, oxidation of (a) -S- group(s) present, where appropriate, in a compound of the formula I to -SO or $-SO_2$- group(s),

ii. if desired, oxidation of (a) -SO- group(s) present, where appropriate, in a compound of the formula I to $-SO_2$- group(s),

iii. if desired, acylation, alkylation or aralkylation of a compound of the formula I in which $R^3$ is hydrogen,

iv. if desired, hydrolysis of a compound of the formula I in which $R^3$ is not hydrogen, and

v. if desired, conversion of a compound of the formula I into its physiologically tolerated salt,

it also being possible for two or more of measures i.-iv. to be carried out in a sequence different from that indicated.

**8.** A compound as claimed in one of claims 1 to 6 for use as medicine.

**9.** A compound as claimed in one of claims 1 to 6 for use as inhibitor of gastric acid secretion.

**10.** A compound as claimed in one of claims 1 to 6 for use as medicine in the treatment of inflammatory intestinal disorders.

**11.** A compound as claimed in one of claims 1 to 6 for use as medicine in the treatment of diarrhoea.

**12.** An agent containing a compound as claimed in one of claims 1 to 6.

**13.** A process for the preparation of an agent as claimed in claim 12, which comprises converting a compound as claimed in one of claims 1 to 6, into a suitable form for administration.

**Claims for the following Contracting States : AT, ES, GR**

**1.** A process for the preparation of a compound of the formula I

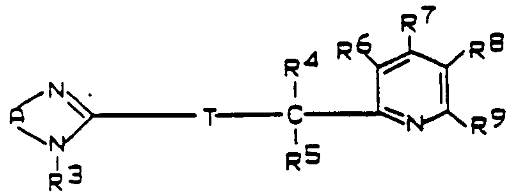

(I)

in which

A is

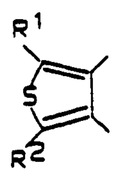

| | |
|---|---|
| T | is -S-, -SO- or $-SO_2-$, |
| $R^1$ and $R^2$ | are identical or different and are hydrogen, halogen, cyano, nitro, trifluoromethyl,$(C_1-C_6)$-alkyl,$(C_1-C_6)$-hydroxyalkyl, $(C_1-C_6)$-alkoxy, $(C_1-C_4)$-fluoroalkoxy, $-OCF_2Cl$, $-O-CF_2-CHFCl$, $(C_1-C_6)$-alkylmercapto, $(C_1-C_6)$-alkylsylfinyl, $(C_1-C_6)$-alkylsulfonyl, $(C_1-C_6)$-alkylcarbonyl, $(C_1-C_6)$-alkoxycarbonyl, carbamoyl, N-$(C_1-C_4)$-alkylcarbamoyl, N,N-di-$(C_1-C_4)$-alkylcarbamoyl, $(C_1-C_6)$-alkylcarbonyloxy, $(C_3-C_8)$-cycloalkyl, phenyl, benzyl, phenoxy, benzyloxy, anilino, N-methylanilino, phenylmercapto, phenylsulfonyl, phenylsulfinyl, sulfamoyl, N-$(C_1-C_4)$-alkylsulfamoyl or N,N,-di-$(C_1-C_4)$-alkylsulfamoyl, |
| $R^3$ | is hydrogen, alkanoyl, $(C_1-C_6)$-alkylcarbamoyl or another physiologically tolerated $N^{im}$ protective group, which can be eliminated preferably in an acid medium and/or under physiological conditions, |
| $R^4$ and $R^5$ | are identical or different and are hydrogen or $(C_1-C_3)$-alkyl, |
| $R^6$, $R^7$, $R^8$ and $R^9$ | are identical or different and are hydrogen, halogen, $(C_1-C_{12})$-alkyl, $(C_1-C_{12})$-alkoxy, $-O-CH_2-C_fH_{(2f+1-g)}F_g$,-NR'R", $(C_1-C_{12})$-alkoxy-$(C_1-C_{12})$-alkyl, $(C_1-C_{12})$-alkoxy-$(C_1-C_{12})$-alkoxy, $(C_7-C_{11})$-aralkyloxy, $(C_1-C_{12})$-alkylmercapto, $(C_1-C_{12})$-alkyl-sulfinyl or $(C_1-C_{12})$-alkylsulfonyl, or |
| $R^5$ and $R^6$ | together are $-[CH_2]_i-$, |
| R' and R" | are identical or different and are hydrogen or $(C_1-C_4)$-alkyl, or |
| R' and R" | together are $-[CH_2]_h-$, in which one $CH_2$-group can be replaced by O, S, N-$(C_1-C_4)$-alkanoylimino or N-$(C_1-C_4)$-alkoxycarbonylimino, |
| f | = 1, 2, 3 or 4, |

g = 1 to (2f + 1),
h = 4, 5 or 6,
i = 1, 2 or 3 and
n = 3 or 4,

or its physiologically tolerated salts, which comprises

a) reaction of a compound of the formula II

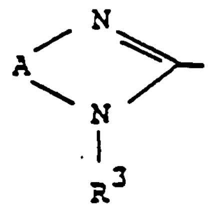

(II)

in which A, $R^1$, $R^2$ and $R^3$ are as defined in claim 1, and

$X^1$ is i. a leaving group or
ii. -SH, $-S^-$ or $SO_2^-$,

with a compound of the formula III

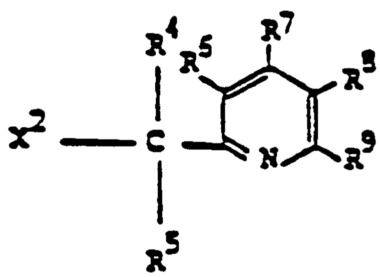

(III)

in which $R^4$, $R^5$, $R^6$, $R^7$, $R^8$ and $R^9$ are as defined above and $X^2$ in the abovementioned case i. is -SH, $-S^-$ or $-SOC_2^-$, and in the abovementioned case ii. is a leaving group or

b) reaction of a compound of the formula IV

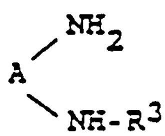

(IV)

in which A, $R^1$, $R^2$, and R3 are as defined above, with a compound of the formula V

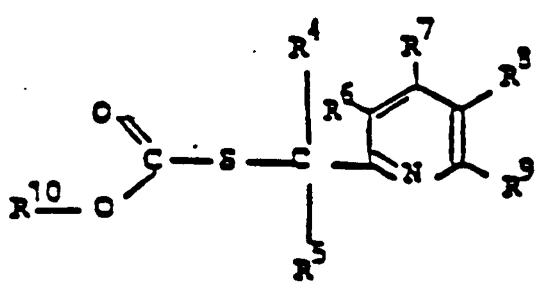

(V)

in which $R^4$, $R^5$, $R^6$, $R^7$, $R^8$ and $R^9$ are as defined above and $R^{10}$ is an esterifying group, and

i. if desired, oxidation of (a) -S- group(s) present, where appropriate, in a compound of the formula I to -SO- or $-SO_2-$ group(s),

ii. if desired, oxidation of (a) -SO- group(s) present, where appropriate, in a compound of the formula I to $-SO_2-$ group(s),

iii. if desired, acylation, alkylation or aralkylation of a compound of the formula I in which $R^3$ is hydrogen,

iv. if desired, hydrolysis of a compound of the formula I in which $R^3$ is not hydrogen, and

v. if desired, conversion of a compound of the formula I into its physiologically tolerated salt,

it also being possible for two or more of measures i.-iv. to be carried out in a sequence different from that indicated.

**2.** The process as claimed in claim 1, in which is prepared a compound of the formula I in which $R^9$ is hydrogen.

**3.** The process as claimed in claim 1 or 2 in which is prepared a compound of the formula I in which T is -SO-.

**4.** The process as claimed in one of claims 1 to 3, in which is prepared a compound of the formula I in which

| | |
|---|---|
| $R^1$ and $R^2$ | are identical or different and are hydrogen, $(C_1-C_3)$-alkyl, halogen $(C_1-C_4)$-alkoxy or $(C_1-C_4)$-alkoxycarbonyl, |
| $R^3$ | is as defined in claim 1, |
| $R^4$ and $R^5$ | are each hydrogen and |
| $R^6$, $R^7$, $R^8$ and $R^9$ | are identical or different and are hydrogen, halogen, $(C_1-C_3)$-alkyl, $(C_1-C_4)$-alkoxy, benzyloxy or $(C_1-C_7)$-alkoxy-$(C_1-C_3)$-alkyl. |

**5.** The process as claimed in one of claims 1 to 4, in which

| | |
|---|---|
| $R^1$ and $R^2$ | are identical or different and are hydrogen or $(C_1-C_3)$-alkyl, |
| $R^3$ | is as defined in claim 1, |
| $R^4$ and $R^5$ | are each hydrogen, |
| $R^6$ and $R^8$ | are identical or different and are hydrogen, chlorine, methyl or ethyl, |
| $R^9$ | is hydrogen and |
| $R^7$ | is hydrogen, $(C_1-C_4)$-alkoxy $(C_1-C_3)$-alkyl or benzyloxy. |

**6.** The process as claimed in claim 1 for preparing 2-[4-(2,2,3,3,4,4,4-heptafluorobutyloxy)-2-picolylsulfinyl]-1H-thieno[3,4-d]imidazole or the physiologically tolerated salts thereof.

**7.** A process for the preparation of an agent containing a compound prepared as claimed in one of claims 1 to 6 which comprises converting this compound into a suitable form for administration.

**Revendications**

**Revendications pour les Etats contractants suivants : BE, CH, DE, FR, GB, IT, LI, LU, NL, SE**

**1.** Composé de formule I

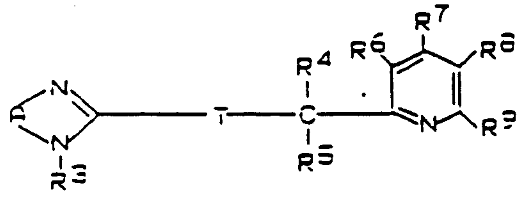

(I)

dans laquelle

A représente

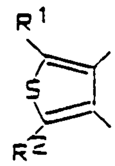

| | |
|---|---|
| T | représente -S-, -SO- ou -$SO_2$-, |
| $R^1$ et $R^2$ | sont identiques ou différents, et représentent un atome d'hydrogène ou d'halogène ou un groupe cyano, nitro, trifluorométhyle, alkyle en $C_1$-$C_6$, hydroxyalkyle en $C_1$-$C_6$, alcoxy en $C_1$-$C_6$, fluoro-alcoxy en $C_1$-$C_4$, -$OCF_2Cl$, -O-$CF_2$-CHFCl, alkyl($C_1$-$C_6$)-mercapto, alkyl($C_1$-$C_6$)-sulfinyle, alkyl($C_1$-$C_6$)-sulfonyle, alkyl($C_1$-$C_6$)-carbonyle, alcoxy($C_1$-$C_6$)-carbonyle, carbamoyle, N-alkyl($C_1$-$C_4$)-carbamoyle, N,N-dialkyl($C_1$-$C_4$)-carbamoyle, alkyl($C_1$-$C_6$)-carbonyloxy, cycloalkyle en $C_3$-$C_8$, phényle, benzyle, phénoxy, benzyloxy, anilino, N-méthylanilino, phénylmercapto, phénylsulfonyle, phénylsulfinyle, sulfamoyle, N-alkyl($C_1$-$C_4$)-sulfamoyle ou N,N-dialkyl($C_1$-$C_4$)-sulfamoyle, |
| $R^3$ | représente un atome d'hydrogène ou un groupe alcanoyle, alkyl($C_1$-$C_6$)-carbamoyle ou un autre groupe protecteur d'azote en fonction imine, physiologiquement acceptable, de préférence séparable en milieu acide et/ou dans des conditions physiologiques, |
| $R^4$ et $R^5$ | sont identiques ou différents, et représentent un atome d'hydrogène ou un groupe alkyle en $C_1$-$C_3$, |
| $R^6$, $R^7$, $R^8$ et $R^9$ | sont identiques ou différents, et représentent un atome d'hydrogène ou d'halogène, ou un groupe alkyle en $C_1$-$C_{12}$, alcoxy en $C_1$-$C_{12}$, -O-$CH_2$-$C_fH_{(2f+1-g)}F_g$, -NR'R", alcoxy($C_1$-$C_{12}$)-alkyle($C_1$-$C_{12}$), alcoxy($C_1$-$C_{12}$)-alcoxy($C_1$-$C_{12}$), aralkyloxy en $C_7$-$C_{11}$, alkyl($C_1$-$C_{12}$)-mercapto, alkyl($C_1$-$C_{12}$)-sulfinyle ou alkyl($C_1$-$C_{12}$)-sulfonyle, ou |
| $R^5$ et $R^6$ | représentent ensemble -$[CH_2]_i$-, |
| R' et R" | sont identiques ou différents, et représentent un atome d'hydrogène ou un groupe alkyle en $C_1$-$C_4$, ou |
| R' et R" | forment ensemble un groupement -$[CH_2]_h$- dans lequel un groupe $CH_2$ peut être remplacé par O, S ou par un groupe N-alcanoyl($C_1$-$C_4$)-imino ou N-alcoxy-($C_1$-$C_4$)-carbonylimino, |
| f = | 1, 2, 3 ou 4, |
| g | va de 1 à (2f + 1), |
| h = | 4, 5 ou 6, |
| i = | 1, 2 ou 3 et |
| n = | 3 ou 4, |

et sels physiologiquement acceptables de celui-ci.

**2.** Composé de formule I selon la revendication 1, dans lequel $R^9$ représente un atome d'hydrogène.

**3.** Composé de formule I selon la revendication 1 ou 2, dans lequel T représente -SO-.

**4.** Composé de formule I selon l'une des revendications 1 à 3, dans lequel

| | |
|---|---|
| $R^1$ et $R^2$ | sont identiques ou différents, et représentent un atome d'hydrogène ou d'halogène ou un groupe alkyle en $C_1$-$C_3$, alcoxy en $C_1$-$C_4$, ou alcoxy($C_1$-$C_4$)-carbonyle, |
| $R^3$ | est tel que défini dans la revendication 1, |
| $R^4$ et $R^5$ | représentent chacun un atome d'hydrogène, et |
| $R^6$, $R^7$, $R^8$ et $R^9$ | sont identiques ou différents, et représentent un atome d'hydrogène ou d'halogène, ou un groupe alkyle en $C_1$-$C_3$, alcoxy en $C_1$-$C_4$, benzyloxy ou alcoxy-($C_1$-$C_7$)-alkyle($C_1$-$C_3$). |

**5.** Composé de formule I selon l'une des revendications 1 à 4, dans lequel

| | |
|---|---|
| $R^1$ et $R^2$ | sont identiques ou différents, et représentent un atome d'hydrogène ou un groupe alkyle en $C_1$-$C_3$, |

| | |
|---|---|
| $R^3$ | est tel que défini dans la revendication 1, |
| $R^4$ et $R^5$ | représentent chacun un atome d'hydrogène, |
| $R^6$ et $R^8$ | sont identiques ou différents, et représentent un atome d'hydrogène ou de chlore, ou le groupe méthyle ou éthyle, |
| $R^9$ | représente un atome d'hydrogène, et |
| $R^7$ | représente un atome d'hydrogène ou un groupe alcoxy en $C_1$-$C_4$, alkyle en $C_1$-$C_3$ ou benzyloxy. |

**6.** 2-[4-(2,2,3,3,4,4,4-heptafluorobutyloxy)-2-picolylsulfinyl]-1H-thiéno[3,4-d]imidazole ou sels physiologiquement acceptables de celui-ci.

**7.** Procédé pour la préparation d'un composé de formule I selon l'une des revendications 1 à 6, caractérisé en ce que

a) on fait réagir un composé de formule II

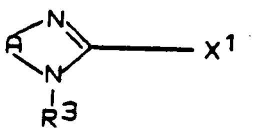

(II)

dans laquelle A, $R^1$, $R^2$ et $R^3$ sont tels que définis dans la revendication 1 et

$X^1$ représente

I. un groupe séparable ou

II. -SH, $-S^-$ ou $-SO_2^-$,

avec un composé de formule III

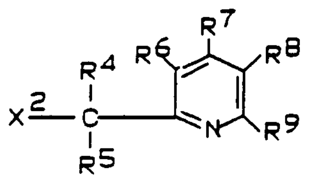

(III)

dans laquelle $R^4$, $R^5$, $R^6$, $R^7$, $R^8$ et $R^9$ sont tels que définis dans la revendication 1, et

$X^2$ représente

dans le cas I mentionné ci-dessus, -SH, $-S^-$ ou $-SO_2^-$ et

dans le cas II mentionné ci-dessus, un groupe séparable, ou

b) on fait réagir un composé de formule IV

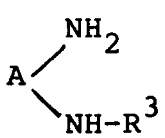

(IV)

dans laquelle A, $R^1$, $R^2$ et $R^3$ sont tels que définis dans la revendication 1, avec un composé de formule V

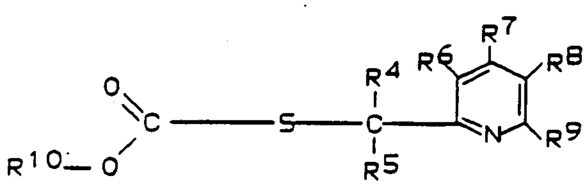

(V)

dans laquelle $R^4$, $R^5$, $R^6$, $R^7$, $R^8$ et $R^9$ sont tels que définis dans la revendication 1, et $R^{10}$ représente un groupe estérifiant, et

I. on oxyde si on le désire en groupe(s) $-SO$ ou $-SO_2$ un ou des atome(s) de S éventuellement présent(s) dans un composé de formule I;

II. on oxyde si on le désire en groupe(s) $-SO_2$ un ou des groupe(s) $-SO$ éventuellement présent(s) dans un composé de formule I;

III. on soumet si on le désire à une acylation, alkylation ou aralkylation un composé de formule I dans lequel $R^3$ représente un atome d'hydrogène;

IV. on saponifie si on le désire un composé de formule I dans lequel $R^3$ ne représente pas un atome d'hydrogène, et

V. on transforme si on le désire un composé de formule I en un de ses sels physiologiquement acceptable;

deux des opérations I-IV ou plus pouvant également être effectuées dans un ordre différent de celui indiqué.

**8.** Composé selon l'une des revendications 1 à 6, pour utilisation en tant que médicament.

**9.** Composé selon l'une des revendications 1 à 6, pour utilisation en tant qu'inhibiteur de la sécrétion d'acide gastrique.

**10.** Composé selon l'une des revendications 1 à 6, pour utilisation en tant que médicament dans le traitement de maladies intestinales inflammatoires.

**11.** Composé selon l'une des revendications 1 à 6, pour utilisation en tant que médicament dans le traitement de la diarrhée.

**12.** Médicament contenant un composé selon l'une des revendications 1 à 6.

**13.** Procédé pour la fabrication d'un médicament selon la revendication 12, caractérisé en ce que l'on met sous une forme pharmaceutique appropriée un composé selon l'une des revendications 1 à 6.

**Revendications pour les Etats contractants suivants : AT, ES, GR**

**1.** Procédé pour la préparation d'un composé de formule I

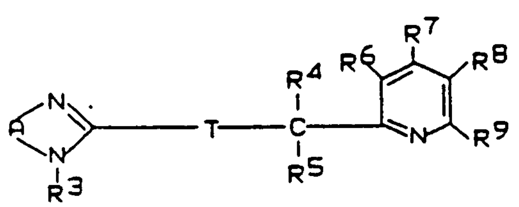

(I)

dans laquelle

A représente

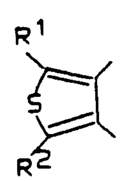

T représente $-S-$, $-SO-$ ou $-SO_2-$,

$R^1$ et $R^2$ sont identiques ou différents, et représentent un atome d'hydrogène ou d'halogène ou un groupe cyano, nitro, trifluorométhyle, alkyle en $C_1$-$C_6$, hydroxyalkyle en $C_1$-$C_6$, alcoxy en $C_1$-$C_6$, fluoro-alcoxy en $C_1$-$C_4$, $-OCF_2Cl$, $-O-CF_2-CHFCl$, alkyl($C_1$-$C_6$)-mercapto, alkyl($C_1$-$C_6$)-sulfinyle, alkyl($C_1$-$C_6$)-sulfonyle, alkyl($C_1$-

$C_6$)-carbonyle, alcoxy($C_1$-$C_6$)-carbonyle, carbamoyle, N-alkyl($C_1$-$C_4$)-carbamoyle, N,N-dialkyl($C_1$-$C_4$)-carbamoyle, alkyl($C_1$-$C_6$)-carbonyloxy, cycloalkyle en $C_3$-$C_8$, phényle, benzyle, phénoxy, benzyloxy, anilino, N-méthylanilino, phénylmercapto, phénylsulfonyle, phénylsulfinyle, sulfamoyle, N-alkyl($C_1$-$C_4$)-sulfamoyle ou N,N-dialkyl($C_1$-$C_4$)-sulfamoyle,

$R^3$ représente un atome d'hydrogène ou un groupe alcanoyle, alkyl($C_1$-$C_6$)-carbamoyle ou un autre groupe protecteur d'azote en fonction imine, physiologiquement acceptable, de préférence séparable en milieu acide et/ou dans des conditions physiologiques,

$R^4$ et $R^5$ sont identiques ou différents, et représentent un atome d'hydrogène ou un groupe alkyle en $C_1$-$C_3$,

$R^6$, $R^7$, $R^8$ et $R^9$ sont identiques ou différents, et représentent un atome d'hydrogène ou d'halogène, ou un groupe alkyle en $C_1$-$C_{12}$, alcoxy en $C_1$-$C_{12}$, $-O-CH_2-C_fH_{(2f+1-g)}F_g$, -NR'R", alcoxy($C_1$-$C_{12}$)-alkyle($C_1$-$C_{12}$), alcoxy($C_1$-$C_{12}$)-alcoxy($C_1$-$C_{12}$), aralkyloxy en $C_7$-$C_{11}$, alkyl($C_1$-$C_{12}$)-mercapto, alkyl($C_1$-$C_{12}$)-sulfinyle ou alkyl($C_1$-$C_{12}$)-sulfonyle, ou

$R^5$ et $R^6$ représentent ensemble $-[CH_2]_i-$,

R' et R" sont identiques ou différents, et représentent un atome d'hydrogène ou un groupe alkyle en $C_1$-$C_4$, ou

R' et R" forment ensemble un groupement $-[CH_2]_h-$ dans lequel un groupe $CH_2$ peut être remplacé par O, S ou par un groupe N-alcanoyl($C_1$-$C_4$)-imino ou N-alcoxy-($C_1$-$C_4$)-carbonylimino,

$f =$ 1, 2, 3 ou 4,

g va de 1 à $(2f+1)$,

$h =$ 4, 5 ou 6,

$i =$ 1, 2 ou 3 et

$n =$ 3 ou 4,

ou de ses sels physiologiquement acceptables,
caractérisé en ce que

a) on fait réagir un composé de formule II

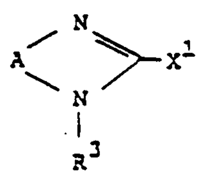

(II)

dans laquelle A, $R^1$, $R^2$ et $R^3$ sont tels que définis ci-dessus, et

$X^1$ représente

I. un groupe séparable ou

II. -SH, $-S^-$ ou $-SO_2^-$,

avec un composé de formule III

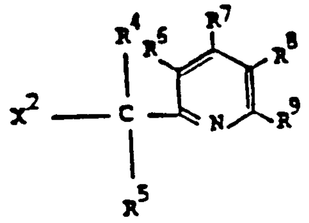

(III)

dans laquelle $R^4$, $R^5$, $R^6$, $R^7$, $R^8$ et $R^9$ sont tels que définis ci-dessus, et

$X^2$ représente

dans le cas I mentionné ci-dessus, -SH, $-S^-$ ou $-SO_2^-$ et

dans le cas II mentionné ci-dessus, un groupe séparable, ou

b) on fait réagir un composé de formule IV

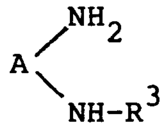

(IV)

dans laquelle A, et $R^3$ sont tels que définis ci-dessus, avec un composé de formule V

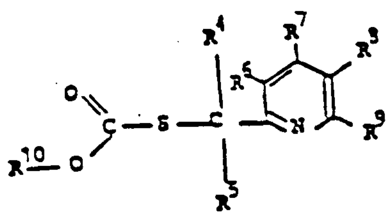

(V)

dans laquelle $R^4$, $R^5$, $R^6$, $R^7$, $R^8$ et $R^9$ sont tels que définis ci-dessus, et $R^{10}$ représente un groupe estérifiant, et

I. on oxyde si on le désire en groupe(s) -SO ou $-SO_2$ un ou des atome(s) de S éventuellement présent(s) dans un composé de formule I;

II. on oxyde si on le désire en groupe(s) $-SO_2$ un ou des groupe(s) -SO éventuellement présent(s) dans un composé de formule I;

III. on soumet si on le désire à une acylation, alkylation ou aralkylation un composé de formule I dans lequel $R^3$ représente un atome d'hydrogène;

IV. on saponifie si on le désire un composé de formule I, dans lequel $R^3$ ne représente pas un atome d'hydrogène, et

V. on transforme si on le désire un composé de formule I en un de ses sels physiologiquement acceptable;

deux des opérations I-IV ou plus pouvant également être effectuées dans un ordre différent de celui indiqué.

**2.** Procédé selon la revendication 1, caractérisé en ce que l'on prépare un composé de formule I dans lequel $R^9$ représente un atome d'hydrogène.

**3.** Procédé selon la revendication 1 ou 2, caractérisé en ce que l'on prépare un composé de formule I dans lequel T représente -SO-.

**4.** Procédé selon l'une des revendications 1 à 3, caractérisé en ce que l'on prépare un composé de formule I dans lequel

| | |
|---|---|
| $R^1$ et $R^2$ | sont identiques ou différents, et représentent un atome d'hydrogène ou d'halogène ou un groupe alkyle en $C_1$-$C_3$, alcoxy en $C_1$-$C_4$, ou alcoxy($C_1$-$C_4$)-carbonyle, |
| $R^3$ | est tel que défini dans la revendication 1, |
| $R^4$ et $R^5$ | représentent chacun un atome d'hydrogène, et |
| $R^6$, $R^7$, $R^8$ et $R^9$ | sont identiques ou différents, et représentent un atome d'hydrogène ou d'halogène, ou un groupe alkyle en $C_1$-$C_3$, alcoxy en $C_1$-$C_4$, benzyloxy ou alcoxy-($C_1$-$C_7$)-alkyle($C_1$-$C_3$). |

**5.** Procédé selon l'une des revendications 1 à 4, caractérisé en ce que l'on prépare un composé de formule I dans lequel

| | |
|---|---|
| $R^1$ et $R^2$ | sont identiques ou différents, et représentent un atome d'hydrogène ou un groupe alkyle en $C_1$-$C_3$, |
| $R^3$ | est tel que défini dans la revendication 1, |
| $R^4$ et $R^5$ | représentent chacun un atome d'hydrogène, |
| $R^6$ et $R^8$ | sont identiques ou différents, et représentent un atome d'hydrogène ou de chlore, ou le groupe méthyle ou éthyle, |
| $R^9$ | représente un atome d'hydrogène, et |

$R^7$ représente un atome d'hydrogène ou un groupe alcoxy en $C_1$-$C_4$, alkyle en $C_1$-$C_3$ ou benzyloxy.

**6.** Procédé selon la revendication 1 pour la préparation du 2-[4-(2,2,3,3,4,4,4-heptafluorobutyloxy)-2-picolylsulfinyl]-1H-thiéno[3,4-d]imidazole ou de ses sels physiologiquement acceptables.

**7.** Procédé pour la préparation d'un médicament contenant un composé selon l'une des revendications 1 à 6, caractérisé en ce que l'on met ce composé sous une forme pharmaceutiquement appropriée.